# EUROPEAN PATENT APPLICATION

(11) **EP 3 427 697 A2**
(43) Date of publication of application: **16.01.2019**
(21) Application number: 18163348.8
(22) Date of filing: 22.03.2018
(51) Int. Cl.: A61F 2/28, A61F 2/30, A61F 2/44, A61F 2/46

(54) **3-D PRINTING OF POROUS IMPLANTS**

(30) Priority: 18.04.2017 US 201715490380
(71) Applicant: Warsaw Orthopedic, Inc., Warsaw, IN 46581 (US)
(72) Inventor: WEI, Guobao, Miltown, NJ New Jersey 08850 (US)
(74) Representative: Viering, Jentschura & Partner mbB Patent- und Rechtsanwälte

(57) **Abstract**

Computer implemented methods of producing a porous implant are provided including obtaining a 3-D image of an intended tissue repair site; generating a 3-D digital model of the porous implant based on the 3-D image of the intended tissue repair site. The method also includes determining an implant material and an amount of a porogen to add to an implant material to obtain a desired porosity of the porous implant. The desired porosity is based on a combination of macropores, micropores and/or nanopores structures. The 3-D digital model developed is stored on a database coupled to a processor, wherein the processor has instructions for combining the implant material with the porogen based on the stored 3-D digital model and for instructing a 3-D printer to produce the porous implant. A layered 3-D printed porous implant prepared by the computer implemented method is also provided.

## Description

### BACKGROUND

Three-dimensional (3-D) printing is an additive printing process used to make three-dimensional solid objects from a digital model. 3-D printing techniques are considered additive processes because they involve the application of successive layers of material.

3-D printing technology is applied in various industries for manufacturing and planning. For example, the automotive, aerospace and consumer goods industries use 3-D printing to create prototypes of parts and products. 3-D printing has also been used in the architectural industry for printing structural models. The applications of 3-D printing in private and government defense have grown rapidly as well.

3-D printing has had a significant impact in the medical fields. Medical applications have been used to make dental implants and prosthetics. 3-D printing has also been used in the fabrication of drug delivery devices that can be used for direct treatment. A variety of drug delivery devices may be created which allow for customizable drug release profiles.

Traditional 3-D printing allows an object to be created by depositing a material over a flat fabrication platform one layer at a time. Once a first layer is deposited, a second layer is deposited on top of the first layer. The process is repeated as necessary to create a multi-layered solid object. However, 3-D printing does not allow for continuous extrusion to create an object.

Bone defects may be caused by a number of different factors, including but not limited to trauma, pathological disease or surgical intervention. Because bone provides both stability and protection to an organism, these defects can be problematic. In order to address these defects, compositions that contain both natural and synthetic materials have been developed. These compositions may, depending upon the materials contained within them, be used to repair tissues and to impart desirable biological and/or mechanical properties to the bone defect.

Among the known bone repair materials and bone void fillers is autologous cancellous bone. This type of bone has the advantage of being both osteoinductive and non-immunogenic. Unfortunately, this type of bone is not available under all circumstances. Moreover, donor site morbidity and trauma add to the limitations of autologous cancellous bone.

In order to avoid the issues that attach to the use of autologous cancellous bone, one may use synthetic materials. However, known synthetic materials suffer from one or more of the following drawbacks, including unacceptable workability, handling and setting parameters; insufficient density; undesirable absorption rates; and an inability to impart adequate stability.

Generally, bone tissue regeneration is achieved by filling a bone repair site with a bone graft. Over time, the bone graft is incorporated by the host and new bone remodels the bone graft. In order to place the bone graft, it is common to use a monolithic bone graft or to form an osteoimplant comprising particulated bone in a carrier. The carrier material is thus chosen to be biocompatible, to be resorbable, and to have release characteristics such that the bone graft is accessible. Ordinarily, the formed implant, whether monolithic or particulated and in a carrier, is substantially solid at the time of implantation and, thus may not easily conform to the implant site. Further, the implant is substantially complete at the time of implantation and thus provides little ability for customization, for example, by the addition or subtraction of autograft material.

Traditional methods of 3D printing do not allow for producing a porous implant by controlling the amount of a porogen to add to the implant during manufacturing to form the desired macropores, micropores, and/or nanopores in the implant that aid in influx and efflux of cells to repair the damaged tissue. Thus, there is a need for a computer implemented method of producing a porous implant having the desired macropores, micropores, and/or nanopores in the implant for insertion into a tissue repair site that aids in influx and efflux of cells to repair the damaged tissue. There is also need for a computer system that can be used to implement the steps required to produce the porous implant having the desired macropores, micropores, and/or nanopores in the implant.

### SUMMARY

Provided is a computer implemented method for producing a porous implant having the desired macropores, micropores, and/or nanopores in the implant for insertion into a tissue repair site that aids in influx and efflux of cells to repair the damaged tissue. The method comprises obtaining a 3-D image of an intended tissue repair site; generating a 3-D digital model of the porous implant based on the 3-D image of the intended tissue repair site, the 3-D digital model of the porous implant being configured to fit within the tissue repair site. The method also includes determining an amount of a porogen to add to an implant material to obtain a desired porosity for the porous implant. The desired porosity is based on a plurality of macropores, micropores, nanopores structures or a combination thereof. The 3-D digital model thus developed is stored on a database coupled to a processor, wherein the processor has instructions for combining the implant material with the porogen based on the stored 3-D digital model and for instructing a 3-D printer to produce the porous implant.

In another embodiment, the computer implemented method for producing a porous implant described above further comprises removing the porogen from the porous implant.

According to other aspects, provided is a layered 3-D printed porous implant. In some embodiments, the layered porous implant comprises a first layer of implant material; a second layer of porogen disposed on the first layer of implant material; a third layer of implant material disposed on the second layer, each layer repeating until a 3-D printer has completed the porous implant. In another embodiment, the layered porous implant comprises a first layer of implant material mixed with porogen; a second layer of implant material mixed with porogen, the second layer disposed on the first layer; a third layer of implant material mixed with porogen, the third layer disposed on the second layer, each layer repeating until the 3-D printer has completed the porous implant.

According to other embodiments, provided is a method of treating a bone defect in a patient in need thereof by implanting the porous implant in the bone defect. In certain aspects, the method comprises administering a layered 3-D printed porous implant which comprises a first layer of implant material; a second layer of porogen disposed on the first layer of implant material; a third layer of implant material disposed on the second layer, each layer repeating until a 3-D printer has completed the porous implant. In other aspects, the method of treatment includes administering a layered 3-D porous implant which comprises a first layer of implant material mixed with porogen; a second layer of implant material mixed with porogen, the second layer disposed on the first layer; a third layer of implant material mixed with porogen, the third layer disposed on the second layer, each layer repeating until the 3-D printer has completed the porous implant.

While multiple embodiments are disclosed, still other embodiments of the present application will become apparent to those skilled in the art from the following detailed description, which is to be read in connection with the accompanying drawings. As will be apparent, the present disclosure is capable of modifications in various obvious aspects, all without departing from the scope of the present disclosure. Accordingly, the detailed description is to be regarded as illustrative in nature and not restrictive.

### BRIEF DESCRIPTION OF THE FIGURES

In part, other aspects, features, benefits and advantages of the embodiments will be apparent regarding the following description, appended claims and accompanying drawings.
FIG. 1 illustrates a perspective view of an exemplary 3-D printing device according to an aspect of the present application. The 3-D printing device includes a rotatable printing surface to facilitate continuous extrusion of a predetermined porous hollow implant;
FIG. 2 illustrates a perspective view of components of an exemplary 3-D printing device according to an aspect of the present application. Specifically, shown is a printing surface having a cylindrical shape configured to create a cylindrically shaped hollow structure that is a porous implant, such as a mesh bag. The printing surface is adjacent to and/or contacts a print head;
FIG. 3 illustrates a perspective view of components of an exemplary 3-D printing device according to an aspect of the present application. Specifically, shown is a printing surface having a rectangular cross section configured to create a rectangular or square shaped hollow structure that is a porous implant, such as a mesh bag;
FIG. 4 illustrates a perspective view of an exemplary hollow structure created through use of a 3-D printing device, according to an aspect of the present application. The depicted hollow structure includes a rectangular cross section;
FIG. 5 illustrates a perspective view of components of an exemplary 3-D printing device according to an aspect of the present application. Specifically, shown is the movement of a printing surface while a print head, such as, for example, an applicator continuously extrudes material to the surface to form a mesh pattern;
FIG. 5A illustrates a perspective view of a porous implant such as a mesh bag having a hollow interior region formed from a 3-D printing device according to an aspect of the present application;
FIG. 5B illustrates a perspective view of a mesh bag as in FIG. 5A containing an osteogenic material in the hollow interior region or compartment;
FIG. 6 illustrates a side view of components of an exemplary 3-D printing device according to an aspect of the present application. Specifically, shown is a print head which processes material to be extruded to the printing surface;
FIG. 7 illustrates a side view of components of an exemplary 3-D printing device according to an aspect of the present application. Specifically, shown is a radiation source, such as, for example, a laser mounted adjacent the print head to apply an energy to sinter or melt the material discharged from the print head;
FIG. 8 illustrates an embodiment of a computer-implemented system for producing a hollow structure, such as a mesh bag;
FIG. 9 is a flow diagram illustrating an embodiment of the computer-implemented system for producing a hollow structure, such as a mesh bag;
FIG. 10 is a flow diagram illustrating an embodiment of a system for producing a hollow structure, such as a mesh implant or bag, through the use of a 3-D printing machine having a rotating printing surface;
FIG. 11 is a flow diagram illustrating representative steps for producing a porous implant according to an embodiment of this application; and
FIG. 12 is a flow diagram illustrating representative steps for producing a porous implant according to another embodiment of this application.

It is to be understood that the figures are not drawn to scale. Further, the relation between objects in a figure may not be to scale, and may in fact have a reverse relationship as to size. The figures are intended to bring understanding and clarity to the structure of each object shown, and thus, some features may be exaggerated in order to illustrate a specific feature of a structure.

### DETAILED DESCRIPTION

### Definitions

For the purposes of this specification and appended claims, unless otherwise indicated, all numbers expressing quantities of ingredients, percentages or proportions of materials, reaction conditions, and other numerical values used in the specification and claims, are to be understood as being modified in all instances by the term "about." Similarly, when values are expressed as approximations, by use of the antecedent "about," it will be understood that the particular value forms another embodiment that is +/- 10% of the recited value. Accordingly, unless indicated to the contrary, the numerical parameters set forth in the following specification and attached claims are approximations that may vary depending upon the desired properties sought to be obtained by the present disclosure. At the very least, and not as an attempt to limit the application of the doctrine of equivalents to the scope of the claims, each numerical parameter should at least be construed in light of the number of reported significant digits and by applying ordinary rounding techniques. Also, as used in the specification and including the appended claims, the singular forms "a," "an," and "the" include the plural, and reference to a particular numerical value includes at least that particular value, unless the context clearly dictates otherwise. Ranges may be expressed herein as from "about" or "approximately" one particular value and/or to "about" or "approximately" another particular value. When such a range is expressed, another embodiment includes from the one particular value and/or to the other particular value.

Notwithstanding that the numerical ranges and parameters setting forth the broad scope of this application are approximations, the numerical values set forth in the specific examples are reported as precisely as possible. Any numerical value, however, inherently contains certain errors necessarily resulting from the standard deviation found in their respective testing measurements. Moreover, all ranges disclosed herein are to be understood to encompass any and all subranges subsumed therein. For example, a range of "1 to 10" includes any and all subranges between (and including) the minimum value of 1 and the maximum value of 10, that is, any and all subranges having a minimum value of equal to or greater than 1 and a maximum value of equal to or less than 10, for example, 5.5 to 10.

Allograft, as used herein, refers to a graft of tissue obtained from a donor of the same species as, but with a different genetic make-up from the recipient, as a tissue transplant between two humans.

Bioactive agent or bioactive compound is used herein to refer to a compound or entity that alters, inhibits, activates, or otherwise affects biological or chemical events. For example, bioactive agents may include, but are not limited to, osteogenic or chondrogenic proteins or peptides, anti-AIDS substances, anti-cancer substances, antibiotics, immunosuppressants, anti-viral substances, enzyme inhibitors, hormones, neurotoxins, opioids, hypnotics, anti-histamines, lubricants, tranquilizers, anti-convulsants, muscle relaxants and anti-Parkinson substances, anti-spasmodics and muscle contractants including channel blockers, miotics and anti-cholinergics, anti-glaucoma compounds, anti-parasite and/or anti-protozoal compounds, modulators of cell-extracellular matrix interactions including cell growth inhibitors and antiadhesion molecules, vasodilating agents, inhibitors of DNA, RNA or protein synthesis, anti-hypertensives, analgesics, anti-pyretics, steroidal and non-steroidal anti-inflammatory agents, anti-angiogenic factors, angiogenic factors, anti-secretory factors, anticoagulants and/or antithrombotic agents, local anesthetics, ophthalmics, prostaglandins, anti-depressants, anti-psychotic substances, anti-emetics, and imaging agents. In certain embodiments, the bioactive agent is a drug. Bioactive agents further include RNAs, such as siRNA, and osteoclast stimulating factors. In some embodiments, the bioactive agent may be a factor that stops, removes, or reduces the activity of bone growth inhibitors. In some embodiments, the bioactive agent is a growth factor, cytokine, extracellular matrix molecule or a fragment or derivative thereof, for example, a cell attachment sequence such as RGD. A more complete listing of bioactive agents and specific drugs suitable for use in the present application may be found in Pharmaceutical Substances: Syntheses, Patents, Applications by Axel Kleemann and Jurgen Engel, Thieme Medical Publishing, 1999; Merck Index: An Encyclopedia of Chemicals, Drugs, and Biologicals, edited by Susan Budavari et al., CRC Press, 1996; and United States Pharmacopeia-25/National Formulary-20, published by the United States Pharmacopeia Convention, Inc., Rockville Md., 2001, each of which is incorporated herein by reference. In some embodiments, the porous implant can contain a bioactive agent.

Biocompatible, as used herein, is intended to describe materials that, upon administration *in vivo,* do not induce undesirable long-term effects.

Biodegradable includes compounds or components that will degrade over time by the action of enzymes, by hydrolytic action and/or by other similar mechanisms in the human body. In various embodiments, "biodegradable" includes that components can break down or degrade within the body to non-toxic components as cells (e.g., bone cells) infiltrate the components and allow repair of the defect. By "biodegradable" it is meant that the compounds or components will erode or degrade over time due, at least in part, to contact with substances found in the surrounding tissue, fluids or by cellular action. By "bioabsorbable" it is meant that the compounds or components will be broken down and absorbed within the human body, for example, by a cell or tissue. "Biocompatible" means that the compounds or components will not cause substantial tissue irritation or necrosis at the target tissue site and/or will not be carcinogenic.

Bone, as used herein, refers to bone that is cortical, cancellous or cortico-cancellous of autogenous, allogenic, xenogenic, or transgenic origin.

Bone graft, as used herein, refers to any implant prepared in accordance with the embodiments described herein and therefore may include expressions such as bone material and bone membrane.

Demineralized, as used herein, refers to any material generated by removing mineral material from tissue, for example, bone tissue. In certain embodiments, demineralized bone material may be added to the implant. The demineralized bone material described herein includes preparations containing less than 5%, 4%, 3%, 2% or 1% calcium by weight. Partially demineralized bone (for example, preparations with greater than 5% calcium by weight but containing less than 100% of the original starting amount of calcium) is also considered within the scope of the disclosure. In some embodiments, partially demineralized bone contains preparations with greater than 5%, 10%, 15%, 20%, 25%, 30%, 35%, 40%, 45%, 50%, 55%, 60%, 65%, 70%, 75%, 80%, 85%, 90%, 95%, 96%, 97%, 98% or 99% of the original starting amount of calcium. In some embodiments, demineralized bone has less than 95% of its original mineral content. In some embodiments, demineralized bone has less than 95%, 90%, 85%, 80%, 75%, 70%, 65%, 60%, 55%, 50%, 45%, 40%, 35%, 30%, 25%, 20%, 15%, 10%, or 5% of its original mineral content. Demineralized is intended to encompass such expressions as "substantially demineralized," "partially demineralized," "superficially demineralized," and "fully demineralized." In some embodiments, part or the entire surface of the bone can be demineralized. For example, part or the entire surface of the bone material can be demineralized to a depth of from about 100 to about 5000 microns, or about 150 microns to about 1000 microns. In some embodiments, part or all of the surface of the bone material can be demineralized to a depth of from about 100, 150, 200, 250, 300, 350, 400, 450, 500, 550, 600, 650, 700, 750, 800, 850, 900, 950, 1000, 1050, 1100, 1150, 1200, 1250, 1300, 1350, 1400, 1450, 1500, 1550, 1600, 1650, 1700, 1750, 1800, 1850, 1900, 1950, 2000, 2050, 2100, 2150, 2200, 2250, 2300, 2350, 2400, 2450, 2500, 2550, 2600, 2650, 2700, 2750, 2800, 2850, 2900, 2950, 3000, 3050, 3100, 3150, 3200, 3250, 3300, 3350, 3400, 3450, 3500, 3550, 3600, 3650, 3700, 3750, 3800, 3850, 3900, 3950, 4000, 4050, 4100, 4150, 4200, 4250, 4300, 4350, 4400, 4450, 4500, 4550, 4600, 4650, 4700, 4750, 4800, 4850, 4900, 4950 to about 5000 microns. If desired, the bone void filler can comprise demineralized material.

Partially demineralized bone is intended to refer to preparations with greater than 5% calcium by weight but containing less than 100% of the original starting amount of calcium. In some embodiments, partially demineralized bone comprises 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98 and/or 99% of the original starting amount of calcium.

In some embodiments, the demineralized bone may be surface demineralized from about 1-99%. In some embodiments, the demineralized bone is 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98 and/or 99% surface demineralized. In various embodiments, the demineralized bone may be surface demineralized from about 15-25%. In some embodiments, the demineralized bone is 15, 16, 17, 18, 19, 20, 21, 22, 23, 24 and/or 25% surface demineralized.

Demineralized bone matrix (DBM), as used herein, refers to any material generated by removing mineral material from bone tissue. In some embodiments, the DBM compositions as used herein include preparations containing less than 5% calcium and, in some embodiments, less than 1% calcium by weight. In some embodiments, the DBM compositions include preparations that contain less than 5, 4, 3, 2 and/or 1% calcium by weight. In other embodiments, the DBM compositions comprise partially demineralized bone (for example, preparations with greater than 5% calcium by weight but containing less than 100% of the original starting amount of calcium).

DBM preparations have been used for many years in orthopedic medicine to promote the formation of bone. For example, DBM has found use in the repair of fractures, in the fusion of vertebrae, in joint replacement surgery, and in treating bone destruction due to underlying disease such as a bone tumor. DBM has been shown to promote bone formation *in vivo* by osteoconductive and osteoinductive processes. The osteoinductive effect of implanted DBM compositions results from the presence of active growth factors present on the isolated collagen-based matrix. These factors include members of the TGF-R, IGF, and BMP protein families. Particular examples of osteoinductive factors include TGF-*B*, IGF-1, IGF-2, BMP-2, BMP-7, parathyroid hormone (PTH), and angiogenic factors. Other osteoinductive factors such as osteocalcin and osteopontin are also likely to be present in DBM preparations as well. There are also likely to be other unnamed or undiscovered osteoinductive factors present in DBM.

Superficially demineralized, as used herein, refers to bone-derived elements possessing at least about 90 weight percent of their original inorganic mineral content. In some embodiments, superficially demineralized contains at least about 90, 91, 92, 93, 94, 95, 96, 97, 98 and/or 99 weight percent of their original inorganic material. The expression "fully demineralized" as used herein refers to bone containing less than 8% of its original mineral context. In some embodiments, fully demineralized contains about less than 8, 7, 6, 5, 4, 3, 2 and/or 1% of its original mineral content.

The expression "average length to average thickness ratio" as applied to the DBM fibers of the present application means the ratio of the longest average dimension of the fiber (average length) to its shortest average dimension (average thickness). This is also referred to as the "aspect ratio" of the fiber.

Fibrous, as used herein, refers to bone elements whose average length to average thickness ratio or aspect ratio of the fiber is from about 50:1 to about 1000:1. In some embodiments, average length to average thickness ratio or aspect ratio of the fiber is from about 50:1, 75:1, 100:1, 125:1, 150:1, 175:1, 200:1, 225:1, 250:1, 275:1, 300:1, 325:1, 350:1, 375:1, 400:1, 425:1, 450:1, 475:1, 500:1, 525:1, 550:1, 575:1, 600:1, 625:1, 650:1, 675:1, 700:1, 725:1, 750:1, 775:1, 800:1, 825:1, 850:1, 875:1, 900:1, 925:1, 950:1, 975:1 and/or 1000:1. In overall appearance, the fibrous bone elements can be described as bone fibers, threads, narrow strips, or thin sheets. Often, where thin sheets are produced, their edges tend to curl up toward each other. The fibrous bone elements can be substantially linear in appearance or they can be coiled to resemble springs. In some embodiments, the bone fibers are of irregular shapes including, for example, linear, serpentine or curved shapes. The bone fibers are preferably demineralized however some of the original mineral content may be retained when desirable for a particular embodiment. In various embodiments, the bone fibers are mineralized. In some embodiments, the fibers are a combination of demineralized and mineralized.

Non-fibrous, as used herein, refers to elements that have an average width substantially larger than the average thickness of the fibrous bone element or aspect ratio of less than from about 50:1 to about 1000:1. The non-fibrous bone elements can be shaped in a substantially regular manner or specific configuration, for example, triangular prism, sphere, cube, cylinder and other regular shapes. By contrast, particles such as chips, shards, or powders possess irregular or random geometries. It should be understood that some variation in dimension will occur in the production of the elements of this application and elements demonstrating such variability in dimension are within the scope of this application and are intended to be understood herein as being within the boundaries established by the expressions "mostly irregular" and "mostly regular."

The bone implant devices and methods provided enhance bone growth by reducing the gaps that may exist between the DBM particles and reduce the distance for cells (for example, osteoclasts, osteoblasts, etc.) to travel throughout the device to allow those cells to receive an adequate osteoinductive signal as opposed to only along the surface of the device. In some embodiments, the device improves the fusion of adjacent interspinous processes.

Osteoconductive, as used herein, refers to the ability of a substance to serve as a template or substance along which bone may grow.

Osteogenic, as used herein, refers to materials containing living cells capable of differentiation into bone tissue.

Osteoinductive, as used herein, refers to the quality of being able to recruit cells from the host that have the potential to stimulate new bone formation. Any material that can induce the formation of ectopic bone in the soft tissue of an animal is considered osteoinductive. For example, most osteoinductive materials induce bone formation in athymic rats when assayed according to the method of Edwards et al., "Osteoinduction of Human Demineralized Bone: Characterization in a Rat Model," Clinical Orthopaedics & Rel. Res., 357:219-228, December 1998, incorporated herein by reference.

Osteoimplant is used herein in its broadest sense and is not intended to be limited to any particular shapes, sizes, configurations, compositions, or applications. Osteoimplant refers to any device or material for implantation that aids or augments bone formation or healing. An osteoimplant may include any material, such as allograft, xenograft, or synthetic material, used to promote or support bone healing. The osteoimplant may be homogeneous or heterogeneous. Osteoimplants are often applied at a bone defect site, e.g., one resulting from injury, defect brought about during the course of surgery, infection, malignancy, inflammation, or developmental malformation. Osteoimplants can be used in a variety of orthopedic, neurosurgical, dental, and oral and maxillofacial surgical procedures such as the repair of simple and compound fractures and non-unions, external, and internal fixations, joint reconstructions such as arthrodesis, general arthroplasty, deficit filling, disectomy, laminectomy, anterior cervical and thoracic operations, or spinal fusions.

Tissue, as used herein, includes soft tissue, muscle, ligaments, tendons, cartilage and/or bone unless specifically referred to otherwise.

Plasticizer, as used herein, refers to an additive that softens hard polymers or plastics. The plasticizer makes the polymer formable or flexible. Plasticizers are thought to work by embedding themselves between the chains of polymers, spacing them apart, and thus lowering the glass transition temperature. Preferably, the plasticizers used in the porous implant of this disclosure are non-toxic and biocompatible.

As used herein, the terms "polynucleotide", "nucleic acid", or "oligonucleotide" refer to a polymer of nucleotides. The terms "polynucleotide", "nucleic acid", and "oligonucleotide", may be used interchangeably. Typically, a polynucleotide comprises at least three nucleotides. DNAs and RNAs are exemplary polynucleotides. The polymer may include natural nucleosides (for example, adenosine, thymidine, guanosine, cytidine, uridine, deoxyadenosine, deoxythymidine, deoxyguanosine, and deoxycytidine), nucleoside analogs (for example, 2-aminoadenosine, 2-thiothymidine, inosine, pyrrolo-pyrimidine, 3-methyl adenosine, C5-propynylcytidine, C5-propynyluridine, C5-bromouridine, C5-fluorouridine, C5-iodouridine, C5-methylcytidine, 7-deazaadenosine, 7-deazaguanosine, 8-oxoadenosine, 8-oxoguanosine, O(6)-methylguanine, and 2-thiocytidine), chemically modified bases, biologically modified bases (for example, methylated bases), intercalated bases, modified sugars (for example, 2'-fluororibose, ribose, 2'-deoxyriboses, arabinose, and hexose), or modified phosphate groups (for example, phosphorothioates and 5'-N-phosphoramidite linkages). The polymer may also be a short strand of nucleic acids such as RNAi, siRNA, or shRNA.

As used herein, a "polypeptide", "peptide", or "protein" includes a string of at least three amino acids linked together by peptide bonds. The terms "polypeptide", "peptide", and "protein", may be used interchangeably. In some embodiments, peptides may contain only natural amino acids, although non-natural amino acids (for example, compounds that do not occur in nature but that can be incorporated into a polypeptide chain) and/or amino acid analogs as are known in the art may alternatively be employed. Also, one or more of the amino acids in a peptide may be modified, for example, by the addition of a chemical entity such as a carbohydrate group, a phosphate group, a farnesyl group, an isofarnesyl group, a fatty acid group, and a linker for conjugation, functionalization, or other modification. In one embodiment, the modifications of the peptide lead to a more stable peptide (for example, greater half-life in vivo). These modifications may include cyclization of the peptide, the incorporation of D-amino acids. None of the modifications should substantially interfere with the desired biological activity of the peptide.

The terms "polysaccharide" or "oligosaccharide", as used herein, refer to any polymer or oligomer of carbohydrate residues. The polymer or oligomer may consist of anywhere from two to hundreds to thousands of sugar units or more. "Oligosaccharide" generally refers to a relatively low molecular weight polymer, while "polysaccharide" typically refers to a higher molecular weight polymer. Polysaccharides may be purified from natural sources such as plants or may be synthesized de novo in the laboratory. Polysaccharides isolated from natural sources may be modified chemically to change their chemical or physical properties (e.g., reduced, oxidized, phosphorylated, cross-linked). Carbohydrate polymers or oligomers may include natural sugars (e.g., glucose, fructose, galactose, mannose, arabinose, ribose, xylose) and/or modified sugars (e.g., 2'-fluororibose, 2'-deoxyribose). Polysaccharides may also be either straight or branched. They may contain both natural and/or unnatural carbohydrate residues. The linkage between the residues may be the typical ether linkage found in nature or may be a linkage only available to synthetic chemists. Examples of polysaccharides include cellulose, maltin, maltose, starch, modified starch, dextran, poly(dextrose), and fructose. Glycosaminoglycans are also considered polysaccharides. Sugar alcohol, as used herein, refers to any polyol such as sorbitol, mannitol, xylitol, galactitol, erythritol, inositol, ribitol, dulcitol, adonitol, arabitol, dithioerythritol, dithiothreitol, glycerol, isomalt, and hydrogenated starch hydrolysates.

Porogen, as used herein, refers to a chemical compound that can be part of the porous implant or removed from the porous implant during or after manufacturing. Typically, the porogen diffuses, dissolves, and/or degrades to leave a pore in the porous implant. The porogen essentially reserves space in the implant while the implant is being molded but during or after manufacturing, the porogen diffuses, dissolves, or degrades, thereby inducing porosity into the implant. In this way, the porogen provides "latent pores." The porogen may also be leached out of the implant before implantation. This resulting porosity of the implant is thought to allow infiltration by cells, bone formation, bone remodeling, osteoinduction, osteoconduction, and/or faster degradation of the implant. A porogen may be a gas (e.g., carbon dioxide, nitrogen, or other inert gas), liquid (e.g., water, biological fluid), or solid. Porogens are typically water soluble such as salts, sugars, polysaccharides, water soluble small molecules, or a combination thereof. A porogen can also be natural or synthetic polymers that are water soluble or degrade quickly under physiological conditions. Exemplary polymers include poly(vinylpyrollidone), pullulan, poly(glycolide), poly(lactide), poly(lactide-co-glycolide), other polyesters, and starches.

The abbreviation "DLG" refers to poly(DL-lactide-co-glycolide).

The abbreviation "PDL" refers to poly(DL-lactide).

The abbreviation "PLG" refers to poly(L-lactide-co-glycolide).

The abbreviation "PCL" refers to polycaprolactone.

The abbreviation "DLCL" refers to poly(DL-lactide-co-caprolactone).

The abbreviation "LCL" refers to poly(L-lactide-co-caprolactone).

The abbreviation "PPG" refers to polyglycolide.

The abbreviation "PEG" refers to poly(ethylene glycol).

The abbreviation "PLGA" refers to poly(lactide-co-glycolide) also known as poly(lactic-co-glycolic acid), which are used interchangeably.

The abbreviation "PLA" refers to polylactide.

The abbreviation "PEA" refers to poly(ester)amides.

The abbreviation "POE" refers to poly(orthoester).

The terms "three-dimensional printing system," "three-dimensional printer," "printing," describe various solid freeform fabrication techniques for making three-dimensional articles or objects by selective deposition, jetting, fused deposition modeling, multijet modeling, and other additive manufacturing techniques that use a build material or ink to fabricate three-dimensional objects.

Reference will now be made in detail to certain embodiments of the invention, examples of which are illustrated in the accompanying drawings. While the invention will be described in conjunction with the illustrated embodiments, it will be understood that they are not intended to limit the invention to those embodiments. On the contrary, the invention is intended to cover all alternatives, modifications, and equivalents that may be included within the invention as defined by the appended claims.

### 3-D Printer Device

Provided is 3-D printing devices and methods of use for creating porous implants, such as mesh implants or bags. These porous implants have the desired macropores, micropores, and/or nanopores in the implants for insertion into tissue repair sites that aids in influx and efflux of cells to repair the damaged tissue. Also provided are 3-D printing devices including a rotatable printing surface to create such porous implants. Further provided are devices and methods for 3-D printing onto a rotatable printing surface by continuous extrusion instead of stratified layers. Additionally, provided are devices and methods for creating porous structures having a mesh design that are strong, flexible, stretchable and biocompatible.

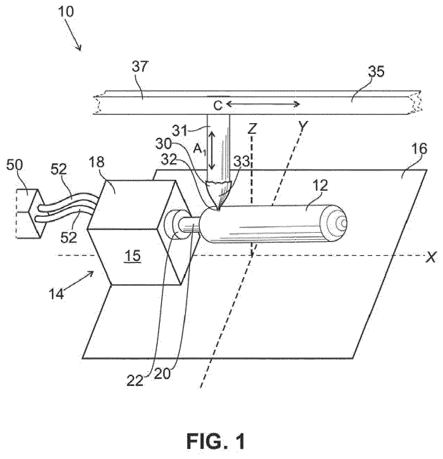

Turning now to FIGS. 1-7, provided is a 3-D printing device 10 for fabricating hollow structures, such as mesh bags 70. 3-D printing is typically done in 2 dimensions, one layer at a time. Material is laid out on a flat surface and the three-dimensional structures are built up one layer at a time, usually through a melting or sintering process. In some embodiments, a 3-D printer having a rotatable printing surface is provided to allow printing hollow structures, such as, for example, mesh bags 70. In some embodiments, a print head 30 applies material to the print surface through continuous extrusion instead of stratified layers, as is done by traditional 3-D printing devices. In some embodiments, 3-D printing device 10 creates stronger structures and generates less waste than traditional 3-D printing devices.

As shown in FIG. 1, provided is 3-D printing device 10 for use in the fabrication of mesh bags 70. 3-D printing device 10 includes a table 14 having a base 16 and a printing surface 12. In some embodiments, printing surface 12 is mounted onto table 14 including base 16. Base 16 is configured for planar movement. In some embodiments, base 16 is movable in the x-y plane and is laterally movable in both the x axis and the y axis for precise positioning of printing surface 12. Printing surface 12, in some embodiments, is fixedly disposed with table 14 such that lateral movement of base 16 causes lateral movement of printing surface 12. Movement of base 16 allows for positioning of printing surface 12 relative to print head 30 to facilitate depositing materials onto printing surface 12, as discussed herein.

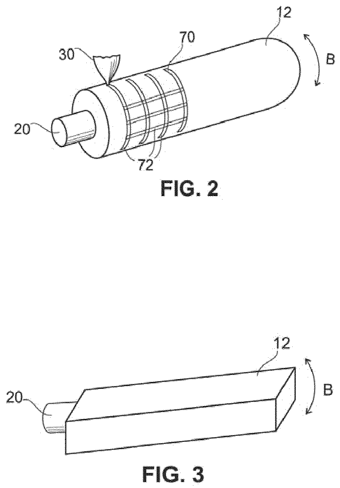

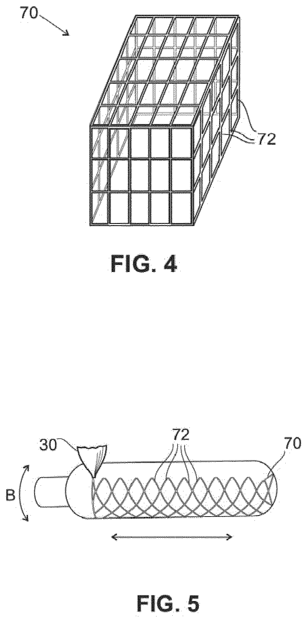

Printing surface 12 is rotatable about an axis of rotation, as shown in FIGS. 2 and 3. In some embodiments, rotating printing surface 12 includes a cylindrical shape extending along a longitudinal axis, as shown in FIG. 2. This allows printing of a round or circular implant with a hollow region as the implant takes on the shape of printing surface 12. In some embodiments, printing surface 12 includes other cross-sectional shapes, such as, for example, rectangular, oval, polygonal, irregular, undulating, or lobed. For example, as shown in FIG. 3, printing surface 12 may have a rectangular cross-section extending along a longitudinal axis. This allows printing of a square or rectangular implant, as printing surface 12 rotates, the implant will take the shape of printing surface 12. In alternative embodiments, printing surface 12 includes a uniform diameter and/or cross-section along its entire length. In other embodiments, printing surface 12 includes a changing diameter or cross-section along its length. For example, in some embodiments the diameter may increase from one end of printing surface 12 to the other. In some embodiments, the cross section of printing surface 12 changes from one end to the other. For example, one end of printing surface 12 may have a circular cross-section while the opposite end may have a rectangular cross-section. The size and shape of printing surface 12 may be changed according to the specifications and needs of a particular medical procedure. In some embodiments, mesh bags 70 are printed onto printing surface 12 into which another object, such as for example, bone material (e.g. surface demineralized bone chips and fully demineralized bone fibers), can be placed inside a hollow region or compartment. The shape of printing surface 12 defines the shape of the hollow structure created. As shown in FIG. 2, the shape of mesh implant or bag 70 created is cylindrical. As shown in FIG. 4, the shape of mesh implant or bag 70 created is that of a hollowed out rectangular prism.

Printing surface 12 is rotatable about a rotation of axis defined by extension shaft 20, as discussed herein. In various embodiments, printing surface 12 is rotatable in either clockwise or counterclockwise directions. In various embodiments, printing surface 12 is rotatable in both clockwise and counterclockwise directions, as shown by arrow B in FIGS. 2 and 3. Printing surface 12 is configured to change direction of rotation multiple times throughout the course of fabrication of a hollow structure, such as, for example, mesh implant or bag 70, as discussed herein. For example, printing surface 12 can rotate along a rotational axis 360 degrees clockwise and/or counterclockwise to print the implant.

In some embodiments, printing surface 12 is movable between an expanded configuration and a collapsed configuration. In some embodiments, a material 40 (which can be a biodegradable polymer) is deposited onto printing surface 12 while in the expanded configuration, and printing surface 12 is moved to the collapsed configuration to remove the printed hollow structure. Print head 30 can contact printing surface 12 or there can be a gap between printing surface 12 and print head 30 so that material 40 can be printed on printing surface 12.

In some embodiments, printing surface 12 is fixedly disposed with table 14 via a mounting bracket 18. Mounting bracket 18 may include covering 15 for protection. In some embodiments, mounting bracket 18 includes a motor to provide a rotational force to move printing surface 12. In some embodiments, mounting bracket 18 is connected to extension shaft 20. Printing surface 12 is connected to extension shaft 20 at a first end of printing surface 12. Extension shaft 20 defines an axis of rotation for printing surface 12 and is connected to mounting bracket 18 via collet 22. In some embodiments, collet 22 is expandable to loosen the grip on extension shaft 20. This allows extension shaft 20 and printing surface 12 to be changed out for another printing surface 12 which may be sized and/or shaped differently to cater to the needs of a particular procedure.

In some embodiments, 3-D printing device 10 further includes print head 30, such as, for example, an applicator that is movable in a direction transverse to the plane of movement for base 16. In some embodiments, print head 30 is movable in the z axis, as shown by arrow A₁ in FIG. 1, to allow for different size fixtures, variable surface structures and to control the thickness of the extruded layer. Thus, print head 30 is movable to have an adjustable distance from printing surface 12. Additionally, print head 30 is movable to accommodate printing surfaces having various diameters or printing surfaces having gradient diameters. In some embodiments, print head 30 is also movable in the x and y planes parallel with the plane of movement for base 16. Thus, in some embodiments, print head 30 is movable in an opposite direction from the movement of printing surface 12 to facilitate faster printing. In some embodiments, print head 30 is suspended from a track 35. Track 35 provides a base of support for print head 30. In some embodiments, track 35 provides a predefined route of allowable movement for print head 30 in directions shown as arrow C. In some embodiments, track 35 is hollow to allow flow of material 40 to be delivered to printing surface 12, as described herein.

In some embodiments, printing surface 12 is treated with an adhesive material. The adhesive material may be textured or coated onto printing surface 12. The adhesive may be heat sensitive or heat activated such that printing surface 12 becomes adhesive to material 40 when printing surface 12 is heated, as discussed herein. An adhesive coating aids in preventing printed material 40 from falling off printing surface 12 during rotation. In some embodiments, the adhesive is deactivated through cooling. In some embodiments, the adhesive may be removed by placing printing surface 12 in a solvent to dissolve the adhesive material. Once the adhesive material is removed, a hollow structure printed to printing surface 12 may be removed.

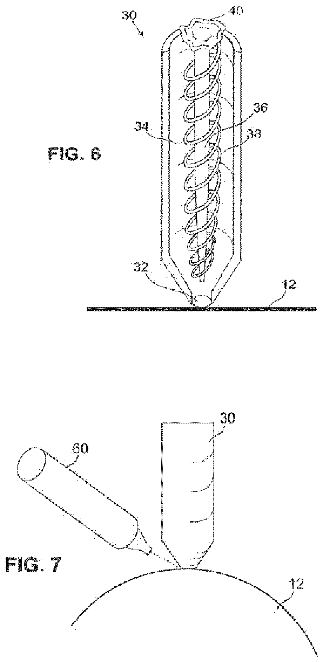

As shown in FIGS. 1 and 6, print head 30 includes a distal opening 32 through which material 40 is deposited on printing surface 12. A tube portion 31 of print head 30 includes a first diameter and extends distally to a head portion 33 having a second diameter. In some embodiments, the second diameter is smaller than the first diameter. In various embodiments, material 40 includes a biodegradable polymer. In some embodiments, material 40 comprises a bioerodible, a bioabsorbable, and/or a biodegradable biopolymer. Examples of suitable biopolymers include but are not limited to poly (alpha-hydroxy acids), poly (lactide-co-glycolide) (PLGA), polylactide (PLA), polyglycolide (PG), polyethylene glycol (PEG), conjugates of poly (alpha-hydroxy acids), poly(orthoester)s (POE), polyaspirins, polyphosphagenes, collagen, starch, pre-gelatinized starch, hyaluronic acid, chitosans, gelatin, alginates, albumin, fibrin, vitamin E compounds, such as alpha tocopheryl acetate, d-alpha tocopheryl succinate, D,L-lactide, or L-lactide, caprolactone, dextrans, vinylpyrrolidone, polyvinyl alcohol (PVA), PVA-g-PLGA, PEGT-PBT copolymer (polyactive), PEO-PPO-PAA copolymers, PLGA-PEO-PLGA, PEG-PLG, PLA-PLGA, poloxamer 407, PEG-PLGA-PEG triblock copolymers, SAIB (sucrose acetate isobutyrate) or combinations thereof. In various embodiments, material 40 comprises poly(lactide-co-glycolide) (PLGA), polylactide (PLA), polyglycolide (PGA), D-lactide, D,L-lactide, L-lactide, D,L-lactide-co-*s*-caprolactone, D,L-lactide-co-glycolide-co-s-caprolactone, L-lactide-co-s-caprolactone or a combination thereof.

Print head 30 includes an inner lumen 34 and a central feed shaft 36 as illustrated in FIG. 6. Feed shaft 36 is configured to turn feed threads 38 to feed material 40 from the proximal end of print head 30 through opening 32. Material 40 is maintained in an external reservoir (not shown) and fed into lumen 34. In some embodiments, material 40 is driven into lumen 34 by gravity. In some embodiments material 40 is drawn into lumen 34 by turning feed shaft 36 and feed threads 38. In some embodiments, 3-D printing device 10 includes multiple print heads 30, each configured to deposit material 40 onto printing surface 12.

In some embodiments, as illustrated in FIG. 1, 3-D printing device 10 further includes a temperature control unit 50 such as, for example, a heating or cooling unit connected to printing surface 12. In some embodiments, temperature control unit 50 includes a heating unit. In other embodiments, temperature control unit 50 includes a cooling unit. In some embodiments, temperature control unit 50 is used to heat printing surface 12 through electric heating elements underneath the surface of printing surface 12. Sufficient energy may be supplied through such electric conduits to provide a temperature on the surface of printing surface 12 to melt and bond material 40 applied from print head 30. In such an embodiment, as illustrated in FIG. 1, conduits 52 are electric heating conduits. In some embodiments, where material 40 comprises a highly viscous material, a heated printing surface 12 allows material 40 to flow. In other embodiments, material 40 is heated or cooled in a reservoir 37 to allow the desired flowability or viscosity of material 40 to make the implant.

In some embodiments, temperature control unit 50 comprises a cooling unit. The cooling unit is used to cool printing surface 12 through refrigerant supply and return lines underneath printing surface 12. In such an embodiment, the supply and return lines are conduits 52. Conduits 52 supply cooling fluid to printing surface 12 to cool and solidify hot material 40 extruded onto the surface. In alternative embodiments, reservoir 37 can have the cooling and heating unit to allow cooling or heating of material 40.

According to some aspects, 3-D printing device 10 includes a radiation source configured to supply and transfer energy to at least a portion of the polymer, which can be in powder form applied to the surface. In some embodiments, the radiation source is a laser 60 positioned adjacent print head 30. Laser 60 articulates such that the supplied beam can be focused on selected portions of printing surface 12. As shown in FIG. 7, laser 60 is configured to be used during or after print head 30 deposits material 40 onto printing surface 12. The beam of laser 60 is focused onto portions of material 40 on printing surface 12 to melt or sinter material 40 as desired. Once the printed hollow structure is complete, it may be removed from the residual powdered material 40 left on printing surface 12, or the residual powdered material 40 is brushed away. In some embodiments, the laser is focused at a point adjacent opening 32 to sinter material 40 as it is deposited onto printing surface 12. Such embodiments may facilitate the elimination of waste since the majority of material 40 extruded onto printing surface 12 is sintered.

In some embodiments, laser 60 may include any wavelength of visible light or UV light. In some embodiments, laser 60 emits alternative forms of radiation, such as, for example, microwave, ultrasound or radio frequency radiation. In some embodiments, laser 60 is configured to be focused on a portion of printing surface 12 to sinter material 40 deposited thereon. Laser 60 may be emitted in a beam having a small diameter. For example, the diameter of the beam may be between about 0.01 mm and about 0.8 mm. In some embodiments, the diameter of the beam may be between about 0.1 mm and about 0.4 mm. In some embodiments, the diameter of the beam is adjustable to customize the intensity of the sintering. In some embodiments, material 40 is deposited on printing surface 12 and print head 30 removes by, for example, heating material 40 to remove unwanted material 40 from printing surface 12 to make the implant. Material 40 remaining on printing surface 12 after removal of the unwanted material 40 will be the implant. The material can include a polymer, a porogen or a combination thereof. These can be in separate print heads or combined in one print head.

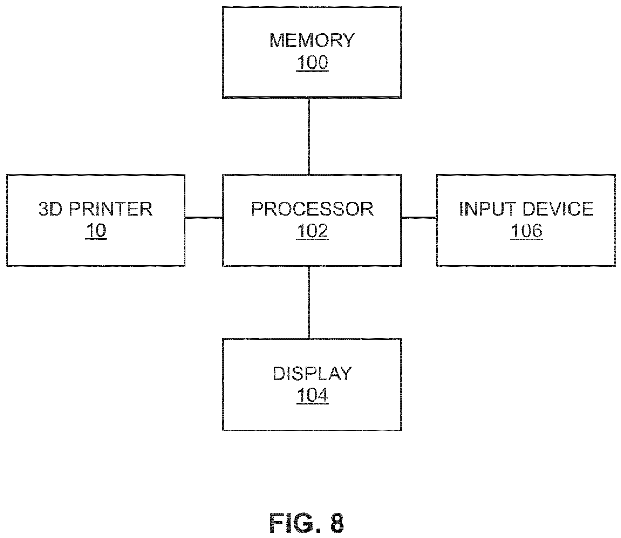

In other aspects, as illustrated in FIG. 8, 3-D printing device 10 includes a controller or processor 102 to accept instructions and automatically manufacture a hollow structure, such as, for example, a mesh implant or bag 70, based on the instructions. In some embodiments, processor 102 comprises memory 100 for temporary or permanent storage of instructions. Various instructions may be programmed and stored in memory 100 to make multiple designs of mesh implant or bag 70. In some embodiments, 3-D printing device 10 includes an input device 106, such as, for example, a keyboard to input commands and instructions. In some embodiments, processor 102 of 3-D printing device 10 is configured to receive commands and instructions from an external computer. For example, various instructions may be stored and executed locally on an external computer to operate 3-D printing device 10. In some embodiments, the computer and the 3-D printing device can be one single device with component parts.

In some embodiments, processor 102 comprises logic to execute one or more instructions to carry out instructions of the computer system (for example, transmit instructions to the 3-D printer, etc.). The logic for executing instructions may be encoded in one or more tangible media for execution by processor 102. For example, processor 102 may execute codes stored in a computer-readable medium such as memory 100. The computer-readable medium may be stored in, for example, electronic (for example, RAM (random access memory), ROM (read-only memory), EPROM (erasable programmable read-only memory), magnetic, optical (for example, CD (compact disc), DVD (digital video disc)), electromagnetic, semiconductor technology, or any other suitable medium. The computer includes logic to calculate the desired amount of porogen to add to form the porous implant based on the 3D digital model of the porous implant.

In some embodiments, the instructions include dimensions of a mesh implant or bag 70 to be made. For example, the instructions may include programming as to the length and thickness of the mesh implant or bag 70. Processor 102 carries out the instructions by causing movement of base 16 relative to print head 30 while material 40 is applied to printing surface 12. Additionally, processor 102 may cause movement of print head 30 in a direction away from printing surface 12 to allow for a thicker layer of material 40, according to the predetermined specifications in the instructions. In some embodiments, processor 102 is configured to provide a single layer of material 40 to make mesh implant or bag 70. The layer of material 40 deposited onto printing surface 12 may have uniform thicknesses or may include varied thicknesses, such as thickness gradients across the length of mesh implant or bag 70. In some embodiments, the dimensions of mesh implant or bag 70 may range from about 1 cm to about 1 meter in length, or from about 3 cm to about 8 cm in length, from about 2 mm to about 30 mm in thickness, or from about 2 mm to about 10 mm in thickness, and from about 2 mm to about 30 mm in width, or from about 2 mm to about 10 mm in width. The computer includes the processor with logic to calculate the desired amount of porogen to add to form the porous implant based on the 3D digital model of the porous implant.

Once processor 102 receives the instructions, processor 102 directs 3-D printing device 10 to make mesh implant or bag 70 based on the received instructions. In some embodiments, processor 102 directs the lateral movement of base 16 and printing surface 12, and the movement of print head 30 transverse to base 16 and printing surface 12. In some embodiments, processor 102 also controls the direction of rotation, the degree of rotation and the speed of rotation of printing surface 12. In some embodiments, processor 102 moves, focuses and directs laser 60 to emit radiation at a predetermined point on printing surface 12. In some embodiments, processor 102 directs temperature control unit 50 to heat or cool printing surface 12. Based on the instructions received, processor 102 coordinates simultaneous and/or ordered movement of base 16, printing surface 12, and print head 30 relative to one another. Processor 102 also controls the application of material 40 onto printing surface 12. For example, processor 102 directs the pressure at which material 40 is released onto printing surface 12. Processor 102 also directs the patterns of application onto printing surface 12, including portions where material 40 is not applied to printing surface 12 to reduce waste. Processor 102 may also direct laser 60 to emit radiation, such as for example, focused beams of light, in controlled pulses to sinter preselected portions of material 40 on printing surface 12.

In some embodiments, processor 102 directs motors which control the movement and rotation of at least base 16, printing surface 12, and print head 30 relative to one another. In some embodiments, processor 102 directs coarse and/or fine movement of components of 3-D printing device 10.

Although the components of the system of FIG. 8 are shown as separate, they may be combined in one or more computer systems. Indeed, they may be one or more hardware, software, or hybrid components residing in (or distributed among) one or more local or remote computer systems. It also should be readily apparent that the components of the system as described herein may be merely logical constructs or routines that are implemented as physical components combined or further separated into a variety of different components, sharing different resources (including processing units, memory, clock devices, software routines, logic commands, etc.) as required for the particular implementation of the embodiments disclosed. Indeed, even a single general purpose computer (or other processor-controlled device) executing a program stored on an article of manufacture (for example, recording medium or other memory units) to produce the functionality referred to herein may be utilized to implement the illustrated embodiments. It also will be understood that the plurality of computers or servers can be used to allow the system to be a network based system having a plurality of computers linked to each other over the network, Wi-Fi or Internet or the plurality of computers can be connected to each other to transmit, edit, and receive data via cloud computers or in a data drop box.

The computer (for example, memory, processor, storage component, etc.) may be accessed by authorized users. Authorized users may include at least one engineer, technician, surgeon, physician, nurse, and/or health care provider, manufacturer, etc.

The user can interface with the computer via a user interface that may include one or more display devices 104 (for example, CRT, LCD, or other known displays) or other output devices (for example, printer, etc.), and one or more input devices (for example, keyboard, mouse, stylus, touch screen interface, or other known input mechanisms) for facilitating interaction of a user with the system via user interface. The user interface may be directly coupled to a database or directly coupled to a network server system via the Internet, WiFi or cloud computing. In accordance with one embodiment, one or more user interfaces are provided as part of (or in conjunction with) the illustrated systems to permit users to interact with the systems.

The user interface device may be implemented as a graphical user interface (GUI) containing a display 104 or the like, or may be a link to other user input/output devices known in the art. Individual ones of a plurality of devices (for example, network/stand-alone computers, personal digital assistants (PDAs), WebTV (or other Internet-only) terminals, set-top boxes, cellular phones, screen phones, pagers, blackberry, smart phones, iPhone, iPad, tablet, peer/non-peer technologies, kiosks, or other known (wired or wireless) communication devices, etc.) may similarly be used to execute one or more computer programs (for example, universal Internet browser programs, dedicated interface programs, etc.) to allow users to interface with the systems in the manner described. Database hardware and software can be developed for access by users through personal computers, mainframes, and other processor-based devices. Users may access and data stored locally on hard drives, CD-ROMs, stored on network storage devices through a local area network, or stored on remote database systems through one or more disparate network paths (for example, the Internet).

The database can be stored in storage devices or systems (for example, Random Access Memory (RAM), Read Only Memory (ROM), hard disk drive (HDD), floppy drive, zip drive, compact disk-ROM, DVD, bubble memory, flash drive, redundant array of independent disks (RAID), network accessible storage (NAS) systems, storage area network (SAN) systems, etc.). CAS (content addressed storage) may also be one or more memory devices embedded within a CPU, or shared with one or more of the other components, and may be deployed locally or remotely relative to one or more components interacting with the memory or one or more modules. The database may include data storage device, a collection component for collecting information from users or other computers into a centralized database, a tracking component for tracking information received and entered, a search component to search information in the database or other databases, a receiving component to receive a specific query from a user interface, and an accessing component to access centralized database. A receiving component is programmed for receiving a specific query from one of a plurality of users. The database may also include a processing component for searching and processing received queries against a data storage device containing a variety of information collected by a collection device.

The disclosed system may, in some embodiments, be a computer network based system. The computer network may take any wired/wireless form of known connective technology (for example, corporate or individual LAN, enterprise WAN, intranet, Internet, Virtual Private Network (VPN), combinations of network systems, etc.) to allow a server to provide local/remote information and control data to/from other locations (for example, other remote database servers, remote databases, network servers/user interfaces, etc.). In accordance with one embodiment, a network server may be serving one or more users over a collection of remote and disparate networks (for example, Internet, intranet, VPN, cable, special high-speed ISDN lines, etc.). The network may comprise one or more interfaces (for example, cards, adapters, ports) for receiving data, transmitting data to other network devices, and forwarding received data to internal components of the system (for example, 3-D printers, printer heads, etc.).

In accordance with one embodiment of the present application, the data may be downloaded in one or more textual/graphical formats (for example, RTF, PDF, TIFF, JPEG, STL, XML, XDFL, TXT etc.), or set for alternative delivery to one or more specified locations (for example, via e-mail, etc.) in any desired format (for example, print, storage on electronic media and/or computer readable storage media such as CD-ROM, etc.). The user may view the search results and underlying documents at the user interface, which allows viewing of one or more documents on the same display 104.

### Porous Mesh Formulations

In some embodiments, mesh implants or bags 70 are formed from material 40 extruded from print head 30. Mesh implants or bags 70 comprise a system of threads 72 which are extruded directly onto printing surface 12. Threads 72 may be extruded in various patterns, and may be sized according to the requirements of a particular application. For example, threads 72 may be extruded from print head 30 in a weave pattern in which threads 72 are interwoven with one another such that each thread 72 alternatingly interlaces above and below adjacent threads 72. In other embodiments, threads 72 may be extruded in other ways. For example, horizontal rows of threads 72 may be extruded in a first step, and in second step vertical rows of threads 72 may be extruded on top of the horizontal rows. A radiation source, such as laser 60 may be configured to sinter the extruded rows together to form a mesh implant or bag 70.

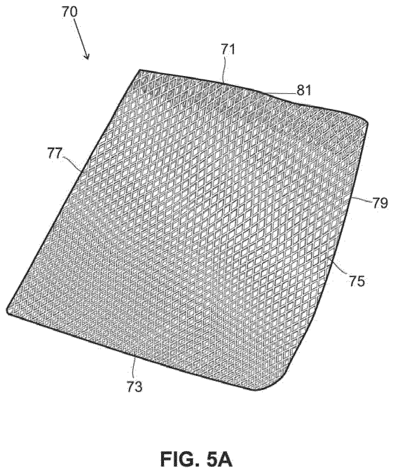

In some embodiments as shown in FIG. 5A, a completely printed mesh implant or bag 70 is formed having a continuous surface 75 formed from threads 72. Mesh implant or bag 70 includes oppositely positioned ends 77, 79. There is no seal at these ends as mesh implant or bag 70 was 3-D printed allowing for continuous manufacture. Mesh implants or bags 70 that are not manufactured by 3-D printing would have seals on three of the four corners of the bag. In one embodiment of the 3-D printed mesh implant or bag 70, a bottom end 73 of mesh implant or bag 70 is the only one sealed so that contents do not fall out. In other embodiments, an end 71 is open to allow placement of bone material in the hollow region or a compartment 81 of the mesh implant or bag 70. Opening 71 allows entrance into the hollow region or compartment 81 of mesh implant or bag 70, where bone material is placed inside of it; the implant is then placed at a bone defect and mesh implant or bag 70 allows the osteoinductive factors to leave mesh implant or bag 70 and allows influx of bone cells into mesh implant or bag 70. Mesh implant or bag 70 is porous so as to allow influx and efflux of material 40.

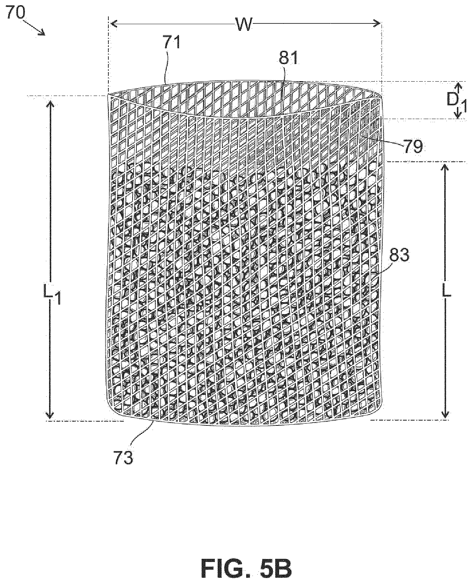

In FIG. 5B, the hollow region or compartment 81 of mesh implant or bag 70 is shown having opening 71. Mesh implant or bag 70 is filled manually by hand or via an automated process with bone particles 83 (for example, surface demineralized chips and fully demineralized fibers) for use to enhance bone growth. The computer system may have a sensor to determine the proper level of filing of the mesh implant with bone material.

In some embodiments, the dimensions of printing surface 12 allows for printing a mesh implant or bag 70 of different dimensions and shapes that correspond to printing surface 12 (for example, circular, rectangular, square, etc.). The rotation of printing surface 12 shown as arrow B in FIGS. 2 and 3, allows the implant (for example, mesh implant or bag 70) to be printed continuously so that there is a reduced need for sealing the hollow region of the implant. The computer system can calculate the proper volume, length, width, and thickness of the cover to match the volume, length, width, and thickness of the compartment and/or mesh implant or bag 70.

In some embodiments, mesh implant or bag 70 is flexible and can be packed flat and extending between oppositely positioned ends 77 and 79. In some embodiments, mesh implant or bag 70 forms a cylindrical shape between oppositely positioned ends 77 and 79.

Threads 72 may be configured to allow ingrowth of cells while also retaining the osteogenic material within the compartment of mesh implant or bag 70. In some embodiments, print head 30 is configured to extrude threads 72 having a predetermined thickness. In some embodiments, threads 72 have a thickness of about 0.01 mm to about 2.0 mm. In some embodiments, threads 72 have a thickness of about 0.05 mm to about 1.0 mm, or about 0.1 to about 0.5 mm. The thickness of threads 72 may be uniform along the length of each thread, or varied across the length of each thread. In some embodiments, some threads 72 have a greater thickness than other threads 72 in a mesh implant or bag 70. Threads 72 may be sized to allow for customizable pore sizes between threads 72. In some embodiments, porous mesh implant or bag 70 is configured to facilitate transfer of substances and/or materials surrounding the surgical site. Upon implantation to a surgical site, mesh implant or bag 70 may participate in, control, or otherwise adjust, or may allow penetration of mesh implant or bag 70 by surrounding materials, such as cells or tissue.

In various embodiments, mesh implant or bag 70 may be sized according to the needs of a particular application. For example, mesh bag or implant 70 may include dimensions between about 1 mm to about 100 mm in diameter shown as W in FIG. 5B. In some embodiments, mesh implant or bag 70 includes a diameter D1 as illustrated in FIG. 5B of about 5 mm, 10 mm, 15 mm, 20 mm, 25 mm, 30 mm, 35 mm, 40 mm, 45 mm, 50 mm, 55 mm, 60 mm, 65 mm, 70 mm, 75 mm, 80 mm, 85 mm, 90 mm, 95mm, or 100 mm. In some embodiments, mesh implant or bag 70 includes a length or depth from about 0.1 cm to about 10 cm, illustrated as L1 in FIG. 5B. In some embodiments, mesh implant or bag 70 includes a length or depth of about 1 cm, 2 cm, 3 cm, 4 cm, 5 cm, 6 cm, 7 cm, 8 cm, 9 cm, or 10 cm. The desired dimensions can be selected by the user and the computer system can print the implant according to the selection.

In various embodiments, based on the foregoing dimensions, the volume of a 3-D printed tubular shaped mesh bag can be easily calculated. For example, in some embodiments, a 3-D printed tubular mesh bag having a diameter of 0.5 cm and a length of 0.1 cm would provide a volume of 0.02 cc. In other embodiments, a 3-D printed tubular mesh bag having a diameter of 1 cm and a length of 1 cm would provide a volume of 0.79 cc. In yet other embodiments, a 3-D printed tubular mesh bag having a diameter of 1.5 cm and length of 3 cm would provide a volume of 5.3 cc.

In some embodiments, threads 72 are extruded onto printing surface 12 in a wave-like configuration having alternating peaks and crests. In some embodiments, printing surface 12 is rotated in alternating clockwise and counterclockwise directions while material 40 is extruded onto the surface to create sinusoidal shaped waves having evenly shaped curves on the peaks and crests. In some embodiments, the peaks and crests of the waves are pointed to impart variable characteristics to mesh implant or bag 70. In some embodiments, threads 72 are extruded adjacent to one another such that the peaks of a first thread 72 is extruded to contact the crest of an adjacent second strand 72. In some embodiments, mesh implant or bag 70 may be created entirely from threads 72 having this configuration. Wave-shaped threads 72 impart flexibility and stretchable characteristics onto the manufactured mesh implant or bag 70. The wavelength of the wave-shaped threads 72 may be altered to customize stretchability of mesh implant or bag 70. For example, threads 72 having shorter wavelengths will be able to be stretched more than threads 72 having longer wavelengths. In some embodiments, the stretchability of mesh implant or bag 70 is uniform across its length. In some embodiments, mesh implant or bag 70 includes regions of increased stretchability according to the needs of a surgical application.

The shape, mesh size, thickness, and other structural characteristics, of mesh implants or bags 70, for example, architecture, may be customized for the desired application. For example, to optimize cell or fluid migration through the mesh, the pore size may be optimized for the viscosity and surface tension of the fluid or the size of the cells. For example, pore sizes between threads 72 on the order of approximately 100-200 *µ*m may be used if cells are to migrate through the mesh. In other embodiments, the wave-shaped threads 72 may be extruded to have larger peaks and crests and the size of the pores may be larger. For example, in some embodiments, the pore size between threads 72 may be about 0.1 mm to about 5 mm, about 0.5 mm to about 3 mm, or about 1 mm to about 2 mm. Mesh size may be controlled by physically weaving threads and by controlling the thickness of threads 72 extruded and sintered on printing surface 12.

In various embodiments, mesh implant or bag 70 made by 3-D printing device 10 may have varying degrees of permeability across its surface. It may be permeable, semi-permeable, or non-permeable. Permeability may be with respect to cells, to liquids, to proteins, to growth factors, to bone morphogenetic proteins, or other. In further embodiments, material 40 may be braided.

Mesh implant or bag 70 may have any suitable configuration. For example, mesh implant or bag 70 may be 3-D printed onto a printing surface 12 having a variety of shapes, such as, for example, a ring, a cylinder, a cage, a rectangular shape, a suture-like wrap, a continuous tube, or other configurations. Printing surface 12 provides a scaffold onto which mesh implant or bag 70 is 3-D printed and from which mesh implant or bag 70 derives its shape. In specific embodiments, mesh implant or bag 70 may be formed as a thin tube designed to be inserted through catheters or an introducer tube; a rectangular shape designed to fit adjacent to spinal processes for posterolateral spine fusion; a cube; a rectangular prism like structure, as shown in FIG. 4, designed to fit between vertebral bodies or within cages for interbody spinal fusion; a tube-like shape; relatively flat shapes; rectangular shapes; structures pre-shaped to fit around various implants (e.g., dental, doughnut with hole for dental implants); or relatively elastic ring-like structures that will stretch and then conform to shapes (e.g. rubber band fitted around processes). In an embodiment wherein the mesh implant or bag is formed as a cage, the cage may comprise a plurality of crossed threads 72, which define between them a series of openings for tissue ingrowth. Any of these shapes may be used to contain osteogenic material such as bone material, as discussed herein. Mesh implant or bag 70 may be printed and sintered onto printing surface 12 in such a way as to have one open end, as shown in FIGS. 5A and 5B.

Additionally, the flexible character of the mesh material allows for the mesh implant or bag 70 to be manipulated into a plurality of compartments. For example, in a tubular embodiment, the tube may be formed into a plurality of compartments by tying a cord around the tube at one or more points, or by other suitable mechanism such as crimping, twisting, knotting, stapling, or sewing and also including 3-D printing based on a 3-D digital model as more particularly described in this application.

A suitable mesh implant or bag that can be made by the 3-D printing device of the current application is the MAGNIFUSE® Bone Graft, available from Medtronic, which comprises surface demineralized bone chips mixed with non-demineralized cortical bone fibers or fully demineralized bone fibers sealed in an absorbable poly(glycolic acid) (PGA) mesh implant or bag or pouch.

In certain embodiments, a bone void can be filled by mesh implant or bag 70 containing bone materials. A compartment within mesh implant or bag 70 can be at least partially filled with a bone repair substance. In various embodiments, at least partially filled as used herein, can mean that a percentage of the volume of a compartment or hollow interior region is at least 70% occupied, at least 75% occupied, at least 80% occupied, at least 85% occupied, at least 90% occupied, at least 95% occupied, or 100% occupied. In various embodiments, a sensing means or sensor in communication with the hollow compartment of mesh implant or bag 70 and also coupled to a computer processor can instruct the processor when a desired percentage volume of the compartment is occupied. The processor can then instruct the 3-D printer to generate a covering for enclosing the bone material within mesh implant or bag 70. Mesh implant or bag 70 can be inserted into an opening in the defect until the defect is substantially filled. In various embodiments, substantially filled, as used herein, can mean that a percentage of the volume of a defect is at least 70% occupied, at least 75% occupied, at least 80% occupied, at least 85% occupied, at least 90% occupied, at least 95% occupied, or 100% occupied.

In some embodiments, mesh implant or bag 70 may be labeled. Such labeling may be done in any suitable manner and at any suitable location on mesh implant or bag 70. In some embodiments, labeling may be done by using a silk screen printing, using an altered weaving or knotting pattern, by using different colored threads 72, or other means. The labeling may indicate information regarding mesh implant or bag 70. Such information might include a part number, donor ID number, number, lettering or wording indicating order of use in the procedure or implant size, etc.

In one embodiment, mesh implant or bag 70 may comprise a penetrable material at a first compartment configured for placement adjacent bone and a substantially impenetrable material at a second compartment configured for placement adjacent soft tissue. For example, the pore size between threads 72 at a first region of mesh implant or bag 70 may be sized large enough to allow cell migration through mesh implant or bag 70, but the pore size between threads 72 at a second region of mesh implant or bag 70 may be sized small enough (or may include a lack of pores altogether) to prevent cell migration. Alternatively, material 40 of the mesh implant or bag 70 may have a uniform configuration such that adjacent compartments may have substantially identical characteristics. By way of example only, mesh implant or bag 70 may have a porous surface that is positioned adjacent bone, and a separate or opposite surface that has a generally impenetrable surface that is positioned adjacent soft tissue. Alternatively, mesh implant or bag 70 may have one compartment that comprises a porous material, and a second compartment that comprises a substantially impenetrable material.

For either single and multi-compartment mesh implant or bags 70, the mesh implant or bag 70 may be closed after filling with substances. Accordingly, mesh implant or bag 70 may be provided in an unfilled, unsealed state immediately following fabrication with 3-D printing device 10. After a substance for delivery is placed in mesh implant or bag 70, mesh implant or bag 70 may be permanently or temporarily closed. Permanent closure may be, for example, by 3-D printing a covering for enclosing the bone material within compartment 81 of the mesh implant or bag 70. Temporary closure may be by tying, fold lock, cinching, or other means. A temporarily closed mesh implant or bag 70 can be opened without damaging mesh implant or bag 70 during surgical implantation to add or remove substances in mesh implant or bag 70.

Suitable adhesives for use for closing the mesh bag may include, for example, cyanoacrylates (such as histoacryl, B Braun, which is n-butyl-2 cyanoacrylate; or Dermabond, which is 2-octylcyanoacrylate); epoxy-based compounds, dental resin sealants, dental resin cements, glass ionomer cements, polymethyl methacrylate, gelatin-resorcinol-formaldehyde glues, collagen-based glues, inorganic bonding agents such as zinc phosphate, magnesium phosphate or other phosphate-based cements, zinc carboxylate, L-DOPA (3,4-dihydroxy-L-phenylalanine), proteins, carbohydrates, glycoproteins, mucopolysaccharides, other polysaccharides, hydrogels, protein-based binders such as fibrin glues and mussel-derived adhesive proteins, and any other suitable substance. Adhesives may be selected for use based on their bonding time; for example, in some circumstances, a temporary adhesive may be desirable, for example, for fixation during the surgical procedure and for a limited time thereafter, while in other circumstances a permanent adhesive may be desired. Where the compartment is made of a material that is resorbable, the adhesive can be selected that would adhere for about as long as the material is present in the body.

In some embodiments, biological attachment may be via mechanisms that promote tissue ingrowth such as by a porous coating or a hydroxyapatite-tricalcium phosphate (HA/TCP) coating. Generally, hydroxyapatite bonds by biological effects of new tissue formation. Porous ingrowth surfaces, such as titanium alloy materials in a beaded coating or tantalum porous metal or trabecular metal may be used and facilitate attachment at least by encouraging bone to grow through the porous implant surface. These mechanisms may be referred to as biological attachment mechanisms. In some embodiments, mesh implant or bag 70 may be attached to a tissue structure through a wrap, a suture, a wire, a string, an elastic band, a cable, a cable tie, or a combination thereof. In some embodiments the attachment mechanism can be (i) integral to the 3-D printed seamless biodegradable mesh implant or bag 70 or (ii) is provided separately from the 3-D printed seamless biodegradable mesh implant or bag 70 and can be attached to the 3-D printed seamless biodegradable mesh implant or bag 70 for use at an intended graft site.

In some embodiments, mesh implant or bag 70 comprises an extruded material 40 arranged in a mesh configuration. In some embodiments, material 40 of mesh implant or bag 70 is biodegradable. In some embodiments, mesh implant or bag 70 includes only one material 40 which is uniformly extruded to form the entirety of mesh implant or bag 70. In some embodiments, mesh implant or bag 70 comprises a blend of suitable materials 40. In some embodiments, a first group of threads 72 may comprise a first material 40 and a second group of threads 72 comprises a second material 40. In some embodiments, print head 30 is configured to extrude more than one type of material 40. In some embodiments, a first print head 30 is configured to extrude a first material 40 to form threads 72 and a second print head 30 is configured to extrude a second material 40 to form threads 72.

In other embodiments, suitable materials include natural materials, synthetic polymeric resorbable materials, synthetic polymeric non-resorbable materials, and other materials. Natural mesh materials include silk, extracellular matrix (such as DBM, collagen, ligament, tendon tissue, or other), silk-elastin, elastin, collagen, and cellulose. Synthetic polymeric resorbable materials include poly(lactic acid) (PLA), poly(glycolic acid) (PGA), poly(lactic acid-glycolic acid) (PLGA), polydioxanone, PVA, polyurethanes, polycarbonates, and others.

In various embodiments, the material of mesh implant or bag 70 comprises a polymer matrix. In some embodiments, DBM fibers and/or DBM powder are suspended in the polymer matrix to facilitate transfer of cells into and out of mesh implant or bag 70 to induce bone growth at the surgical site. In other embodiments, mesh implant or bag 70 further comprises mineralized bone fibers suspended in the polymer matrix. In some embodiments, the DBM powder is suspended in the polymer matrix between the DBM fibers and the mineralized bone fibers. In some embodiments, the DBM powder is suspended between the DBM fibers in the polymer matrix so as to reduce and/or eliminate gaps that exist between the fibers. In some embodiments, the DBM powder is suspended between the DBM fibers in the polymer matrix to improve osteoinductivity for facilitating bone fusion, for example, interspinous process fusion.

In some embodiments, the polymer matrix comprises a bioerodible, a bioabsorbable, and/or a biodegradable biopolymer that may provide immediate release or sustained release. Examples of suitable sustained release biopolymers include, but are not limited to, poly (alpha-hydroxy acids), poly (lactide-co-glycolide) (PLGA), polylactide (PLA), polyglycolide (PG), polyethylene glycol (PEG), conjugates of poly (alpha-hydroxy acids), poly(orthoester)s (POE), polyaspirins, polyphosphagenes, collagen, starch, pre-gelatinized starch, hyaluronic acid, chitosans, gelatin, alginates, albumin, fibrin, vitamin E compounds, such as alpha tocopheryl acetate, d-alpha tocopheryl succinate, D,L-lactide, or L-lactide, caprolactone, dextrans, vinylpyrrolidone, polyvinyl alcohol (PVA), PVA-g-PLGA, PEGT-PBT copolymer (polyactive), PEO-PPO-PAA copolymers, PLGA-PEO-PLGA, PEG-PLG, PLA-PLGA, poloxamer 407, PEG-PLGA-PEG triblock copolymers, SAIB (sucrose acetate isobutyrate), or combinations thereof. As persons of ordinary skill in the art are aware, mPEG and/or PEG may be used as a plasticizer for PLGA, but other polymers/excipients may be used to achieve the same effect. mPEG imparts malleability to the polymer. In some embodiments, these biopolymers may also be coated on mesh implant or bag 70 to provide a desired release profile or ingrowth of tissue. In some embodiments, the coating thickness may be thin, for example, from about 5, 10, 15, 20, 25, 30, 35, 40, 45 or 50 microns to thicker coatings of 60, 65, 70, 75, 80, 85, 90, 95 or 100 microns to delay release of the substance from the medical device. In some embodiments, the range of the coating on mesh implant or bag 70 ranges from about 5 microns to about 250 microns or 5 microns to about 200 microns.

In various embodiments, various components of mesh implant or bag 70 comprise poly(lactide-co-glycolide) (PLGA), polylactide (PLA), polyglycolide (PGA), D-lactide, D,L-lactide, L-lactide, D,L-lactide-co-*s*-caprolactone, D,L-lactide-co-glycolide-co-*s*-caprolactone, L-lactide-co-s-caprolactone or a combination thereof.

In some embodiments, material 40 of mesh implant or bag 70 further comprises bone morphogenetic proteins (BMPs), growth factors, antibiotics, angiogenesis promoting materials, bioactive agents or other actively releasing materials.

Mesh implant or bag 70 may be used to deliver a substance comprising any suitable biocompatible material. In specific embodiments, mesh implant or bag 70 may be used to deliver surface demineralized bone chips, optionally of a predetermined particle size, demineralized bone fibers, optionally pressed, and/or allograft. For embodiments wherein the substance is biologic, the substance may be autogenic, allogenic, xenogenic, or transgenic. Other suitable materials that may be positioned in mesh implant or bag 70 include, for example, protein, nucleic acid, carbohydrate, lipids, collagen, allograft bone, autograft bone, cartilage stimulating substances, allograft cartilage, TCP, hydroxyapatite, calcium sulfate, polymer, nanofibrous polymers, growth factors, carriers for growth factors, growth factor extracts of tissues, DBM, dentine, bone marrow aspirate, bone marrow aspirate combined with various osteoinductive or osteoconductive carriers, concentrates of lipid derived or marrow derived adult stem cells, umbilical cord derived stem cells, adult or embryonic stem cells combined with various osteoinductive or osteoconductive carriers, transfected cell lines, bone forming cells derived from periosteum, combinations of bone stimulating and cartilage stimulating materials, committed or partially committed cells from the osteogenic or chondrogenic lineage, or combinations of any of the above.

In accordance with some embodiments, the material to be positioned in the hollow compartment of the mesh implant or bag may be supplemented, further treated, or chemically modified with one or more bioactive agents or bioactive compounds. Bioactive agent or bioactive compound, as used herein, refers to a compound or entity that alters, inhibits, activates, or otherwise affects biological or chemical events. For example, bioactive agents may include, but are not limited to, osteogenic or chondrogenic proteins or peptides; DBM powder; collagen, insoluble collagen derivatives, etc., and soluble solids and/or liquids dissolved therein; anti-AIDS substances; anti-cancer substances; antimicrobials and/or antibiotics such as erythromycin, bacitracin, neomycin, penicillin, polymycin B, tetracyclines, biomycin, chloromycetin, and streptomycins, cefazolin, ampicillin, azactam, tobramycin, clindamycin and gentamycin, etc.; immunosuppressants; anti-viral substances such as substances effective against hepatitis; enzyme inhibitors; hormones; neurotoxins; opioids; hypnotics; anti-histamines; lubricants; tranquilizers; anti-convulsants; muscle relaxants and anti-Parkinson substances; anti-spasmodics and muscle contractants including channel blockers; miotics and anti-cholinergics; anti-glaucoma compounds; anti-parasite and/or anti-protozoal compounds; modulators of cell-extracellular matrix interactions including cell growth inhibitors and antiadhesion molecules; vasodilating agents; inhibitors of DNA, RNA, or protein synthesis; anti-hypertensives; analgesics; anti-pyretics; steroidal and non-steroidal anti-inflammatory agents; anti-angiogenic factors; angiogenic factors and polymeric carriers containing such factors; anti-secretory factors; anticoagulants and/or antithrombotic agents; local anesthetics; ophthalmics; prostaglandins; anti-depressants; anti-psychotic substances; anti-emetics; imaging agents; biocidal/biostatic sugars such as dextran, glucose, etc.; amino acids; peptides; vitamins; inorganic elements; co-factors for protein synthesis; endocrine tissue or tissue fragments; synthesizers; enzymes such as alkaline phosphatase, collagenase, peptidases, oxidases and the like; polymer cell scaffolds with parenchymal cells; collagen lattices; antigenic agents; cytoskeletal agents; cartilage fragments; living cells such as chondrocytes, bone marrow cells, mesenchymal stem cells; natural extracts; genetically engineered living cells or otherwise modified living cells; expanded or cultured cells; DNA delivered by plasmid, viral vectors, or other member; tissue transplants; autogenous tissues such as blood, serum, soft tissue, bone marrow, or the like; bioadhesives; bone morphogenetic proteins (BMPs including BMP-2); osteoinductive factor (IFO); fibronectin (FN); endothelial cell growth factor (ECGF); vascular endothelial growth factor (VEGF); cementum attachment extracts (CAE); ketanserin; human growth hormone (HGH); animal growth hormones; epidermal growth factor (EGF); interleukins, for example, interleukin-1 (IL-1), interleukin-2 (IL-2); human alpha thrombin; transforming growth factor (TGF-beta); insulin-like growth factors (IGF-1, IGF-2); parathyroid hormone (PTH); platelet derived growth factors (PDGF); fibroblast growth factors (FGF, BFGF, etc.); periodontal ligament chemotactic factor (PDLGF); enamel matrix proteins; growth and differentiation factors (GDF); hedgehog family of proteins; protein receptor molecules; small peptides derived from growth factors above; bone promoters; cytokines; somatotropin; bone digesters; antitumor agents; cellular attractants and attachment agents; immuno-suppressants; permeation enhancers, for example, fatty acid esters such as laureate, myristate and stearate monoesters of polyethylene glycol, enamine derivatives, alpha-keto aldehydes, and nucleic acids.

In certain embodiments, the bioactive agent may be a drug. In some embodiments, the bioactive agent may be a growth factor, cytokine, extracellular matrix molecule, or a fragment or derivative thereof, for example, a protein or peptide sequence such as RGD.

In some embodiments, the polymer material may have a modulus of elasticity in the range of from about 1 x 10² to about 6 x 10⁵ dynes/cm², or 2 x 10⁴ to about 5 x 10⁵ dynes/cm², or 5 x 10⁴ to about 5 x 10⁵ dynes/cm².

The material may have functional characteristics. Alternatively, other materials having functional characteristics may be incorporated into mesh implant or bag 70. Functional characteristics may include radiopacity, bacteriocidity, source for released materials, tackiness or a combination thereof. Such characteristics may be imparted substantially throughout mesh implant or bag 70 or at only certain positions or portions of mesh implant or bag 70.

Suitable radiopaque materials include, for example, ceramics, mineralized bone, ceramics/calcium phosphates/calcium sulfates, metal particles, fibers, and iodinated polymer (see, for example, WO/2007/143698). Polymeric materials may be used to form a bone graft or mesh implant or bag 70 and be made radiopaque by iodinating them, such as taught for example in U.S. Pat. No. 6,585,755, herein incorporated by reference in its entirety. Other techniques for incorporating a biocompatible metal or metal salt into a polymer to increase radiopacity of the polymer may also be used. Suitable bacteriocidal materials may include, for example, trace metallic elements. In some embodiments, trace metallic elements may also encourage bone growth.

In some embodiments, mesh implant or bag 70 may comprise a carrier material that becomes tacky upon wetting. Such material may be, for example, a protein or gelatin based material. Tissue adhesives, including mussel adhesive proteins and cryanocrylates, may be used to impart tackiness to mesh implant or bag 70. In further examples, alginate or chitosan material may be used to impart tackiness to mesh implant or bag 70. In further embodiments, an adhesive substance or material may be placed on a portion of mesh implant or bag 70 or in a particular region of mesh implant or bag 70 to anchor that portion or region of mesh implant or bag 70 in place at an implant site.

### Bone Material

In various embodiments, bone grafts, for example, mesh implants or bags 70 made by 3-D printing device 10 include compartments to hold osteogenic material, such as bone material. In other embodiments, for example, porous implants prepared by 3-D printing for an intended tissue repair site also comprise bone material as described in this disclosure. In various embodiments, the bone material may be particulated such as, for example, in bone chip, powder or fiber form. If the bone is demineralized, the bone may be made into a particulate before, during or after demineralization. In some embodiments, the bone may be monolithic and may not be a particulate.

The bone may be milled and ground or otherwise processed into particles of an appropriate size before or after demineralization. The particles may be particulate (for example, powder) or fibrous. The terms milling or grinding are not intended to be limited to production of particles of a specific type and may refer to production of particulate or fibrous particles. In certain embodiments, the particle size may be greater than 25 microns, such as ranging from about 25 to about 2000 microns, or from about 25 to about 500 microns or from about 200 to about 4000 microns. In some embodiments, the size of the bone particles are less than 100 microns. In some embodiments, the size of the bone particles are less than 500 microns. In some embodiments, the size of the bone particles are more than 4000 microns.

After grinding, the bone particles may be sieved to select those particles of a desired size. In certain embodiments, the particles may be sieved though a 25 micron sieve, a 50 micron sieve, a 75 micron sieve, a 100 micron sieve, a 125 micron sieve, a 150 micron sieve, a 175 micron sieve and/or a 200 micron sieve.

In some embodiments, the bone comprises DBM and/or mineralized bone. In some embodiments, the size of the bone particles is less than 25 microns. In some embodiments, the bone particle size is about 1, 2, 3, 4, 5, 6, 7, 8, 9, 10, 11, 12, 13, 14, 15, 16, 17, 18, 19, 20, 21, 22, 23 and/or 24 microns.

In various embodiments, the bone particles and/or the DBM and/or mineralized bone fibers have a sticky outer surface such that the bone can adhere to DBM and/or mineralized bone fibers. In various embodiments, the bone particles are naturally sticky. In some embodiments, an adhesive agent is applied to the bone particles and/or the bone fibers comprising a bioadhesive, glue, cement, cyanoacrylate, silicones, hot melt adhesives and/or cellulosic binders. In various embodiments, the adhesive may be applied to the surface of the bone particles by spraying or brushing. In some embodiments, a charge is applied to the fibers and an opposite charge is applied to the bone particles, (i.e., the technique of electrostatic precipitation). The bone particles will be attracted to, and tenaciously adhere to, the surface of the fiber. Any of these application techniques can be repeated one or more times to build up a relatively thick layer of adherent bone particles on the surface of the fibers.

The bone particles can be applied directly to the DBM fiber and/or fully mineralized fiber and the mixture can be disposed in mesh implant or bag 70. In some embodiments, the bone material inserted into a mesh implant or bag 70 contains pores having a pore size from about 0.5 to about 2,000 microns. In some embodiments, bone material inserted into a mesh implant or bag 70 contains pores having a pore size of from about 0.5, 5, 50, 100, 150, 200, 250, 300, 350, 400, 450, 500, 550, 600, 650, 700, 750, 800, 850, 900, 950, 1,000, 1,050, 1,100, 1,150, 1,200, 1,250, 1,300, 1,350, 1,400, 1,450, 1,500, 1,550, 1,600, 1,650, 1,700, 1,750, 1,800, 1,850, 1,900, 1,950 to about 2,000 microns. In some embodiments, the pore size of the bone material is uniform. In some embodiments, the pore size of bone material is non-uniform and includes various pore sizes in the range from 0.5 to about 2,000 microns. Alternatively, the DBM fiber, and DBM particles can be placed in a polymer (for example, collagen) and inserted into a porous biodegradable graft body (for example, a pouch, container, mesh bag, and the like).

Following shaving, milling or other technique whereby they are obtained, the bone material is subjected to demineralization in order to reduce its inorganic content to a very low level, in some embodiments, to not more than about 5% by weight of residual calcium, or to not more than about 1% by weight of residual calcium. Demineralization of the bone material ordinarily results in its contraction to some extent.

Bone used in the methods described herein may be autograft, allograft, or xenograft. In various embodiments, the bone may be cortical bone, cancellous bone, or cortico-cancellous bone. While specific discussion is made herein to demineralized bone matrix, bone matrix treated in accordance with the teachings herein may be non-demineralized, demineralized, partially demineralized, or surface demineralized. This discussion applies to demineralized, partially demineralized, and surface demineralized bone matrix. In one embodiment, the demineralized bone is sourced from bovine or human bone. In another embodiment, demineralized bone is sourced from human bone. In one embodiment, the demineralized bone is sourced from the patient's own bone (autogenous bone). In another embodiment, the demineralized bone is sourced from a different animal (including a cadaver) of the same species (allograft bone).

Any suitable manner of demineralizing the bone may be used. Demineralization of the bone material can be conducted in accordance with known conventional procedures. For example, in a demineralization procedure, the bone materials useful for the implantable composition of this application are subjected to an acid demineralization step that is followed by a defatting/disinfecting step. The bone material is immersed in acid over time to effect its demineralization. Acids which can be employed in this step include inorganic acids such as hydrochloric acid and organic acids such as peracetic acid, acetic acid, citric acid, or propionic acid. The depth of demineralization into the bone surface can be controlled by adjusting the treatment time, temperature of the demineralizing solution, concentration of the demineralizing solution, agitation intensity during treatment, and other applied forces such as vacuum, centrifuge, pressure, and other factors such as known to those skilled in the art. Thus, in various embodiments, the bone material may be fully demineralized, partially demineralized, or surface demineralized.

After acid treatment, the bone is rinsed with sterile water for injection, buffered with a buffering agent to a final predetermined pH and then finally rinsed with water for injection to remove residual amounts of acid and buffering agent or washed with water to remove residual acid and thereby raise the pH. Following demineralization, the bone material is immersed in solution to effect its defatting. A type of defatting/disinfectant solution is an aqueous solution of ethanol, the ethanol being a good solvent for lipids and the water being a good hydrophilic carrier to enable the solution to penetrate more deeply into the bone. The aqueous ethanol solution also disinfects the bone by killing vegetative microorganisms and viruses. Ordinarily at least about 10 to 40 weight percent by weight of water (i.e., about 60 to 90 weight percent of defatting agent such as alcohol) should be present in the defatting/disinfecting solution to produce optimal lipid removal and disinfection within the shortest period of time. A concentration range of the defatting solution is from about 60 to 85 weight percent alcohol or about 70 weight percent alcohol.

Further in accordance with this application, the DBM material can be used immediately for preparation of the implant composition or it can be stored under aseptic conditions, advantageously in a critical point dried state prior to such preparation. The bone material can retain some of its original mineral content such that the composition is rendered capable of being imaged utilizing radiographic techniques.

In various embodiments, this application also provides bone matrix compositions comprising critically point dried (CPD) fibers. DBM includes the collagen matrix of the bone together with acid insoluble proteins including bone morphogenetic proteins (BMPs) and other growth factors. It can be formulated for use as granules, gels, sponge material or putty and can be freeze-dried for storage. Sterilization procedures used to protect from disease transmission may reduce the activity of beneficial growth factors in the DBM. DBM provides an initial osteoconductive matrix and exhibits a degree of osteoinductive potential, inducing the infiltration and differentiation of osteoprogenitor cells from the surrounding tissues.

DBM preparations have been used for many years in orthopedic medicine to promote the formation of bone. For example, DBM has been used in the repair of fractures, in the fusion of vertebrae, in joint replacement surgery, and in treating bone destruction due to underlying disease such as rheumatoid arthritis. DBM is thought to promote bone formation *in vivo* by osteoconductive and osteoinductive processes. The osteoinductive effect of implanted DBM compositions is thought to result from the presence of active growth factors present on the isolated collagen-based matrix. These factors include members of the TGF-*B*, IGF, and BMP protein families. Particular examples of osteoinductive factors include TGF-*B*, IGF-1, IGF-2, BMP-2, BMP-7, parathyroid hormone (PTH), and angiogenic factors. Other osteoinductive factors such as osteocalcin and osteopontin are also likely to be present in DBM preparations as well. There are also likely to be other unnamed or undiscovered osteoinductive factors present in DBM.

In various embodiments, the DBM provided in the methods described in this application is prepared from elongated bone fibers which have been subjected to critical point drying. The elongated CPD bone fibers employed in this application are generally characterized as having relatively high average length to average width ratios, also known as the aspect ratio. In various embodiments, the aspect ratio of the elongated bone fibers is at least from about 50:1 to at least about 1000:1. Such elongated bone fibers can be readily obtained by any one of several methods, for example, by milling or shaving the surface of an entire bone or relatively large section of bone.

In other embodiments, the length of the fibers can be at least about 3.5 cm and the average width can be from about 20 mm to about 1 cm. In various embodiments, the average length of the elongated fibers can be from about 3.5 cm to about 6.0cm and the average width can be from about 20 mm to about 1 cm. In other embodiments, the elongated fibers can have an average length from about 4.0 cm to about 6.0 cm and an average width from about 20 mm to about 1 cm.

In yet other embodiments, the diameter or average width of the elongated fibers is, for example, not more than about 1 cm, not more than 0.5 cm or not more than about 0.01 cm. In still other embodiments, the diameter or average width of the fibers can be from about 0.01 cm to about 0.4 cm or from about 0.02 cm to about 0.3 cm.

In another embodiment, the aspect ratio of the fibers can be from about 50:1 to about 950:1, from about 50:1 to about 750:1, from about 50:1 to about 500:1, from about 50:1 to about 250:1; or from about 50:1 to about 100:1. Fibers according to this disclosure can advantageously have an aspect ratio from about 50:1 to about 1000:1, from about 50:1 to about 950:1, from about 50:1 to about 750:1, from about 50:1 to about 600:1, from about 50:1 to about 350:1, from about 50:1 to about 200:1, from about 50:1 to about 100:1, or from about 50:1 to about 75:1.

In some embodiments, the chips to fibers ratio is about 90:10, 80:20, 75:25, 70:30, 60:40, 50:50, 40:60, 30:70, 25:75, 20:80 and/or 10:90. In various embodiments, the ratio of surface demineralized chips to fibers is about 90:10, 80:20, 75:25, 70:30, 60:40, 50:50, 40:60, 30:70, 25:75, 20:80 and/or 10:90. In some embodiments, a surface demineralized chips to fully demineralized fibers ratio is about 90:10, 80:20, 75:25, 70:30, 60:40, 50:50, 40:60, 30:70, 25:75, 20:80 and/or 10:90.

In some embodiments, the DBM fibers have a thickness of about 0.5-4 mm. In various embodiments, the DBM fibers have a thickness of about 0.5, 0.6, 0.7, 0.8, 0.9, 1, 1.5, 2, 2.5, 3, 3.5 and/or 4 mm. In various embodiments, the ratio of DBM fibers to DBM powder is about 40:60 to about 90:10 W/W, W/V or V/V. In some embodiments, the ratio of mineralized bone fibers to DBM powder is about 25:75 to about 75:25 W/W, W/V or V/V. In various embodiments, the device comprises DBM fibers and mineralized fibers in a ratio of 40:60 to about 90:10 W/W, W/V or V/V. In some embodiments, the DBM fibers to DBM powder ratio, mineralized bone fibers to DBM powder ratio and/or the DBM fibers and mineralized fibers ratio is from 5:95 to about 95:5 W/W, W/V or V/V. In some embodiments, the DBM fibers to DBM powder ratio, mineralized bone fibers to DBM powder ratio and/or the DBM fibers and mineralized fibers ratio is 5:95, 10:90, 15:85, 20:80, 25:75, 30:70, 35:65, 40:60, 45:55, 50:50, 55:45, 60:40, 65:35, 70:30, 75:25, 80:20, 85:15, 90:10 and/or 95:5 W/W, W/V or V/V.

In some embodiments, the bone material comprises demineralized bone material comprising demineralized bone, fibers, powder, chips, triangular prisms, spheres, cubes, cylinders, shards or other shapes having irregular or random geometries. These can include, for example, "substantially demineralized," "partially demineralized," or "fully demineralized" cortical and/or cancellous bone. These also include surface demineralization, where the surface of the bone construct is substantially demineralized, partially demineralized, or fully demineralized, yet the body of the bone construct is fully mineralized.

In various embodiments, the bone graft material comprises fully DBM fibers and surface demineralized bone chips. In some embodiments, the ratio of fully DBM fibers to surface demineralized bone chips is from 5:95 to about 95:5 fibers to chips. In some embodiments, the ratio of fully DBM fibers to surface demineralized bone chips is 5:95, 10:90, 15:85, 20:80, 25:75, 30:70, 35:65, 40:60, 45:55, 50:50, 55:45, 60:40, 65:35, 70:30, 75:25, 80:20, 85:15, 90:10 and/or 95:5 fibers to chips. In various embodiments, the fully DBM fibers have a thickness of about 0.5-4 mm. In various embodiments, the fully DBM fibers have a thickness of about 0.5, 0.6, 0.7, 0.8, 0.9, 1, 1.5, 2, 2.5, 3, 3.5 and/or 4 mm.

In various embodiments, the fibers and/or the powder is surface DBM. In some embodiments, the fibers and/or the powder is surface DBM cortical allograft. In various embodiments, surface demineralization involves surface demineralization to at least a certain depth. For example, the surface demineralization of the allograft can be from about 0.25 mm, 0.5 mm, 1 mm, 1.5 mm, 2.0 mm, 2.5 mm, 3.0 mm. 3.5 mm, 4 mm, 4.5 mm, to about 5 mm. The edges of the bone fibers and/or powder may further be machined into any shape or to include features such as grooves, protrusions, indentations, etc., to help improve fit and limit any movement or micromotion to help fusion and/or osteoinduction to occur.

To prepare the osteogenic DBM, a quantity of fibers is combined with a biocompatible carrier material to provide a demineralized bone matrix.

DBM typically is dried, for example via lyophilization or solvent drying, to store and maintain the DBM in active condition for implantation. Moreover, each of these processes is thought to reduce the overall surface area structure of bone. As may be appreciated, the structural damage of the exterior surface reduces the overall surface area. Physical alterations to the surface and reduction in surface area can affect cell attachment, mobility, proliferation, and differentiation. The surface's affinity for growth factors and release kinetics of growth factors from the surface may also be altered.

Accordingly, in some embodiments, methods for drying bone to store and maintain the bone in active condition for implantation that maintains or increases the surface area of the bone are provided. In one embodiment, the bone matrix is treated using a critical point drying (CPD) technique, thereby reducing destruction of the surface of the bone. While specific description is made to critical point drying, it is to be appreciated that, in alternative embodiments, super critical point treatment may be used. In various embodiments utilizing CPD, a percentage of collagen fibrils on the surface of the bone are non-denatured after drying to a residual moisture content of approximately 15% or less. In some embodiments, after drying, the bone matrix has a residual moisture content of approximately 8% or less. In some embodiments, after drying, the bone matrix has a residual moisture content of approximately 6% or less. In some embodiments, after drying, the bone matrix has a residual moisture content of approximately 3% or less.

Evaporative drying and freeze drying of specimens can cause deformation and collapse of surface structures, leading to a decrease in surface area. Without wishing to be bound by a particularly theory, this deformation and structure is thought to occur because as a substance crosses the boundary from liquid to gas, the substance volatilizes such that the volume of the liquid decreases. As this happens, surface tension at the solid-liquid interface pulls against any structures to which the liquid is attached. Delicate surface structures tend to be broken apart by this surface tension. Such damage may be caused by the effects of surface tension on the liquid/gas interface. Critical point drying is a technique that avoids effects of surface tension on the liquid/gas interface by substantially preventing a liquid/gas interface from developing. Critical point or supercritical drying does not cross any phase boundary, instead passing through the supercritical region, where the distinction between gas and liquid ceases to apply. As a result, materials dehydrated using critical point drying are not exposed to damaging surface tension forces. When the critical point of the liquid is reached, it is possible to pass from liquid to gas without an abrupt change in state. Critical point drying can be used with bone matrices to phase change from liquid to dry gas without the effects of surface tension. Accordingly, bone dehydrated using critical point drying can retain or increase at least some of the surface structure and therefore the surface area.

In some embodiments, critical point drying is carried out using carbon dioxide. However, other mediums such as Freon, including Freon 13 (chlorotrifluoromethane), may be used. Generally, fluids suitable for supercritical drying include carbon dioxide (critical point 304.25 K at 7.39 MPa or 31.1° C at 1072 psi or 31.2° C and 73.8 bar) and Freon (about 300 K at 3.5-4 MPa or 25 to 30° C at 500-600 psi). Nitrous oxide has similar physical behavior to carbon dioxide, but is a powerful oxidizer in its supercritical state. Supercritical water is also a powerful oxidizer, partly because its critical point occurs at such a high temperature (374° C) and pressure (3212 psi/647K and 22.064 MPa).

In some embodiments, the bone may be pretreated to remove water prior to critical point drying. Thus, in accordance with one embodiment, bone matrix is dried using carbon dioxide in (or above) its critical point status. After demineralization, bone matrix samples (in water) may be dehydrated to remove residual water content. Such dehydration may be obtained, for example, by using a series of graded ethanol solutions (for example, 20%, 50%, 70%, 80%, 90%, 95%, 100% ethanol in deionized water). In some embodiments, penetrating the tissue with a graded series of ethanol solutions or alcohols may be accomplished in an automated fashion. For example, pressure and vacuum could be used to accelerate penetration into the tissue.

### Methods of Making a Porous Mesh Implant

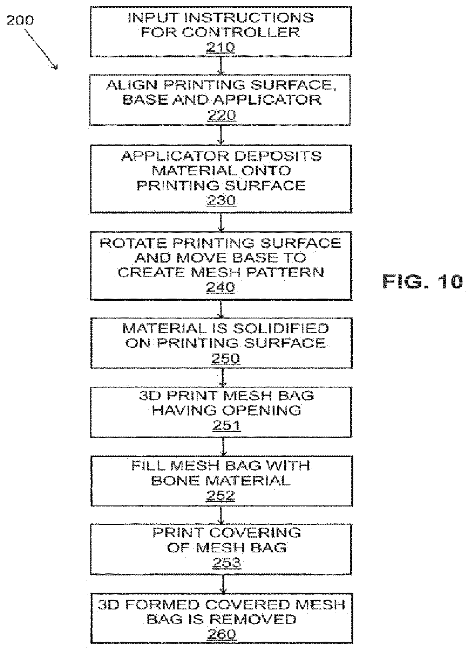

In various embodiments as shown in FIG. 10, a computer implemented method 200 of fabricating a hollow structure, such as a mesh implant or bag 70, through use of a 3-D printing device 10 is provided. In some embodiments, the method includes step 210 for inputting instructions for a computer processor 102 to carry out the fabrication, step 220 for aligning the printing surface, base and print head relative to one another, step 230 for depositing material onto the printing surface, step 240 for rotating the printing surface and moving the base to create a mesh pattern, step 250 for solidifying material on the printing surface, step 251 for 3-D printing of a mesh implant or bag having a compartment accessible through an opening, step 252 for filling the compartment with bone material, step 253 for enclosing the mesh implant by 3-D printing a covering for enclosing the bone material within the compartment of the mesh implant and step 260 for removing the 3-D formed and covered implant or mesh bag. In some embodiments, the method comprises: rotating a print surface in alternating clockwise and counterclockwise directions, ejecting material from a print head to the printing surface to make a thread having a wave-like pattern with alternating peaks and crests, and rotating the print head such an angular distance to create a plurality of interconnected threads on the printing surface.

In some embodiments, a method for fabricating a hollow structure is provided which includes providing a 3-D printing machine 10 having a table 14, a base 16 and a printing surface 12. In various embodiments, printing surface 12 is rotatable about an axis of rotation. Base 16 is configured for planar movement. Printing surface 12 is fixedly disposed with table 14 such that lateral movement of base 16 causes lateral movement of printing surface 12. In some embodiments, base 16 is movable in the x-y plane and is laterally movable in both the x axis and the y axis for precise positioning of printing surface 12. Movement of base 16 allows for positioning of printing surface 12 relative to extension shaft 20 to facilitate depositing materials onto printing surface 12, as discussed herein. 3-D printing device 10 further includes a print head 30 to deposit material 40 onto printing surface 12. The deposit material 40 includes material used to make the mesh (e.g., biodegradable polymer, etc.).

In other embodiments, a processor 102 receives instructions for the fabrication of a mesh implant or bag 70. A user may input instructions directly into 3-D printing device 10 or may input instructions into an external computer in communication with processor 102. Processor 102 directs movement of base 16, printing surface 12 and print head 30 relative to one another. Processor 102 also directs application of material 40 from print head 30 onto printing surface 12.

According to various aspects, a user loads a material reservoir (not shown) in communication with print head 30 with a suitable material 40. The material 40 may be in powder form, particulate form, gel form, or solid form. Processor 102 moves the printing surface 12 and one or more print heads 30 into place relative to one another. Once positioned, print head begins to deposit material 40 onto printing surface 12. In some embodiments, print head 30 continuously deposits material 40 as printing surface 12 is rotated and/or moved laterally along the x-y plane. In some embodiments, printing surface 12 is rotated in the clockwise and counterclockwise directions while base 16 moves laterally to form wave-shaped threads 72. The degree of rotation may be adjusted to impart flexible and stretchable qualities onto each of the formed threads 72. For example, threads 72 having shorter wavelengths will be able to be stretched more than threads 72 having longer wavelengths. In some embodiments, processor 102 directs rotation of printing surface 12 and lateral movement of base 16 to impart stretchability of mesh implant or bag 70 that is uniform across its length. In some embodiments, processor 102 directs variable rotation of printing surface 12 and lateral movement of base 16 such that mesh implant or bag 70 includes regions of increased stretchability according to the needs of a surgical application.

The movement of base 16, printing surface 12 and print head 30 relative to one another and the application of material 40 onto printing surface 12 is repeated a number of times such that threads 72 encompass the surface of printing surface 12. That is, each time a thread having a wave-like shape is applied to printing surface 12, a similar thread 72 is applied to printing surface 12 adjacent first thread 72. In some embodiments, threads 72 are extruded adjacent to one another such that the peaks of a first thread 72 are extruded to contact the crest of an adjacent second thread 72. In some embodiments, mesh implant or bag 70 may be created entirely from threads 72 having this configuration.

In some embodiments, print head 30 deposits material 40 in powdered form to printing surface 12. Material 40 must be sintered and/or melted to form threads 72. In some embodiments, a radiation source, such as laser 60 may be used in conjunction with print head 30. Processor 102 directs laser 60 to be focused at a point on which material 40 has been deposited adjacent print head 30. Processor 102 also provides power to laser 60 during desired intervals to prevent unwanted damage to mesh implant or bag 70 and/or printing surface 12 according to the instructions. That is, laser 60 will emit a beam while sintering material 40 to create threads 72, but will not emit a beam when printing surface 12 is being repositioned relative to print head 30. Once all desired sintering has been completed, any excess material 40 may be brushed away from printing surface 12 to be discarded or recycled.

In some embodiments, material 40 may be sintered through use of a temperature control unit 50 that is a heating unit as illustrated in FIG. 1. Temperature control unit 50 provides energy to printing surface 12 such that powdered material 40 melts and molds together. An amount of heat may be provided such that material 40 melts quickly upon contact with printing surface 12.

In some embodiments, printing surface 12 is heated or cooled using temperature control unit 50 to remove mesh implant or bag 70. In some embodiments, printing surface 12 may be removed from 3-D printing device 10 and submerged in a solvent to loosen and remove mesh implant or bag 70.

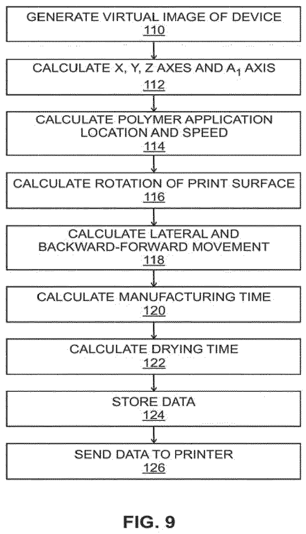

As shown in FIG. 9, a computer implemented method for producing a hollow structure such as a mesh implant or bag is illustrated. In a first step 110, a user or a designer generates a virtual image of the object or a 3-D digital model to be created with the 3-D printing machine, such as, for example, mesh implant or bag 70 including a virtual volume of the compartment to enclose the bone material therein and a virtual depth, thickness and volume of the mesh implant and a covering configured for enclosing the compartment of mesh implant or bag 70. The computer can generate a virtual 3D image of the cover including a virtual volume, length, and width of the covering to be printed. Commercially available CAM software can make the CAD drawing/design of the medical implant into a computer code, (for example, g-code). This code is sent to the device and the controller controls the device and the loading of the print head with the material, the heating and cooling temperature and time of the material, laser emit time, rotation, rotation speed of the print surface, print head, table, lateral movement of the print surface, print head, and table as well as other parameters. The controller device creates a medical implant from or in the material based on the 3-D digital model. In some embodiments, the 3-D digital model of the mesh implant is generated based on the 3-D image of an intended bone repair site. The 3-D image of a bone repair site can be obtained by using (i) one or more X-ray images; (ii) a computer aided design (CAD) program; (iii) a cone beam imaging device; (iv) a computed tomography (CT) scan device; (v) a magnetic resonance imaging (MRI); (vi) 3-D laser camera, or a combination thereof.

In a second step 112, processor 102 calculates the X, Y, Z and A₁ axes. The device employs Cartesian coordinate system (X, Y, Z) for 3-D motion control and employs a 4th axis (A₁) for the rotation of the printing surface (for example, 360 degrees) relative to the print head. The implant can be designed virtually in the computer with a CAD/CAM program, which is on a computer display. The user inputs specific parameters into the computer and then presses print on the display to start the 3-D printing manufacturing. The computer logic programs the computer with instructions for loading of the print head with the material; application and thickness of the polymer from the print head; the heating and cooling temperature and time of the device; laser emit time; rotation; rotation speed of the printing surface, print head, and/or print table; and/or lateral movement of the printing surface, print head, and/or table as well as other parameters in accordance with the received instructions. The controller device causes the print head to be located at the appropriate X, Y, Z coordinates for 3-D motion control and employs a 4th axis (A₁) for the rotation of the printing surface (for example, 360 degrees, 180 degrees, 120 degrees) relative to the print head to make a medical implant from or in the material. After the medical implant is produced on all or a portion of the printing surface, it will have a compartment or a hollow region which typically is greater than the diameter or thickness of the printing surface and can be removed by a tool that engages the printing surface. In some embodiments, the device can have a tool to etch, shape, and/or dry the implant before, during or after it is removed from the printing surface.

In a third step 114, processor 102 calculates the polymer application, location and speed by planning coordination of the printing surface and print head. In some embodiments, the current device does not manufacture the implant device by printing the material in successive layers to form the implant. In a fourth step 116 and a fifth step 118, processor 102 calculates the rotation of the printing surface and the lateral and/or backward and forward movement of the printing surface and print head. In some embodiments, the printing surface of the current application has the polymer continuously dispensed from the print head and onto the printing surface as the printing surface rotates in 360 degrees clockwise and/or counterclockwise relative to the print head and the table, and/or printing surface can, in some embodiments, move in a forward, lateral, and/or backward direction so that the threads to make the medical implant (for example, a mesh bag) are formed in accordance with the instructions received from the computer. In some embodiments, the printing surface of the current application has a heat sensitive polymer disposed on it and then the print head receives instructions to heat the surface area to be removed (for example, by laser, heating element, or the like). In this way, threads of the polymer are made by removing the heated portions of the polymer and what is left on the printing surface are the threads for the implant. The printing surface rotates in 360 degrees clockwise and/or counterclockwise relative to the print head and the table, and/or printing surface can, in some embodiments, move in a forward, lateral, and/or backward direction so that the threads to make the medical implant (for example, a mesh implant or bag) are formed as the rest of the polymer is removed from the printing surface in accordance with the instructions received from the computer.

In some embodiments, the printing surface of the current application has the polymer in dry powder form continuously dispensed from the print head and onto the printing surface as the printing surface rotates in 360 degrees clockwise and/or counterclockwise relative to the print head and the table, and/or printing surface can, in some embodiments, move in a forward, lateral, and/or backward direction so that the threads to make the medical implant (for example, a mesh implant or bag) are formed in accordance with the instructions received from the computer. After, the powder application, which can be from the print head from a reservoir therein, the print head (for example, a laser or heating element coupled thereto) can heat the powder polymer and form the threads for the medical implant.

Based on the above calculations, processor 102 calculates a projected amount of time it will take to manufacture the medical implant in step 120. In a subsequent step 122, processor 102 calculates the amount of time it will take for the printed medical device to dry. In some embodiments, the material applied to the printing surface is temperature sensitive and dries and/or cures through heating or cooling. In some embodiments, processor 120 directs a temperature control unit to heat or cool the printing surface. In some embodiments, processor 120 directs a laser to focus its beam on the material applied to printing surface 12 to sinter and cure the material.

In step 124, the data calculated by processor 102 is stored in memory 100 for subsequent implementation. In some embodiments, processor 102 processes and organizes the calculated data into memory 100. In some embodiments, processor 100 includes value-determining logic, development logic, security logic, and/or analytical logic. In some embodiments, processor 102 updates the memory 100 with any new calculation data received from the user. In some embodiments, there is a computer readable storage medium storing instructions that, when executed by a computer, cause the computer to display options for a user to enter, view, and edit some or all features for manufacturing the implant including the loading of the print head with the material; the heating and cooling temperature and time of the material; laser emit time; rotation angle; rotation speed of the printing surface, print head and/or table; lateral movement of the printing surface, print head and table; as well as other parameters. The controller device creates a medical implant from or in the material by instructions received from the computer. The device employs Cartesian coordinate system (X, Y, Z) for 3-D motion control and employs a 4th axis (A₁) for the rotation of the printing surface (for example, 360 degrees) relative to the print head.

In a final step 126, the user inputs a command to send the stored data to the printer to create the medical device. The user inputs specific parameters into the computer and then presses print on the display to start the 3-D printing manufacturing. The computer logic causes the computer to execute loading of the print head with the material; the heating and cooling temperature and time of the device; laser emit time; rotation; rotation speed of the printing surface, print head, and/or table; and/or lateral movement of the printing surface, print head, and/or table; as well as other parameters. The controller device causes the print head to be located at the appropriate X, Y, Z coordinates for 3-D motion control and employs a 4th axis (A₁) for the rotation of the printing surface (for example, 360 degrees, 180 degrees, 120 degrees) relative to the print head to make a medical implant from or in the material.

### Method of Making a Porous Implant

In various embodiments, a computer implemented method for producing a porous implant is provided. The computer implemented method for producing a porous implant includes obtaining a 3-D image of an intended tissue repair site; generating a 3-D digital model of the porous implant based on the 3-D image of the intended tissue repair site, the 3-D digital model of the porous implant being configured to fit within the intended tissue repair site; determining an implant material and an amount of a porogen to add to an implant material to obtain a desired porosity of the porous implant, the porosity being based on a plurality of macropores, micropores, nanopores structures or a combination thereof; storing the 3-D digital model previously obtained on a database coupled to a processor, the processor having instructions for retrieving the stored 3-D digital model of the porous implant, and the processor for combining the implant material with the porogen based on the stored 3-D digital model and for instructing a 3-D printer to produce the porous implant.

In some aspects, the intended tissue repair site can be a bone repair site, an osteochondral defect site, an articular cartilage defect site or a combination thereof. Tissue, as used herein, includes soft tissue, muscle, ligaments, tendons, cartilage and hard tissue as found in bones.

In certain embodiments, the 3-D image of an intended porous implant repair site is a computed tomography image of an unhealthy tissue repair site, based on a computed tomography image of a healthy tissue repair site. In other embodiments, the 3-D image is obtained from (i) one or more X-ray images; (ii) a computer aided design (CAD) program; (iii) a cone beam imaging device; (iv) a computed tomography (CT) scan device; (v) a magnetic resonance imaging (MRI) or a combination thereof.

Generally, in many implementations, the carrier material comprises a biodegradable polymer, a metal, or a combination thereof and the bone material comprises autograft, allograft, demineralized bone matrix fiber, demineralized bone chips or a combination thereof.

### Porogen

In certain embodiments, the porous implant includes a porogen that diffuses, dissolves, and/or degrades after implantation into the porous implant leaving a pore. The porogen may be a gas (e.g., carbon dioxide, nitrogen, argon or air), liquid (e.g., water, blood lymph, plasma, serum or marrow), or solid (e.g., crystalline salt, sugar). The porogen may be a water-soluble chemical compound such as a carbohydrate (e.g., polydextrose, dextran), salt, polymer (e.g., polyvinyl pyrrolidone), protein (e.g., gelatin), pharmaceutical agent (e.g., antibiotics), or a small molecule. In other aspects, the porous implant includes as a porogen polysaccharides comprising cellulose, starch, amylose, dextran, poly(dextrose), glycogen, poly(vinylpyrollidone), pullulan, poly(glycolide), poly(lactide), and/or poly(lactide-co-glycolide). In other aspects, the useful porogens include without limitaions hydroxyapatite or polyethylene oxide, polylactic acid, polycaprolactone. Peptides, proteins of fifty amino acids or less or a parathyroid hormone are also useful porogens.

The porous implants of the present disclosure can exhibit high degrees of porosity over a wide range of effective pore sizes. Thus, porous implants of the present disclosure may have, at once, macroporosity, mesoporosity, microporosity and nanoporosity. Macroporosity is characterized by pore diameters greater than about 100 microns. Mesoporosity is characterized by pore diameters between about 100 microns about 10 microns; and microporosity occurs when pores have diameters below about 10 microns. Microporous implants have pores of diameters below 9 microns, 8 microns, 7 microns, 6 microns, 5 microns, 4 microns, 3 microns, 2 microns, and 1micron. Nanoporosity of nanopores is characterized by pore diameters of about 1 nm and below. In various applications, porogens have different shapes, for example, spheroidal, cuboidal, rectangular, elongated, tubular, fibrous, disc-shaped, platelet-shaped, polygonal or a combination thereof.

In some embodiments, the porous implant has a porosity of at least about 30%. For example, in certain embodiments, the porous implant has a porosity of more than about 50%, more than about 60%, more than about 70%, more than about 80%, or more than about 90%. Advantages of a highly porous implant over a less porous or non-porous implant include, but are not limited to, more extensive cellular and tissue in-growth into the porous implant, more continuous supply of nutrients, more thorough infiltration of therapeutics, and enhanced revascularization, allowing bone growth and repair to take place more efficiently. Furthermore, in certain embodiments, the porosity of the porous implant may be used to load the porous implant with biologically active agents such as drugs, small molecules, cells, peptides, polynucleotides, growth factors, osteogenic factors, for delivery at the porous implant site. Porosity may also render certain porous implants of the present disclosure compressible.

In certain embodiments, the pores of the porous implant are over 100 microns wide available for the invasion of cells and bony in-growth. Klaitwatter, et al, "Application of porous ceramics for the attachment of load bearing orthopedic applications," J. Biomed. Mater. Res. Symp. 2:161, 1971; each of which is incorporated herein by reference. In certain embodiments, the pore size ranges from approximately 50 microns to approximately 500 microns, in other aspects, from approximately 100 microns to approximately 250 microns, from approximately 5 microns, 4 microns, 3 microns, 2 microns, 1 micron to approximately 1 nanometer.

The porosity of the porous implant may be accomplished using any means known in the art. Exemplary methods of creating porosity in a porous implant include, but are not limited to, particular leaching processes, gas foaming processing, supercritical carbon dioxide processing, sintering, phase transformation, freeze-drying, cross-linking, molding, porogen melting, polymerization, melt-blowing, and salt fusion (Murphy et al. Tissue Engineering 8(1):43-52, 2002; incorporated herein by reference). For a review, see Karageorgiou et al, Biomaterials 26:5474-5491, 2005; incorporated herein by reference. The porosity may be a feature of the porous implant during 3-D printing of the porous implant or before implantation, or the porosity may only be available after implantation of the porous implant. For example, the 3-D printed porous implant may include latent pores. These latent pores may arise from including porogens in the porous implant.

The porogen may be any chemical compound that will reserve a space within the porous implant while the porous implant is being molded and will diffuse, dissolve, and/or degrade prior to or after implantation, leaving a pore in the porous implant. Porogens, in some aspects, have the property of not being appreciably changed in shape and/or size during the procedure to make the porous implant moldable. For example, the porogen should retain its shape during the heating of the 3-D printed porous implant to make it moldable. Therefore, the porogen, in other aspects, does not melt upon heating of the porous implant to make it moldable. In certain embodiments, the porogen has a melting point greater than about 60° C, greater than about 70° C, greater than about 80° C, greater than about 85° C, or greater than about 90° C.

Porogens may be of any shape or size. The porogen may be spheroidal, cuboidal, rectangular, elonganted, tubular, fibrous, disc-shaped, platelet-shaped, polygonal, or a combination thereof. In certain embodiments, the porogen is granular with a diameter ranging from approximately 100 microns to approximately 800 microns, from approximately 5 microns, 4 microns, 3 microns, 2 microns, 1 micron to approximately 1 nanometer. In certain embodiments, the porogen is elongated, tubular, or fibrous. Such porogens provide increased connectivity of the pores of the porous implant and/or also allow for a lesser percentage of the porogen in the porous implant. The amount of the porogen may vary in the porous implant from 1% to 80% by weight. In certain embodiments, the plasticizer makes up from about 5% to about 80% by weight of the porous implant. In certain embodiments, the plasticizer makes up from about 10% to about 50% by weight of the porous implant. Pores in the porous implant are thought to improve the osteoinductivity or osteoconductivity of the porous implant by providing holes for cells such as osteoblasts, osteoclasts, fibroblasts, cells of the osteoblast lineage, and stem cells in which these cells can grow. The pores provide the porous implant with biological in-growth capacity. Pores in the porous implant may also provide for easier degradation of the porous implant as bone is formed and/or remodeled. In some embodiments, the porogen is biocompatible. In various embodiments, the computer implemented method described in this application provides 3-D printed porous implants, wherein the porous implant comprises from about 10% to about 80% by weight of porogen, from about 20% to about 90% by weight of polymer and from about 30% to about 50% by weight of bone material.

The porogen may be a gas, liquid, or solid. Exemplary gases that may act as porogens include carbon dioxide, nitrogen, argon, or air. Exemplary liquids include water, organic solvents, or biological fluids (e.g., blood lymph, plasma, serum or marrow). The gaseous or liquid porogen may diffuse out of the porous implant before or after implantation thereby providing pores for biological in-growth. Solid porogens may be crystalline or amorphous. Examples of possible solid porogens include water soluble compounds, for example, sodium chloride, sugar and the like. In certain embodiments, the water-soluble compound has a solubility of greater than 10 g per 100 mL water at 25° C. In certain embodiments, the water-soluble compound has a solubility of greater than 25 g per 100 mL water at 25° C. In certain embodiments, the water-soluble compound has a solubility of greater than 50 g per 100 mL water at 25° C. In certain embodiments, the water-soluble compound has a solubility of greater than 75 g per 100 mL water at 25° C. In certain embodiments, the water-soluble compound has a solubility of greater than 100 g per 100 mL water at 25° C. Examples of porogens include carbohydrates (e.g., sorbitol, dextran polydextrose, starch), salts, sugar alcohols, natural polymers, synthetic polymers, and small molecules. In some implementations, useful porogens also include polysaccharides, for example, cellulose, starch, amylose, dextran, poly(dextrose), glycogen, poly(vinylpyrollidone), pullulan, poly(glycolide), poly(lactide), poly(lactide-co-glycolide); or peptides, proteins of fifty amino acids or less or a parathyroid hormone. Other useful porogens include without limitation, hydroxyapatite or polyethylene oxide, polylactic acid, and polycaprolactone.

In certain embodiments, carbohydrates are used as porogens in the porous implants. The carbohydrate may be a monosaccharide, disaccharide, or polysaccharide. The carbohydrate may be a natural or synthetic carbohydrate. In some embodiments, the carbohydrate is a biocompatible, biodegradable carbohydrate. In certain embodiments, the carbohydrate is a polysaccharide. Exemplary polysaccharides include cellulose, starch, amylose, dextran, poly(dextrose), glycogen, or a combination thereof. In certain embodiments, the polysaccharide is dextran. Very high molecular weight dextran has been found particularly useful as a porogen. For example, the molecular weight of the dextran may range from about 500,000 g/mol to about 10,000,000 g/mol, and in some embodiments, from about 1,000,000 g/mol to about 3,000,000 g/mol. In certain embodiments, the dextran has a molecular weight of approximately 2,000,000 g/mol. Dextrans with a molecular weight higher than 10,000,000 g/mol may also be used as porogens. Dextran may be used in any form (e.g., particles, granules, fibers, elongated fibers) as a porogen. In certain embodiments, fibers or elongated fibers of dextran are used as the porogen in the porous implant. Fibers of dextran may be formed using any known method including extrusion and precipitation. Fibers may be prepared by precipitation by adding an aqueous solution of dextran (e.g., 5-25% dextran) to a less polar solvent such as a 90-100% alcohol (e.g., ethanol) solution. The dextran precipitates out in fibers that are particularly useful as porogens in the porous implant. Dextran may be about 15% by weight to about 30% by weight of the porous implant. In certain embodiments, dextran is about 15% by weight, 20% by weight, 25% by weight, or 30% by weight. Higher and lower percentages of dextran may also be used. Once the porous implant with the dextran as a porogen is implanted into a subject, the dextran dissolves away very quickly. Within approximately 24 hours, substantially all of the dextran is out of the porous implant leaving behind pores in the porous implant. An advantage of using dextran in the porous implant is that dextran exhibits a hemostatic property in the extravascular space. Therefore, dextran in a porous implant can decrease bleeding at or near the site of implantation.

Small molecules including pharmaceutical agents may also be used as porogens in the porous implants. Examples of polymers that may be used as plasticizers include poly(vinyl pyrollidone), pullulan, poly(glycolide), poly(lactide), and poly(lactide-co-glycolide). Typically, low molecular weight polymers are used as porogens. In certain embodiments, the porogen is poly(vinyl pyrrolidone) or a derivative thereof. Plasticizers that are removed faster than the surrounding porous implant can also be considered porogens.

In certain embodiments, the porous implant may include a wetting or lubricating agent. Suitable wetting agents include water, organic protic solvents, organic non-protic solvents, aqueous solutions such as physiological saline, concentrated saline solutions, sugar solutions, ionic solutions of any kind, and liquid polyhydroxy compounds such as glycerol, polyethylene glycol (PEG), polyvinyl alcohol (PVA), and glycerol esters, and mixtures of any of these. Biological fluids may also be used as wetting or lubricating agents. Examples of biological fluids that may be used with the porous implants include blood, lymph, plasma, serum, or marrow. Lubricating agents may include, for example, polyethylene glycol, which can be combined with the polymer and other components to reduce viscosity or even coated on the walls of the delivery device. Alternatively or in addition, the particulate material may be coated with a polymer by sputtering or other techniques known to those skilled in the art.

In certain embodiments, the computer implemented method for producing a 3-D printed porous implant further comprises (i) removing the porogen prior to implantation of the porous implant at the intended tissue repair site or (ii) removing the porogen after implantation of the porous implant at the intended tissue repair site. Porosity can be created in many ways.

In certain embodiments, porosity can be created by 3-D printing of a polymer material, for example a polymer, onto a bed of particles which are not soluble in the polymer and which can be subsequently leached by a non-solvent for the polymer. In this case, the polymer which forms the device is printed onto a bed of particles such as salt, sugar, or polyethylene oxide. After the 3-D printing process is complete, the porous implant is removed from the powder bed and placed in a non-solvent for the implant material which will dissolve the particles. For example, polylactic acid in chloroform could be 3-D printed onto a bed of sugar particles, and the sugar can subsequently be leached with water.

In other embodiments, a solution containing the implant material can be 3-D printed onto a bed of particles which are partially soluble in the printed solvent. An example is printing a polylactic acid (PLA) solution onto a bed of polyethylene oxide (PEO) particles. This procedure may allow interpenetration of PEO into the surface of the PLA and improve surface properties of the final device. Following 3-D printing, the PEO can be leached with water.

In some embodiments, porosity of the implant material can be created by printing a solution containing the implant material onto a heated bed of polymer. An example is 3-D printing polylactic acid in chloroform onto a bed of PLA particles heated to 100° C. The boiling point of chloroform is 60° C, and it will thus boil on hitting the particle bed, causing a foam to form. This method of creating porosity is similar to 3-D printing a solution containing the implant material onto a bed containing a foaming agent, which is another way of achieving porosity.

### Implant Material

In various embodiments, the implant material comprises a carrier material and a bone material. In other implementations, the implant material comprises a natural material, a biodegradable carrier and a growth factor.

In some embodiments, the porous implant includes biodegradable polymers. Exemplary biodegradable materials include lactide-glycolide copolymers of any ratio (for example, 85:15, 40:60, 30:70, 25:75, or 20:80), poly(L-lactide-co-D,L-lactide), polyglyconate, poly(arylates), poly(anhydrides), poly(hydroxy acids), polyesters, poly(ortho esters), poly(alkylene oxides), polycarbonates, poly(propylene fumarates), poly(propylene glycol-co fumaric acid), poly(caprolactones), polyamides, polyesters, polyethers, polyureas, polyamines, polyamino acids, polyacetals, poly(orthoesters), poly(pyrolic acid), poly(glaxanone), poly(phosphazenes), poly(organophosphazene), polylactides, polyglycolides, poly(dioxanones), polyhydroxybutyrate, polyhydroxyvalyrate, polyhydroxybutyrate/valerate copolymers, poly(vinyl pyrrolidone), biodegradable polycyanoacrylates, biodegradable polyurethanes including glucose-based polyurethanes and lysine-based polyurethanes, and polysaccharides (e.g., chitin, starches, celluloses). In certain embodiments, the polymer used in the porous implant is poly(lactide-co-glycolide). The ratio of lactide and glycolide units in the polymer may vary. Particularly useful ratios are approximately 45-80% lactide to approximately 44-20% glycolide. In certain embodiments, the ratio is approximately 50% lactide to approximately 50% glycolide. In other certain embodiments, the ratio is approximately 65% lactide to approximately 45% glycolide. In other certain embodiments, the ratio is approximately 60% lactide to approximately 40% glycolide. In other embodiments, the ratio is approximately 70% lactide to approximately 30% glycolide. In other embodiments, the ratio is approximately 75% lactide to approximately 25% glycolide. In certain embodiments, the ratio is approximately 80% lactide to approximately 20% glycolide. In certain of the above embodiments, lactide is D,L-lactide. In other embodiments, lactide is L-lactide. In certain particular embodiments, RESOMER® 824 (poly-L-lactide-co-glycolide) (Boehringer Ingelheim) is used as the polymer in the porous implant. In certain particular embodiments, RESOMER® 504 (poly-D,L-lactide-co-glycolide) (Boehringer Ingelheim) is used as the polymer in the porous implant. In certain particular embodiments, PURASORB PLG (75/25 poly-L-lactide-co-glycolide) (Purac Biochem) is used as the polymer in the porous implant. In certain particular embodiments, PURASORB PG (polyglycolide) (Purac Biochem) is used as the polymer in the porous implant. In certain embodiments, the polymer is PEGylated-poly(lactide-co-glycolide). In certain embodiments, the polymer is PEGylated-poly(lactide). In certain embodiments, the polymer is PEGylated-poly(glycolide). In other embodiments, the polymer is polyurethane. In other embodiments, the polymer is polycaprolactone.

In certain embodiments, the biodegradable polymer is a copolymer of poly(caprolactone) and poly(lactide). For polyesters, such as poly(lactide) and poly(lactide-co-glycolide), the inherent viscosity of the polymer ranges from about 0.4 dL/g to about 5 dL/g. In certain embodiments, the inherent viscosity of the polymer ranges from about 0.6 dL/g to about 2 dL/g. In certain embodiments, the inherent viscosity of the polymer ranges from about 0.6 dL/g to about 3 dL/g. In certain embodiments, the inherent viscosity of the polymer ranges from about 1 dL/g to about 3 dL/g. In certain embodiments, the inherent viscosity of the polymer ranges from about 0.4 dL/g to about 1 dL/g. For poly(caprolactone), the inherent viscosity of the polymer ranges from about 0.5 dL/g to about 1.5 dL/g. In certain embodiments, the inherent viscosity of the poly(caprolactone) ranges from about 1.0 dL/g to about 1.5 dL/g. In certain embodiments, the inherent viscosity of the poly(caprolactone) ranges from about 1.0 dL/g to about 1.2 dL/g. In certain embodiments, the inherent viscosity of the poly(caprolactone) is about 1.08 dL/g.

Natural polymers, including collagen, polysaccharides, agarose, glycosaminoglycans, alginate, chitin, and chitosan, may also be employed. Tyrosine-based polymers, including but not limited to polyarylates and polycarbonates, may also be employed (Pulapura, et al., "Tyrosine-derived polycarbonates: Backbone-modified "pseudo"-poly(amino acids) designed for biomedical applications," Biopolymers, 1992, 32: 411-417; Hooper, et al., "Diphenolic monomers derived from the natural amino acid a-L-tyrosine: an evaluation of peptide coupling techniques," J. Bioactive and Compatible Polymers, 1995, 10:327-340, the contents of both of which are incorporated herein by reference). Monomers for tyrosine-based polymers may be prepared by reacting an L-tyrosine-derived diphenol compound with phosgene or a diacid (Hooper, 1995; Pulapura, 1992). Similar techniques may be used to prepare amino acid-based monomers of other amino acids having reactive side chains, including imines, amines, thiols, and the like. In one embodiment, the degradation products include bioactive materials, biomolecules, small molecules, or other such materials that participate in metabolic processes.

Polymers may be manipulated to adjust their degradation rates. The degradation rates of polymers are well characterized in the literature (see Handbook of Biodegradable Polymers, Domb, et al., eds., Harwood Academic Publishers, 1997, the entire contents of which are incorporated herein by reference). In addition, increasing the cross-link density of a polymer tends to decrease its degradation rate. The cross-link density of a polymer may be manipulated during polymerization by adding a cross-linking agent or promoter. After polymerization, cross-linking may be increased by exposure to UV light or other radiation. Co-monomers or mixtures of polymers, for example, lactide and glycolide polymers, may be employed to manipulate both degradation rate and mechanical properties.

In some embodiments, the porous implant comprises biodegradable polymeric or non-polymeric material. In some embodiments, the porous implant may include a biodegradable biopolymer that may provide immediate release, or sustained release of the biologically active material. For example, the biodegradable polymer comprises polyether ether ketone (PEEK). In some embodiments, the porous implant may comprise one or more poly (alpha-hydroxy acids), polyglycolide (PG), polyethylene glycol (PEG), conjugates of poly (alpha-hydroxy acids), polyorthoesters (POE), polyaspirins, polyphosphagenes, collagen, hydrolyzed collagen, gelatin, hydrolyzed gelatin, fractions of hydrolyzed gelatin, elastin, starch, pre-gelatinized starch, hyaluronic acid, chitosan, alginate, albumin, fibrin, vitamin E analogs, such as alpha tocopheryl acetate, d-alpha tocopheryl succinate, D,L-lactide, or L-lactide, caprolactone, dextrans, vinylpyrrolidone, polyvinyl alcohol (PVA), PVA-g-PLGA, PEGT-PBT copolymer (polyactive), methacrylates, PEO-PPO-PAA copolymers, PLGA-PEO-PLGA, PEG-PLG, PLA-PLGA, poloxamer 407, PEG-PLGA-PEG triblock copolymers, POE, SAIB (sucrose acetate isobutyrate), polydioxanone, methylmethacrylate (MMA), MMA and N-vinylpyyrolidone, polyamide, oxycellulose, copolymer of glycolic acid and trimethylene carbonate, polyesteramides, polyether ether ketone, polymethylmethacrylate, silicone, hyaluronic acid, chitosan, or combinations thereof.

In some embodiments, the porous implant may not be fully biodegradable. For example, the porous implant may comprise polyurethane, polyurea, polyether(amide), PEBA, thermoplastic elastomeric olefin, copolyester, and styrenic thermoplastic elastomer, steel, aluminum, stainless steel, titanium, metal alloys with high non-ferrous metal content and a low relative proportion of iron, carbon device, glass device, plastics, ceramics, methacrylates, poly (N-isopropylacrylamide), PEO-PPO-PEO (pluronics) or combinations thereof. Typically, these types of matrices may need to be removed after a certain amount of time.

In some embodiments, the porous implant comprises biodegradable polymers wherein the at least one biodegradable polymer comprises one or more of poly(lactide-co-glycolide) (PLGA), polylactide (PLA), polyglycolide (PGA), D-lactide, D,L-lactide, L-lactide, D,L-lactide-co-s-caprolactone, L-lactide-co-*s*-caprolactone, D,L-lactide-co-glycolide-co-*e*-caprolactone, poly(D,L-lactide-co-caprolactone), poly(L-lactide-co-caprolactone), poly(D-lactide-co-caprolactone), poly(D,L-lactide), poly(D-lactide), poly(L-lactide), poly(esteramide) or a combination thereof. In some embodiments, the biologically active material is encapsulated in a biodegradable polymer.

In various embodiments, the particle size distribution of the biodegradable polymer may be about 10 micrometers, 13 micrometers, 85 micrometers, 100 micrometers, 151 micrometers, 200 micrometers and all subranges there between. In some embodiments, at least 75% of the particles have a size from about 10 micrometers to about 200 micrometers. In some embodiments, at least 85% of the particles have a size from about 10 micrometers to about 200 micrometers. In some embodiments, at least 95% of the particles have a size from about 10 micrometers to about 200 micrometers. In some embodiments, all of the particles have a size from about 10 micrometers to about 200 micrometers. In some embodiments, at least 75% of the particles have a size from about 20 micrometers to about 180 micrometers. In some embodiments, at least 85% of the particles have a size from about 20 micrometers to about 180 micrometers. In some embodiments, at least 95% of the particles have a size from about 20 micrometers to about 180 micrometers. In some embodiments, all of the particles have a size from about 20 micrometers to about 180 micrometers.

In some embodiments, the porous implant comprises one or more polymers (e.g., PLA, PLGA, etc.) having a MW of from about 15,000 to about 150,000 Da or from about 25,000 to about 100,000 Da.

In some embodiments, the porous implant comprises at least one biodegradable material in a wt% of from about 99.5%, 99%, 98%, 97%, 96%, 95%, 94%, 93%, 92%, 91%, 90%, 89%, 88%, 87%, 86%, 85%, 84%, 83%, 82%, 81%, 80%, 79%, 78%, 76%, 75%, 74%, 73%, 72%, 71%, 70%, 65%, 60%, 55%, 50%, 45%, 35%, 25%, 20%, 15%, 10%, to about 5% based on the total weight of the porous implant. In some embodiments, the biodegradable polymer comprises a range of about 0.1% to about 20% based on the total weight of the porous implant. In some embodiments, the biodegradable polymer comprises a range of about 0.1% to about 15% based on the total weight of the osteoimplant. In some embodiments, the biodegradable polymer comprises 14%, 13%, 12%, 11%, 9%, 8%, 7%, 6%, or 5% based on the total weight of the matrix or the porous implant.

In some embodiments, the biodegradable polymer is present in the implant material in an amount of about 0.01 wt % to about 50 wt % or about 8.0 wt % to about 50 wt % of the porous implant. In some embodiments, the biodegradable polymer is present in an amount of about 0.1 wt % to about 10 wt %, about 10 wt % to about 20 wt %, about 20 wt % to about 30 wt %, about 30 wt % to about 40 wt %, or about 40 wt % to about 50 wt %. In other embodiments, the biodegradable polymer comprises 0.2 to 2% and the ceramic particles about 98 to 99.8% by weight of the porous implant.

### Plasticizers

The porous implant may also include one or more other components such as a plasticizer. Plasticizers are typically compounds added to polymers or plastics to soften them or make them more pliable. Plasticizers soften, make workable, or otherwise improve the handling properties of a polymer or porous implant. Plasticizers also allow the porous implant to be moldable at a lower temperature, thereby avoiding heat induced tissue necrosis during implantation. The plasticizer may evaporate or otherwise diffuse out of the porous implant over time, thereby allowing the porous implant to harden or set. Plasticizers are thought to work by embedding themselves between the chains of polymers. This forces the polymer chains apart and thus lowers the glass transition temperature of the polymer. Typically, the more plasticizer that is added, the more flexible the resulting polymer or porous implant will be.

In certain embodiments, the plasticizer is based on an ester of a polycarboxylic acid with linear or branched aliphatic alcohols of moderate chain length. For example, some plasticizers are adipate-based. Examples of adipate-based pasticizers include bis(2-ethylhexyl)adipate (DOA), dimethyl adipate (DMAD), monomethyl adipate (MMAD), and dioctyl adipate (DOA). Other plasticizers are based on maleates, sebacates, or citrates such as dibutyl maleate (DBM), diisobutylmaleate (DIBM), dibutyl sebacate (DBS), triethyl citrate (TEC), acetyl triethyl citrate (ATEC), tributyl citrate (TBC), acetyl tributyl citrate (ATBC), trioctyl citrate (TOC), acetyl trioctyl citrate (ATOC), trihexyl citrate (THC), acetyl trihexyl citrate (ATHC), butyryl trihexyl citrate (BTHC), and trimethylcitrate (TMC). Other plasticizers are phthalate based. Examples of phthalate-based plasticizers are N-methyl phthalate, bis(2-ethylhexyl) phthalate (DEHP), diisononyl phthalate (DINP), bis(n-butyl)phthalate (DBP), butyl benzyl phthalate (BBzP), diisodecyl phthalate (DOP), diethyl phthalate (DEP), diisobutyl phthalate (DIBP), and di-n-hexyl phthalate. Other suitable plasticizers include liquid polyhydroxy compounds such as glycerol, polyethylene glycol (PEG), triethylene glycol, sorbitol, monacetin, diacetin, and mixtures thereof. Other plasticizers include trimellitates (e.g., trimethyl trimellitate (TMTM), tri-(2-ethylhexyl) trimellitate (TEHTM-MG), tri-(n-octyl,n-decyl) trimellitate (ATM), tri-(heptyl,nonyl) trimellitate (LTM), n-octyl trimellitate (OTM)), benzoates, epoxidized vegetable oils, sulfonamides (e.g., N-ethyl toluene sulfonamide (ETSA), N-(2-hydroxypropyl)benzene sulfonamide (HP BSA), N-(n-butyl)butyl sulfonamide (BBSA-NBBS)), organophosphates (e.g., tricresyl phosphate (TCP), tributyl phosphate (TBP)), glycols/polyethers (e.g., triethylene glycol dihexanoate, tetraethylene glycol diheptanoate), and polymeric plasticizers. Other plasticizers are described in Handbook of Plasticizers (G. Wypych, Ed., ChemTec Publishing, 2004), which is incorporated herein by reference. In certain embodiments, other polymers are added to the porous implant as plasticizers. In certain particular embodiments, polymers with the same chemical structure as those used in the porous implant are used but with lower molecular weights to soften the overall porous implant. In certain embodiments, oligomers or monomers of the polymers used in the porous implant are used as plasticizers. In other embodiments, different polymers with lower melting points and/or lower viscosities than those of the polymer component of the porous implant are used. In certain embodiments, oligomers or monomers of polymers different from those used in the porous implant are used as plasticizers. In certain embodiments, the polymer used as a plasticizer is poly(ethylene glycol) (PEG). The PEG used as a plasticizer is typically a low molecular weight PEG such as those having an average molecular weight of 1000 to 10000 g/mol, in some aspects, from 4000 to 8000 g/mol. In certain embodiments, PEG 4000 is used in the porous implant. In certain embodiments, PEG 5000 is used in the porous implant. In certain embodiments, PEG 6000 is used in the porous implant. In certain embodiments, PEG 7000 is used in the porous implant. In certain embodiments, PEG 8000 is used in the porous implant. The plasticizer (PEG) is particularly useful in making more moldable porous implants that include poly(lactide), poly(D,L-lactide), poly(lactide-co-glycolide), poly(D,L-lactide-co-glycolide), or poly(caprolactone). In certain embodiments, PEG is grafted onto a polymer of the porous implant or is co-polymerized with a polymer of the porous implant.

A plasticizer may comprise from about 1% to about 40% of the porous implant by weight. In certain embodiments, the plasticizer is from about 10% to about 30% by weight. In certain embodiments, the plasticizer is approximately 10% by weight. In certain embodiments, the plasticizer is approximately 15% by weight. In other embodiments, the plasticizer is approximately 20% by weight. In certain embodiments, the plasticizer is approximately 25% by weight. In some embodiments, the plasticizer is approximately 30% by weight. In other embodiments, the plasticizer is approximately 33% by weight. In various embodiments, the plasticizer is approximately 40% by weight. In other embodiments, a plasticizer is not used in the porous implant. For example, in some polycaprolactone-containing porous implants, a plasticizer is not used.

Mannitol, trehalose, dextran, mPEG and/or PEG may be used as a plasticizer for the polymer. In some embodiments, the polymer and/or plasticizer may also be coated on the porous implant to provide the desired release profile. In some embodiments, the coating thickness may be thin, for example, from about 5, 10, 15, 20, 25, 30, 35, 40, 45 or 50 microns to thicker coatings 60, 65, 70, 75, 80, 85, 90, 95 or 100 microns to delay release of the biologically active material from the porous implant. In some embodiments, the range of the coating on the porous implant ranges from about 5 microns to about 250 microns or 5 microns to about 200 microns to delay release from the porous implant. Exemplary plasticizers include glycerol and poly(ethylene glycol) (PEG) (e.g., PEG 8000, PEG 6000, PEG 4000). In certain embodiments, the polymer component of the porous implant includes PEG blended, grafted, or co-polymerized with the polymer.

In various embodiments, the carrier material can be a metal, for example a biodegradable metal. The term "biodegradable metal" (BM) has been generally used to describe degradable metallic biomaterials for medical applications. Useful biodegradable metals include without limitation magnesium based BMs including pure magnesium, magnesium-calcium alloy, magnesium zinc alloy and iron based BMs include pure iron, iron manganese alloys.

In another embodiment, a magnesium alloy may include from about 90 to about 98 weight % magnesium, from about 0 to about 6 weight % aluminum, from about 0 to about 2 weight % zinc, and from about 0 to about 3% rare earth metal(s). In another embodiment, the magnesium alloy may be AE42, which includes 94 weight % magnesium, 4 weight % aluminum, and 2 weight % rare earth metal(s).

In accordance with some embodiments, the carrier material for use by the 3-D printer with, in, or on a bone material may be supplemented, further treated, or chemically modified with one or more bioactive agents or bioactive compounds. Bioactive agent or bioactive compound, as used herein, refers to a compound or entity that alters, inhibits, activates, or otherwise affects biological or chemical events. For example, bioactive agents may include, but are not limited to, osteogenic or chondrogenic proteins or peptides; DBM powder; collagen, insoluble collagen derivatives, etc., and soluble solids and/or liquids dissolved therein; anti-AIDS substances; anti-cancer substances; antimicrobials and/or antibiotics such as erythromycin, bacitracin, neomycin, penicillin, polymycin B, tetracyclines, biomycin, chloromycetin, and streptomycins, cefazolin, ampicillin, azactam, tobramycin, clindamycin and gentamycin, etc.; immunosuppressants; anti-viral substances such as substances effective against hepatitis; enzyme inhibitors; hormones; neurotoxins; opioids; hypnotics; anti-histamines; lubricants; tranquilizers; anti-convulsants; muscle relaxants and anti-Parkinson substances; anti-spasmodics and muscle contractants including channel blockers; miotics and anti-cholinergics; anti-glaucoma compounds; anti-parasite and/or anti-protozoal compounds; modulators of cell-extracellular matrix interactions including cell growth inhibitors and antiadhesion molecules; vasodilating agents; inhibitors of DNA, RNA, or protein synthesis; anti-hypertensives; analgesics; antipyretics; steroidal and non-steroidal anti-inflammatory agents; anti-angiogenic factors; angiogenic factors and polymeric carriers containing such factors; anti-secretory factors; anticoagulants and/or antithrombotic agents; local anesthetics; ophthalmics; prostaglandins; antidepressants; anti-psychotic substances; anti-emetics; imaging agents; biocidal/biostatic sugars such as dextran, glucose, etc.; amino acids; peptides; vitamins; inorganic elements; co-factors for protein synthesis; endocrine tissue or tissue fragments; synthesizers; enzymes such as alkaline phosphatase, collagenase, peptidases, oxidases and the like; polymer cell scaffolds with parenchymal cells; collagen lattices; antigenic agents; cytoskeletal agents; cartilage fragments; living cells such as chondrocytes, bone marrow cells, mesenchymal stem cells; natural extracts; genetically engineered living cells or otherwise modified living cells; expanded or cultured cells; DNA delivered by plasmid, viral vectors, or other member; tissue transplants; autogenous tissues such as blood, serum, soft tissue, bone marrow, or the like; bioadhesives; bone morphogenetic proteins (BMPs); osteoinductive factor (IFO); fibronectin (FN); endothelial cell growth factor (ECGF); vascular endothelial growth factor (VEGF); cementum attachment extracts (CAE); ketanserin; human growth hormone (HGH); animal growth hormones; epidermal growth factor (EGF); interleukins, for example, interleukin-1 (IL-1), interleukin-2 (IL-2); human alpha thrombin; transforming growth factor (TGF-beta); insulin-like growth factors (IGF-1, IGF-2); parathyroid hormone (PTH); platelet derived growth factors (PDGF); fibroblast growth factors (FGF, BFGF, etc.); periodontal ligament chemotactic factor (PDLGF); enamel matrix proteins; growth and differentiation factors (GDF); hedgehog family of proteins; protein receptor molecules; small peptides derived from growth factors above; bone promoters; cytokines; somatotropin; bone digesters; antitumor agents; cellular attractants and attachment agents; immuno-suppressants; permeation enhancers, for example, fatty acid esters such as laureate, myristate and stearate monoesters of polyethylene glycol, enamine derivatives, alpha-keto aldehydes; and nucleic acids.

In certain embodiments, the bioactive agent may be a drug, a growth factor, a protein or a combination thereof. In some embodiments, the bioactive agent may be a growth factor, cytokine, extracellular matrix molecule, or a fragment or derivative thereof, for example, a protein or peptide sequence such as RGD.

In some embodiments, the carrier material may have a modulus of elasticity in the range of from about 1 x 10² dynes/cm² to about 6 x 10⁵ dynes/cm², or 2 x 10⁴ to about 5 x 10⁵ dynes/cm², or 5 x 10⁴ to about 5 x 10⁵ dynes/cm². After the device is administered to the target site, the carrier material may have a modulus of elasticity in the range of about 1 x 10² to about 6 x 10⁵ dynes/cm², or 2 x 10⁴ to about 5 x 10⁵ dynes/cm², or 5 x 10⁴ to about 5 x 10⁵ dynes/cm².

In other implementations, the implant material further comprises (i) soft tissue particles, inorganic polymers or a combination thereof; (ii) the soft tissue particles comprises cartilage particles; (iii) inorganic particles comprising hydroxyapatite, calcium HA, carbonated calcium HA, beta-tricalcium phosphate (beta-TCP), alpha-tricalcium phosphate (alpha-TCP), amorphous calcium phosphate (ACP), octacalcium phosphate (OCP), tetracalcium phosphate, biphasic calcium phosphate (BCP), anhydrous dicalcium phosphate (DCPA), dicalcium phosphate dihydrate (DCPD), anhydrous monocalcium phosphate (MCPA), monocalcium phosphate monohydrate (MCPM), and combinations thereof.

In some embodiments, the porous implant may be seeded with harvested bone cells and/or bone tissue, such as, for example, cortical bone, autogenous bone, allogenic bones and/or xenogenic bone as discussed elsewhere in this application. In some embodiments, the porous implant may be seeded with harvested cartilage cells and/or cartilage tissue (for example, autogenous, allogenic, and/or xenogenic cartilage tissue). In one aspect, before insertion into the target tissue site, the porous implant can be wetted with the graft bone tissue/cells, usually with bone tissue/cells aspirated from the patient, at a ratio of about 3:1, 2:1, 1:1, 1:3 or 1:2 by volume. The bone tissue/cells are permitted to soak into the porous implant provided, and the porous implant may be kneaded by hand or machine, thereby obtaining a pliable consistency that may subsequently feed into the 3-D printer. In some embodiments, the harvested bone and/or cartilage cells can be mixed with the growth factor and seeded in the interior of the porous implant.

In some embodiments, in order to form a porous implant that can be used as a cartilage graft, the porous implant may contain an inorganic material, such as an inorganic ceramic and/or bone substitute material. Exemplary inorganic materials or bone substitute materials include, but are not limited to aragonite, dahlite, calcite, amorphous calcium carbonate, vaterite, weddellite, whewellite, struvite, urate, ferrihydrate, francolite, monohydrocalcite, magnetite, goethite, dentin, calcium carbonate, calcium sulfate, calcium phosphosilicate, sodium phosphate, calcium aluminate, calcium phosphate, hydroxyapatite, alpha-tricalcium phosphate, dicalcium phosphate, *B*-tricalcium phosphate, tetracalcium phosphate, amorphous calcium phosphate, octacalcium phosphate, BIOGLASS™, fluoroapatite, chlorapatite, magnesium-substituted tricalcium phosphate, carbonate hydroxyapatite, substituted forms of hydroxyapatite (e.g., hydroxyapatite derived from bone may be substituted with other ions such as fluoride, chloride, magnesium sodium, potassium, etc.), or combinations or derivatives thereof.

In other embodiments, in order to form a porous implant that can be used as an osteochondral graft, the porous implant may be fabricated from a natural material which can facilitate bio-ingrowth of the osteochondral graft within the articular cartilage defect site. The natural materials may include, but are not limited to, collagen, chitosan, alginate, hyaluronic acid, silk, elastin, bone allograft, and osteochondral allograft, ceramics, or combinations thereof. Illustrative synthetic biocompatible materials, which may act as suitable matrices for portions of the graft can include poly-alpha-hydroxy acids (e.g. polylactides, polycaprolactones, polyglycolides and their copolymers, such as lactic acid/glycolic acid copolymers and lactic acid/caprolactone copolymers), polyanhydrides, polyorthoesters, polydioxanone, segmented block copolymers of polyethylene glycol and polybutylene terephtalate (Polyactive), poly (trimethylenecarbonate) copolymers, tyrosine derivative polymers, such as tyrosine-derived polycarbonates, or poly (ester-amides). Suitable ceramic materials include, for example, calcium sulfate, calcium phosphate ceramics such as tricalcium phosphate, hydroxyapatite, and biphasic calcium phosphate.

In some embodiments, a growth factor is disposed on the graft, the growth factor facilitating bio-ingrowth of the graft into an articular cartilage defect site. Useful growth factors include without limitations, BMP-2, BMP-7, GDF-5, TGF, PDGF, statin or combinations thereof.

### Bone Material for the Implant Material

In other embodiments, porous implants prepared by 3-D printing for an intended tissue repair site also comprise bone material as described in this disclosure above in connection with 3-D printed mesh bags. In various embodiments, the bone material may be particulated such as, for example, in bone powder or fiber form. Additional bone material useful for the 3-D printed implant is discussed below.

In various implementations, the bone material useful for the computer implemented method for producing the porous implant of this application and which can be used with a 3-D printer includes allograft, demineralized bone matrix fiber, demineralized bone chips or a combination thereof. In some embodiments, the porous implant can contain demineralized bone material disposed therein. The demineralized bone material can comprise demineralized bone, powder, chips, triangular prisms, spheres, cubes, cylinders, shards, fibers or other shapes having irregular or random geometries. These can include, for example, "substantially demineralized," "partially demineralized," or "fully demineralized" cortical and cancellous bone. These also include surface demineralization, where the surface of the bone construct is substantially demineralized, partially demineralized, or fully demineralized, yet the body of the bone construct is fully mineralized. In some embodiments, the covering may comprise some fully mineralized bone material. The configuration of the bone material can be obtained by milling, shaving, cutting or machining whole bone as described in for example U.S. Pat. No. 5,899,939. The entire disclosure is herein incorporated by reference into the present disclosure.

In some embodiments, the porous implant comprises elongated demineralized bone fibers having an average length to average thickness ratio or aspect ratio of the fibers from about 50:1 to about 1000:1. In overall appearance, the elongated demineralized bone fibers can be in the form of threads, narrow strips, or thin sheets. The elongated demineralized bone fibers can be substantially linear in appearance or they can be coiled to resemble springs. In some embodiments, the elongated demineralized bone fibers are of irregular shapes including, for example, linear, serpentine or curved shapes. The elongated bone fibers can be demineralized, however some of the original mineral content may be retained when desirable for a particular embodiment.

In some embodiments, the porous implant comprises elongated demineralized bone fibers and chips. In some embodiments, the porous implant comprises fully demineralized fibers and surface demineralized chips. In some embodiments, the ratio of fibers to chips or powders is from about 5, 10, 15, 20, 25, 30, 35, 40, or 45 fibers to about 30, 35, 40, 45, 50, 55, 60, 65, or 70 chips.

In certain embodiments, the bone graft material that can be placed in the porous implant described in this disclosure can be demineralized bone material (e.g., fibers, chips, powder, or a combination thereof). In some embodiments, the demineralized bone fibers can be elongated and have an aspect ratio of at least from about 50:1 to at least about 1000:1. Such elongated bone fibers can be readily obtained by any one of several methods, for example, by milling or shaving the surface of an entire bone or relatively large section of bone.

In other embodiments, the length of the fibers can be at least about 3.5 cm and can have an average width from about 20 mm to about 1 cm. In various embodiments, the average length of the elongated fibers can be from about 3.5 cm to about 6.0cm and the average width from about 20 mm to about 1 cm. In other embodiments, the elongated fibers can have an average length from about 4.0 cm to about 6.0 cm and an average width from about 20 mm to about 1 cm.

In yet other embodiments, the diameter or average width of the elongated fibers is, for example, not more than about 1 cm, not more than 0.5 cm, or not more than about 0.01 cm. In still other embodiments, the diameter or average width of the fibers can be from about 0.01 cm to about 0.4 cm or from about 0.02 cm to about 0.3 cm.

In another embodiment, the aspect ratio of the fibers can be from about 50:1 to about 950:1, from about 50:1 to about 750:1, from about 50:1 to about 500:1, from about 50:1 to about 250:1; or from about 50:1 to about 100:1. Fibers according to this disclosure can, in some aspects, have an aspect ratio from about 50:1 to about 1000:1, from about 50:1 to about 950:1, from about 50:1 to about 750:1, from about 50:1 to about 600:1, from about 50:1 to about 350:1, from about 50:1 to about 200:1, from about 50:1 to about 100:1, or from about 50:1 to about 75:1.

In some embodiments, the bone chips can be used and they can be combined with bone fibers, where the chips to fibers ratio is about 90:10, 80:20, 70:30, 60:40, 50:50, 40:60, 30:70, 20:80 and/or 10:90. In various embodiments, a surface demineralized bone chips to fibers ratio is about 90:10, 80:20, 70:30, 60:40, 50:50, 40:60, 30:70, 20:80 and/or 10:90 that can be used in the device. In some embodiments, a surface demineralized chips to fully demineralized fibers ratio is about 90:10, 80:20, 70:30, 60:40, 50:50, 40:60, 30:70, 20:80 and/or 10:90 that can be used in the porous implant.

In some embodiments, the porous implant comprises demineralized bone matrix fibers and demineralized bone matrix chips in a 30:60 ratio. In some embodiments, the porous implant comprises demineralized bone fibers and surface demineralized bone chips in a ratio of 25:75 to about 75:25 fibers to chips.

In some embodiments, the porous implant comprises mineral particles that offer compression resistance. In some embodiments, the particles comprise at least 10%, 15%, 20%, 25%, 30%, 35%, 40%, 45%, 50%, 55%, 60%, 65%, 70%, 75%, 80%, 85%, 90%, or 95% by weight of the porous implant. In some embodiments, the particles are predominantly any shape (e.g., round, spherical, elongated, powders, chips, fibers, cylinders, etc.). In some embodiments, the porous implant comprises mineral particles in an amount of about 0.1 wt % to about 95 wt % of the porous implant. In some embodiments, the porous implant comprises mineral particles in an amount of about 50 wt % to about 80 wt % of the porous implant. In some embodiments, the mineral particles in the porous implant comprise 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, or 79% by weight of the porous implant.

In some embodiments, the mineral particles are present in an amount of about 0.1 wt % to about 30 wt % of the porous implant. In some embodiments, the mineral particles are present in an amount between about 0.01 wt % to about 50 wt % of the porous implant. In some embodiments, the mineral particles are present in an amount between about 7.0 wt % to about 50 wt % of the porous implant. In some embodiments, the mineral particles are present in an amount of about 0.1 wt % to about 10 wt %, about 10 wt % to about 20 wt %, about 20 wt % to about 30 wt %, about 30 wt % to about 40 wt %, or about 40 wt % to about 50 wt %.

In some embodiments, the porosity of the particles comprises from about 0% to about 50%, in some embodiments, the porosity of the particles comprises from about 5% to about 25%. In some embodiments, the particles are not entangled with each other but contact each other and portions of each particle overlap in the matrix of the porous implant to provide compression resistance. In some embodiments, at least 50%, 55%, 60%, 65%, 70%, 75%, 80%, 85%, 90%, 95%, or more of the particles overlap each other in the porous implant.

In some embodiments, the particles are randomly distributed throughout the matrix of the porous implant. In other embodiments, the particles are uniformly or evenly distributed throughout the matrix of the porous implant. In some embodiments, the particles may be dispersed in the porous implant using a dispersing agent. In other embodiments, the particles may be stirred in the polymer and the mechanical agitation will distribute the particles in the matrix of the porous implant until the desired distribution is reached (e.g., random or uniform).

In some embodiments, the matrix of the porous implant may be seeded with harvested bone cells and/or bone tissue, such as for example, cortical bone, autogenous bone, allogenic bones and/or xenogenic bone. In some embodiments, the matrix of the porous implant may be seeded with harvested cartilage cells and/or cartilage tissue (e.g., autogenous, allogenic, and/or xenogenic cartilage tissue). For example, before insertion into the target tissue site, the matrix of the porous implant can be wetted with the graft bone tissue/cells, usually with bone tissue/cells aspirated from the patient, at a ratio of about 3:1, 2:1, 1:1, 1:3 or 1:2 by volume.

The bone tissue/cells are permitted to soak into the matrix of the porous implant, and the matrix may be kneaded by hand or machine, thereby obtaining a pliable and cohesive consistency that may subsequently be used as ink for the 3-D printer. In some embodiments, the matrix of the porous implant provides a malleable, non-water soluble carrier that permits accurate placement and retention of the shaped porous 3-D printed implant at the tissue repair site. In some embodiments, the harvested bone and/or cartilage cells can be mixed with a statin to form another embodiment of the 3-D printed porous implant described in this disclosure.

In some embodiments, tissue will infiltrate the matrix of the porous implant to a degree of about at least 50 percent within about 1 month to about 6 months after implantation of the 3-D printed porous implant. In some embodiments, about 75 percent of the matrix of the porous implant will be infiltrated by tissue within about 2-3 months after implantation of the 3D printed porous implant. In some embodiments, the composite will be substantially, e.g., about 90 percent or more, submerged in or enveloped by tissue within about 6 months after implantation of the composite. In some embodiments, the matrix of the 3D printed porous implant will be completely submerged in or enveloped by tissue within about 9 to about 12 months after implantation.

### Curable Ink

In several implementations, the carrier material comprises an ink that dries, is cured or reacts to form a porous, biodegradable, biocompatible material that is osteoinductive and has a load bearing strength comparable to bone. The ink can, in some aspects, be supplied in the form of a precursor powder and a precursor liquid. These may be fed to separate containers in the 3-D printer. Prior to printing, a quantity of the precursor powder and the precursor liquid may be mixed to form the ink to be used for printing the custom porous implant. The printing may be accomplished by delivering quantities of the ink via a suitably sized print nozzle that may be moved in a raster scan with respect to the custom porous implant being printed.

The precursor powder of the ink can contain a variety of ingredients such as, but not limited to, demineralized allograft bone matrix (DMB), a radical polymerization initiator, for example, dibenzoyl peroxide or some combination thereof. The precursor liquid may contain a variety of ingredients such as, for example, methyl methacrylate (MMA), a radiopaque compound, an antibiotic, and a compound to increase the biodegradability, or a combination thereof. In some aspects, a radiopaque compound can be, without limitations, zirconium dioxide or barium sulfate or a combination thereof. In other aspects, useful antibiotics include without limitations amoxicillin, doxycycline, gentamicin, clindamycin or a combination thereof. Other additives which may increase the biodegradability of the ink include, without limitations, cellulose acetate (CA), or cellulose acetate phthalate (CAP) or a combination thereof.

In alternate embodiments, the ink may include synthetic bone substitutes, and other slow reabsorbing biocompatible, bioactive adhesives as discussed above. Examples of artificial bone substitutes include without limitations hydroxyapatite, synthetic calcium phosphate ceramic or a combination thereof. These may be used instead of, or with natural bone particulates such as without limitations allograft, fully demineralized bone fibers and surface demineralized bone chips, or a combination thereof. These may be used with synthetically produced bone morphogenetic agents such as, without limitation, recombinant human bone morphogenetic protein rhBMP-2. Alternate inks may also include other biocompatible, bioactive adhesives such as, for example, glass polyalkenoate cements, oleic methyl ester based adhesives, or a combination thereof.

In accordance with other embodiments, the carrier material for use by the 3-D printer with, in, or on a bone material may be supplemented with other microparticles, and/or nanoparticles which can be incorporated before or during 3-D printing in order to impart certain desirable mechanical, magnetic, piezoelectric properties and/or to stimulate cellular functions upon implantation under a variety of *in vivo* or *in vitro* conditions to the custom made porous implant described in this disclosure.

### Composite Ink

In various embodiments, an ink for use with a 3-D printer system described herein is a composite ink. In some aspects, the 3-D printer can use as ink a composite filament comprising a polymer and chips, microparticles, nanoparticles and/or fibers of demineralized bone, non-demineralized bone or a combination thereof. In some embodiments, the composite filament comprises a bioerodible polymer, one or more ceramics and demineralized bone matrix where the demineralized bone matrix particles are embedded within or coated on the surface of the bioerodible polymer and ceramic particles. In a further embodiment, the demineralized bone matrix particles are dispersed throughout the bioerodible polymer and ceramic particles. In some embodiments, the demineralized bone matrix (DBM) particles are dispersed homogeneously throughout the polymer and ceramic particles.

In certain embodiments, the composite filament comprises a combination of fibers of demineralized bone matrix from allograft bone and fibers of non-allograft bone material, the fibers of non-allograft bone material comprising non-fibrous demineralized bone matrix particles embedded within or disposed on the fibers of the non-allograft bone material.

In other embodiments, the fibers of non-allograft bone material comprise a bioerodible polymer and one or more ceramics either alone or in combination. In some embodiments, the fibers of non-allograft bone material comprise ceramics and collagen, hyaluronic acid, chitosan, keratin, and derivatives thereof, either alone or in combination. In some embodiments, the bioerodible polymer is collagen. In some embodiments, the collagen is porous. In other embodiments, the diameter of the fibers of allograft bone and non-allograft bone material is between about 50 µm and about 1 mm. In some embodiments, the diameter of the fibers of allograft bone and non-allograft bone material is between about 75 nm and about 250 nm. In some embodiments, the length of the fibers of the allograft bone and non-allograft material is between about 5 mm and about 30 mm. In some embodiments, the composite filament composition contains a bioactive agent. In some embodiments, the ceramic is a calcium phosphate ceramic and/or silicon ceramic. In other embodiments, the ceramic is tricalcium phosphate. In some embodiments, the ratio of fibers of demineralized bone matrix from allograft bone to fibers of non-allograft material ranges from about 80:20 to about 70:30, or from about 40:60 to about 60:40. In some embodiments, the ratio of fibers of demineralized bone matrix from allograft bone to fibers of non-allograft material is about 50:50. In some embodiments, the non-fibrous demineralized bone matrix particles embedded within or disposed on the fibers of non-allograft bone material range in diameter size from between about 50 µm and about 30 mm.

The fibers of the non-allograft bone material comprise a bioerodible polymer and a synthetic ceramic to which demineralized bone matrix particles are embedded either within and/or on the surface of the non-allograft bone material. The demineralized bone matrix particles are non-fibrous. In other embodiments, the particles are powders, microspheres, sponges, pastes, gels, and/or granules. In one embodiment, the particles are powders.

DBM particles for use in the present disclosure can be obtained commercially or can be prepared by known techniques. In general, advantageous, osteoinductive DBM materials can be prepared by decalcification of cortical and/or cancellous bone, often by acid extraction. This process can be conducted so as to leave collagen, noncollagenous proteins, and growth factors together in a solid matrix. Methods for preparing such bioactive demineralized bone matrix are known, in respect of which reference can be made to U.S. Pat. Nos. 5,073,373; 5,484,601; and 5,284,655, as examples. DBM products are also available commercially, including for instance, from sources such as Regeneration Technologies, Inc. (Alachua, Fla.), The American Red Cross (Arlington, Va.), and others. For the purposes of this disclosure, any shape and particle size of DBM can be used, including DBM in the form of fragments, slices, pellets, shavings, granules, fibers, or powder, as well as demineralized whole bone. In various embodiments, the demineralized bone is of a small particle size, and in the form of powder. In certain embodiments, the particulate DBM material can have an average particle size of less than about 100 to about 1000 microns. For instance, the DBM material can have particle sizes in the range of 50 to 850 microns. DBM materials that are solely osteoconductive can be prepared using similar techniques that have been modified or supplemented to remove or inactivate (e.g. by crosslinking or otherwise denaturing) components in the bone matrix responsible for osteoinductivity. Osteoinductive and/or osteoconductive DBM materials used in the present disclosure can be derived from human donor tissue, especially in regard to implant devices intended for use in human subjects.

In regard to the incorporated materials considered on a dry weight basis, the particulate DBM material, which are embedded onto the non-allograft fibers, can constitute about 10% to about 50% of the compositions, about 20% to about 40%, and about 25% to about 35% by weight. In various embodiments, particulate DBM material embedded onto the non-allograft fibers can constitute about 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49% or about 50% of the composite filament. In a similar vein, composite filaments can contain about 5% to about 30% by weight insoluble collagen particulate on a dry weight basis, about 8% to about 20%, and about 10% to about 15%; and can contain the ceramics at a level of about 1% to about 20% on a dry weight basis, about 5% to about 15%, and about 8% to about 12%. It will be understood, however, that other amounts of these materials can be used within the broader aspects of the present disclosure.

In some embodiments, the demineralized bone fibers of allograft bone and fibers of non-allograft bone have an average length to average thickness ratio or aspect ratio of the fibers from about 50:1 to about 1000:1. In overall appearance, the elongated demineralized bone fibers can be in the form of threads, narrow strips, and/or thin sheets. The elongated demineralized bone fibers can be substantially linear in appearance or they can be coiled to resemble springs. In some embodiments, the elongated demineralized bone fibers are of irregular shapes including, for example, linear, serpentine and/or curved shapes. The elongated bone fibers can be demineralized however some of the original mineral content may be retained when desirable for a particular embodiment. The porous implant composition of the composite filament may further comprise mineralized bone particles as known in the art.

The bioerodible polymer will exhibit dissolution when placed in a mammalian body and may be hydrophilic (e.g., collagen, hyaluronic acid, polyethylene glycol). Synthetic polymers are suitable according to the present disclosure, as they are biocompatible and available in a range of copolymer ratios to control their degradation.

In some embodiments, hydrophobic polymers (e.g. poly(lactide-co-glycolyde), polyanhydrides) may be used. Alternatively, a combination of hydrophilic and hydrophobic polymers may be used in the porous implant of the disclosure.

Exemplary materials may include biopolymers and synthetic polymers such as human skin, human hair, bone, collagen, fat, thin cross-linked sheets containing fibers and/or fibers and chips, polyethylene glycol (PEG), chitosan, alginate sheets, cellulose sheets, hyaluronic acid sheet, as well as copolymer blends of poly (lactide-co-glycolide) PLGA.

In some embodiments, the particles disclosed herein can also include other biocompatible and bioresorbable substances. These materials may include, for example, natural polymers such as proteins and polypeptides, glycosaminoglycans, proteoglycans, elastin, hyaluronic acid, dermatan sulfate, gelatin, or mixtures or composites thereof. Synthetic polymers may also be incorporated into the porous implant composites. These include, for example biodegradable synthetic polymers such as polylactic acid, polyglycolide, polylactic polyglycolic acid copolymers ("PLGA"), polycaprolactone ("PCL"), poly(dioxanone), poly(trimethylene carbonate) copolymers, polyglyconate, poly(propylene fumarate), poly(ethylene terephthalate), poly(butylene terephthalate), polyethylene glycol, polycaprolactone copolymers, polyhydroxybutyrate, polyhydroxyvalerate, tyrosine-derived polycarbonates and any random or (multi-)block copolymers, such as bipolymer, terpolymer, quaterpolymer, that can be polymerized from the monomers related to previously-listed homo- and copolymers.

In some embodiments, the bioerodible polymer is collagen. Collagen has excellent histocompatibility without antibody formation or graft rejection. Any suitable collagen material may be used, including known collagen materials, or collagen materials as disclosed in U.S. patent application Ser. No. 12/030,181, filed Feb. 12, 2008, hereby incorporated by reference in its entirety. Various collagen materials can be used, alone or in combination with other materials.

Insoluble collagen material for use in the disclosure can be derived from natural tissue sources, (e.g. xenogenic, allogenic, or autogenic relative to the recipient human or other patient) or recombinantly prepared. Collagens can be subclassified into several different types depending upon their amino acid sequence, carbohydrate content and the presence or absence of disulfide crosslinks. Types I and III collagen are two subtypes of collagen and may be used in the present disclosure. Type I collagen is present in skin, tendon and bone, whereas Type III collagen is found primarily in skin. The collagen used in implants of the disclosure can be obtained from skin, bone, tendon, or cartilage and purified by methods well known in the art and industry. Alternatively, the collagen can be purchased from commercial sources.

The collagen can be atelopeptide collagen and/or telopeptide collagen. Still further, either or both of non-fibrillar and fibrillar collagen can be used. Non-fibrillar collagen is collagen that has been solubilized and has not been reconstituted into its native fibrillar form.

Suitable collagen products are available commercially, including for example from Kensey Nash Corporation (Exton, Pa.), which manufactures a fibrous collagen known as Semed F, from bovine hides. Collagen materials derived from bovine hides are also manufactured by Integra Life Science Holding Corporation (Plainsboro, N.J.). Naturally-derived or recombinant human collagen materials are also suitable for use in the disclosure. Illustratively, recombinant human collagen products are available from Fibrogen, Inc. (San Francisco, Calif.).

The solid particulate collagen incorporated into the implant can be in the form of intact or reconstituted fibers, or randomly-shaped particles, for example. In certain beneficial embodiments, the solid particulate collagen will be in the form of particles derived from a sponge material, for example by randomly fragmenting the sponge material by milling, shredding or other similar operations. Such particulated sponge material can have an average maximum particle diameter of less than about 6 mm, less than about 3 mm, and/or in the range of about 0.5 mm to 2 mm. Such materials can, for example, be obtained by milling or grinding a porous sponge material and sieving the milled or ground material through a screen having openings sized about 6 mm or smaller, desirably about 0.5 mm to about 2 mm. Retch grinders with associated sieves are suitable for these purposes. Other sources of chemically crosslinked, particulate collagen, in fiber, irregular or other shapes, can also be used. These crosslinked particulate materials can be provided as starting materials for preparing implants as disclosed herein, and these particles can be individually crosslinked. Crosslinked solid collagen particles can be used in combination with non-crosslinked collagen in compositions of the disclosure, wherein the non-crosslinked collagen can be solid (insoluble) or soluble collagen, or combinations thereof. Such crosslinked and non-crosslinked collagen mixtures can be used, for example, to modulate the residence time of the collagen portion of the porous implant compositions in vivo.

Suitable crosslinking agents include, but are not limited to, mono- and dialdehydes, including glytaraldehyde and formaldehyde; polyepoxy compounds such as glycerol; and sugars such as glucose. In one embodiment, the crosslinking agent is glycerol.

Exemplary collagen particles can be obtained from various collagen sources including human or non-human (bovine, ovine, and/or porcine), as well as recombinant collagen or combinations thereof. Examples of suitable collagen include, but are not limited to, human collagen type I, human collagen type II, human collagen type III, human collagen type IV, human collagen type V, human collagen type VI, human collagen type VII, human collagen type VIII, human collagen type IX, human collagen type X, human collagen type XI, human collagen type XII, human collagen type XIII, human collagen type XIV, human collagen type XV, human collagen type XVI, human collagen type XVII, human collagen type XVIII, human collagen type XIX, human collagen type XXI, human collagen type XXII, human collagen type XXIII, human collagen type XXIV, human collagen type XXV, human collagen type XXVI, human collagen type XXVII, and human collagen type XXVIII, or combinations thereof. Collagen further may comprise hetero- and homo-trimers of any of the above-recited collagen types. In some embodiments, the collagen comprises hetero- or homo-trimers of human collagen type I, human collagen type II, human collagen type III, or combinations thereof. In some embodiments, the collagen is porous.

In some embodiments, the bioerodible polymer may be hyaluronic acid, chitosan, chitin, keratin, cellulose, glycosaminoglycans and derivatives thereof (e.g. esters of hyaluronic acid) or others of synthetic origin which may be used as an alternative to or in combination with collagen.

In some embodiments, the synthetic ceramics disclosed herein may be selected from one or more materials comprising calcium phosphate ceramics or silicon ceramics. Biological glasses such as calcium-silicate-based bioglass, silicon calcium phosphate, tricalcium phosphate (TCP), biphasic calcium phosphate, calcium sulfate, hydroxyapatite, coralline hydroxyapatite, silicon carbide, silicon nitride (Si₃N₄), and biocompatible ceramics may be used. In some embodiments, the ceramic is tri-calcium phosphate or biphasic calcium phosphate and silicon ceramics. In some embodiments, the ceramic is tricalcium phosphate.

In some embodiments, the ceramics are a combination of a calcium phosphate ceramic and a silicon ceramic. In some embodiments, the calcium phosphate ceramic is resorbable biphasic calcium phosphate (BCP) or resorbable tri-calcium phosphate (TCP), most preferably resorbable TCP.

Biphasic calcium phosphate can have a tricalcium phosphate:hydroxyapatite weight ratio of about 50:50 to about 95:5, about 70:30 to about 95:5, about 80:20 to about 90:10, or about 85:15. The mineral material can be a granular particulate having an average particle diameter between about 0.2 and 5.0 mm, between about 0.4 and 3.0 mm, or between about 0.4 and 2.0 mm.

The ceramics of the disclosure may also be oxide ceramics such as alumina (Al₂O₃) or zirconia (ZrO₂) or composite combinations of oxides and non-oxides such as silicon nitride.

The ceramics of the disclosure may be porous and may have pore sizes large enough to permit osteoinduction via invasion of the material by bone forming cells. Examples of porous ceramics are hydroxyapatite and TCP.

In some embodiments, the implant may contain non-allograft bone material including from about 40 to about 60 weight percent collagen, from about 20 to about 50 weight percent DBM, and from about 10 to about 50 weight percent ceramics. In some embodiments, the ratio of DBM particles to collagen and/or ceramics is about 5:1, about 4:1, about 3:1, about 2:1, about 1:1, about 1:5, about 1:4, about 1:3, or about 1:2. In some embodiments, the ratio of DBM particles to collagen and/or ceramics is about 1.5:0.5, about 1:1, or about 0.5:1.5.

In some embodiments, the particles disclosed herein also include synthetic ceramics that are effective to provide a scaffold for bone growth and which are completely bioresorbable and biocompatible. The synthetic ceramics should provide high local concentrations of calcium, phosphate and silicon ions that act as a nidus for de-novo bone formation. The use of such resorbable ceramics provides many advantages over alternative conventional materials. For instance, it eliminates the need for post-therapy surgery for removal and degrades in the human body to biocompatible, bioresorbable products.

In other embodiments, the composite filament for use in a 3-D printer system described herein is a curable composite ink. The composite ink comprises a curable material and, optionally a colorant dispersed in the ink, in amount from about 0.01 to about 5% by weight of the composite ink. In some cases, the colorant is present in the composite ink in an amount between about 0.01 and 3 weight %, between about 0.01 and 1 weight %, between about 0.05 and 5 weight %, between about 0.05 and 3 weight %, between about 0.05 and 1 weight %, between about 0.1 and 5 weight %, between about 0.1 and 3 weight %, or between about 0.1 and 1 weight %. In some aspects, the colorant of a composite ink comprises an inorganic pigment, such as TiO₂ and ZnO. In some embodiments, the colorant of a composite ink comprises a colorant for use in a RGB, sRGB, CMY, CMYK, L*a*b*, or Pantone® colorization scheme. Moreover, in some cases, a particulate colorant described herein has an average particle size of less than 500 nm, such as an average particle size of less than 400 nm, less than 300 nm, less than 250 nm, less than 200 nm, or less than 150 nm. In some instances, a particulate colorant has an average particle size of 50-1000 nm, 50-500 nm, 50-400 nm, 50-300 nm, 50-200 nm, 70-500 nm, 70-300 nm, 70-250 nm, or 70-200 nm.

In certain embodiments, the curable material included in the composite filament is present in an amount up to about 99 weight %, up to about 95 weight %, up to about 90 weight %, or up to about 80 weight %, based on the total weight of the composite ink. In some cases, a composite ink described herein comprises about 10-95 weight % curable material, based on the total weight of the carrier ink. In some embodiments, a carrier ink comprises about 20-80 weight % curable material, about 30-70 weight % curable material, or about 70-90 weight % curable material.

In some cases, a curable material comprises one or more polymerizable components. As used herein, a polymerizable component comprises a component that can be polymerized or cured to provide a 3-D printed article or object. In some embodiments, polymerizing or curing comprises irradiating with electromagnetic radiation having sufficient energy to initiate a polymerization or cross-linking reaction. In other embodiments, ultraviolet (UV) radiation can be used.

In some embodiments, a polymerizable component comprises a monomeric chemical species, such as a chemical species having one or more functional groups or moieties that can react with the same or different functional groups or moieties of another monomeric chemical species to form one or more covalent bonds, such as in a polymerization reaction. A polymerization reaction, in some embodiments, comprises a free radical polymerization, such as between points of unsaturation, including points of ethylenic unsaturation. In some embodiments, a polymerizable component comprises at least one ethylenically unsaturated moiety, such as a vinyl group or allyl group. In some embodiments, a polymerizable component comprises an oligomeric chemical species capable of undergoing additional polymerization, such as through one or more points of unsaturation as described herein. In other embodiments, a polymerizable component comprises one or more monomeric chemical species and one or more oligomeric chemical species as described herein. A monomeric chemical species and/or an oligomeric chemical species described herein can have one polymerizable moiety or a plurality of polymerizable moieties.

In some embodiments, a polymerizable component comprises one or more photo-polymerizable or photo-curable chemical species. A photo-polymerizable chemical species, in some embodiments, comprises a UV-polymerizable chemical species. In some embodiments, a polymerizable component is photo-polymerizable or photo-curable at wavelengths ranging from about 300 nm to about 400 nm. Alternatively, in some embodiments, a polymerizable component is photo-polymerizable at visible wavelengths of the electromagnetic spectrum.

In some embodiments, a polymerizable component described herein comprises one or more species of (meth)acrylates including acrylate or methacrylate or mixtures or combinations thereof. In other embodiments, a polymerizable component comprises an aliphatic polyester urethane acrylate oligomer, a urethane (meth)acrylate resin, and/or an acrylate amine oligomeric resin, such as EBECRYL 7100. In yet other embodiments, a UV polymerizable or curable resin or oligomer can comprise any methacrylate or acrylate resin which polymerizes in the presence of a free radical photoinitiator, is thermally stable in an exposed state for at least one week at a jetting temperature and for at least 4 weeks in an enclosed state, and/or has a boiling point greater than the jetting temperature. In some embodiments, a polymerizable component has a flash point above the jetting temperature.

Urethane (meth)acrylates suitable for use in inks described herein, in some embodiments, can be prepared in a known manner, typically reacting a hydroxyl-terminated urethane with acrylic acid or methacrylic acid to give the corresponding urethane (meth)acrylate, or by reacting an isocyanate-terminated prepolymer with hydroxyalkyl acrylates or methacrylates to give the urethane (meth)acrylate. The weight average molecular weight of such (meth)acrylate oligomers is generally in the range from about 400 to 10,000, or from about 500 to 7,000. Urethane (meth)acrylates are commercially available from the SARTOMER Company under the product names CN980, CN981, CN975 and CN2901, or from Bomar Specialties Co. (Winsted, Conn.) under the product name BR-741. In some embodiments, a urethane (meth)acrylate oligomer has a viscosity ranging from about 140,000 cP to about 160,000 cP at about 50° C or from about 125,000 cP to about 175,000 cP at about 50° C when measured in a manner consistent with ASTM D2983. In some embodiments described herein, a urethane (meth)acrylate oligomer has a viscosity ranging from about 100,000 cP to about 200,000 cP at about 50° C or from about 10,000 cP to about 300,000 cP at about 50° C when measured in a manner consistent with ASTM D2983.

In various embodiments, a polymerizable component comprises one or more low molecular weight materials, such as methacrylates, dimethacrylates, triacrylates, and diacrylates, which can be used in a variety of combinations. In some embodiments, for example, a polymerizable component comprises one or more of tetrahydrofurfuryl methacrylate, triethylene glycol dimethacrylate, 2-phenoxyethyl methacrylate, lauryl methacrylate, ethoxylated trimethylolpropane triacrylate, tricyclodecane dimethanol diacrylate, 2-phenoxyethylacrylate, triethylene glycol diacrylate, a monofunctional aliphatic urethane acrylate, polypropylene glycol monomethacrylate, polyethylene glycol monomethacrylate, cyclohexane dimethanol diacrylate, and tridecyl methacrylate.

In some embodiments, a polymerizable component comprises diacrylate and/or dimethacrylate esters of aliphatic, cycloaliphatic or aromatic diols, including 1,3- or 1,4-butanediol, neopentyl glycol, 1,6-hexanediol, diethylene glycol, triethylene glycol, tetraethylene glycol, polyethylene glycol, tripropylene glycol, ethoxylated or propoxylated neopentyl glycol, 1,4-dihydroxymethylcyclohexane, 2,2-bis(4-hydroxycyclohexyl)propane or bis(4-hydroxycyclohexyl)methane, hydroquinone, 4,4'-dihydroxybiphenyl, bisphenol A, bisphenol F, bisphenol S, ethoxylated or propoxylated bisphenol A, ethoxylated or propoxylated bisphenol F or ethoxylated or propoxylated bisphenol S.

A polymerizable component, in some embodiments, comprises one or more tri(meth)acrylates. In some embodiments, tri(meth)acrylates comprise 1,1-trimethylolpropane triacrylate or methacrylate, ethoxylated or propoxylated 1,1,1-trimethylolpropanetriacrylate or methacrylate, ethoxylated or propoxylated glycerol triacrylate, pentaerythritol monohydroxy triacrylate or methacrylate, or tris(2-hydroxy ethyl) isocyanurate triacrylate.

In other embodiments, a polymerizable component of the composite filament described herein comprises one or more higher functional acrylates or methacrylates such as dipentaerythritol monohydroxy pentaacrylate or bis(trimethylolpropane) tetraacrylate. In some embodiments, a (meth)acrylate of an ink has a molecular weight ranging from about 250 to 700.

In certain embodiments, a polymerizable component comprises allyl acrylate, allyl methacrylate, methyl (meth)acrylate, ethyl (meth)acrylate, n-propyl (meth)acrylate, n-butyl (meth)acrylate, isobutyl (meth)acrylate, n-hexyl (meth)acrylate, 2-ethylhexyl (meth)acrylate, n-octyl (meth)acrylate, n-decyl (meth)acrylate and n-dodecyl (meth)acrylate, 2-hydroxyethyl (meth)acrylate, 2- and 3-hydroxypropyl (meth)acrylate, 2-methoxyethyl(meth)acrylate, 2-ethoxyethyl (meth)acrylate and 2- or 3-ethoxypropyl (meth)acrylate, tetrahydrofurfuryl methacrylate, 2-(2-ethoxyethoxyl)ethyl acrylate, cyclohexyl methacrylate, 2-phenoxyethyl acrylate, glycidyl acrylate, isodecyl acrylate, or a combination thereof.

Additional non-limiting examples of species of polymerizable components useful in some embodiments described herein include the following: isobornyl acrylate (IBOA), commercially available from SARTOMER under the trade name SR 506A; isobornyl methacrylate, commercially available from SARTOMER under the trade name SR 423A; alkoxylated tetrahydrofurfuryl acrylate, commercially available from SARTOMER under the trade name SR 611; monofunctional urethane acrylate, commercially available from RAHN USA under the trade name GENOMER 1122; aliphatic urethane diacrylate, commercially available from ALLNEX under the trade name EBECRYL 8402; triethylene glycol diacrylate, commercially available from SARTOMER under the trade name SR 272; triethylene glycol dimethacrylate, commercially available from SARTOMER under the trade name SR 205; tricyclodecane dimethanol diacrylate, commercially available from SARTOMER under the trade name SR 833S; tris(2-hydroxy ethyl)isocyanurate triacrylate, commercially available from SARTOMER under the trade name SR 368; and 2-phenoxyethyl acrylate, commercially available from SARTOMER under the trade name SR 339. Other commercially available curable materials may also be used.

The composite filament ink useful for the 3-D printing system described in this disclosure can also include one or more additives comprising photoinitiators, inhibitors, stabilizing agents, sensitizers, and combinations thereof. In some embodiments, suitable photoinitiators comprise benzoins, including benzoin, benzoin ethers, such as benzoin methyl ether, benzoin ethyl ether and benzoin isopropyl ether, benzoin phenyl ether and benzoin acetate, acetophenones, including acetophenone, 2,2-dimethoxyacetophenone and 1,1-dichloroacetophenone, benzil, benzil ketals, such as benzil dimethyl ketal and benzil diethyl ketal, anthraquinones, including 2-methylanthraquinone, 2-ethylanthraquinone, 2-tert-butylanthraquinone, 1-chloroanthraquinone and 2-amylanthraquinone, triphenylphosphine, benzoylphosphine oxides, for example 2,4,6-trimethylbenzoyldiphenylphosphine oxide (Lucirin TPO), benzophenones, such as benzophenone and 4,4'-bis(N,N'-dimethylamino)benzophenone, thioxanthones and xanthones, acridine derivatives, phenazine derivatives, quinoxaline derivatives or 1-phenyl-1,2-propanedione, 2-O-benzoyl oxime, 1-aminophenyl ketones or 1-hydroxyphenyl ketones, such as 1-hydroxycyclohexyl phenyl ketone, phenyl 1-hydroxyisopropyl ketone and 4-isopropylphenyl 1-hydroxyisopropyl ketone.

In some cases, suitable photoinitiators comprise those operable for use with a HeCd laser radiation source, including acetophenones, 2,2-dialkoxybenzophenones and 1-hydroxyphenyl ketones, such as 1-hydroxycyclohexyl phenyl ketone or 2-hydroxyisopropyl phenyl ketone (2-hydroxy-2,2-dimethylacetophenone). Additionally, in other aspects, suitable photoinitiators comprise those operable for use with an Ar laser radiation source including benzil ketals, such as benzil dimethyl ketal. In some embodiments, a photoinitiator comprises an *a-*hydroxyphenyl ketone, benzil dimethyl ketal or 2,4,6-trimethylbenzoyldiphenylphosphine oxide or a mixture thereof.

Other suitable photoinitiators comprise ionic dye-counter ion compounds capable of absorbing actinic radiation and generating free radicals for polymerization initiation. In some embodiments, inks containing ionic dye-counter ion compounds can be cured more variably with visible light within the adjustable wavelength range of about 400 nm to about 700 nm.

A photoinitiator can be present in an ink described herein in any amount not inconsistent with the objectives of the present disclosure. In some embodiments, a photoinitiator is present in an ink in an amount of up to about 5 weight percent, based on the total weight of the ink. In some embodiments, a photoinitiator is present in an amount ranging from about 0.1 weight percent to about 5 weight percent.

In some embodiments, a method of printing a 3-D article comprises selectively depositing layers of a composite ink described herein in a fluid state onto a substrate. For example, in some cases, the composite filament ink comprises a curable material and a colorant dispersed in the curable material in an amount of about 0.01 to 5 weight %, based on the total weight of the composite ink. Further, the layers of a composite filament ink can be deposited according to an image of the 3-D article in a computer readable format. In some embodiments, the ink is deposited according to preselected computer aided design (CAD) parameters onto a metal or non-metal substrate.

Moreover, in some cases, one or more layers of a composite ink described herein have a thickness of about 0.03 to about 5 mm, a thickness of about 0.03 to about 3 mm, a thickness of about 0.03 to about 1 mm, a thickness of about 0.03 to about 0.5 mm, a thickness of about 0.03 to about 0.3 mm, a thickness of about 0.03 to about 0.2 mm, a thickness of about 0.05 to about 5 mm, a thickness of about 0.05 to about 1 mm, a thickness of about 0.05 to about 0.5 mm, a thickness of about 0.05 to about 0.3 mm, or a thickness of about 0.05 to about 0.2 mm. Other thicknesses are also possible.

A method described herein can also comprise curing the layers of the composite ink. In some embodiments, a method of printing a 3-D article further comprises subjecting the ink to electromagnetic radiation of sufficient wavelength and intensity to cure the ink, where curing can comprise polymerizing one or more polymerizable functional groups of one or more components of the ink. In some embodiments of printing a 3-D article, a layer of deposited ink is cured prior to the deposition of another or adjacent layer of ink.

In some embodiments, a preselected amount of ink described herein is heated to the appropriate temperature and jetted through the print head or a plurality of print heads of a suitable inkjet printer to form a layer on a print pad in a print chamber. In some embodiments, each layer of ink is deposited according to the preselected CAD parameters. A suitable print head to deposit the ink, in some embodiments, is a piezoelectric print head. Additional suitable print heads for the deposition of ink and support material described herein are commercially available from a variety of ink jet printing apparatus manufacturers. For example, Xerox, Hewlett Packard, or Ricoh print heads may also be used in some instances.

In some embodiments, a method of printing a 3-D article comprises using a composite ink, wherein the composite ink remains substantially fluid upon deposition. In other embodiments, the ink exhibits a phase change upon deposition and/or solidifies upon deposition. In some embodiments, the temperature of the printing environment can be controlled so that the jetted droplets of ink solidify on contact with the receiving surface. In other embodiments, the jetted droplets of ink do not solidify on contact with the receiving surface, remaining in a substantially fluid state. In some embodiments, after each layer is deposited, the deposited material is planarized and cured with electromagnetic (e.g., UV) radiation prior to the deposition of the next layer. Optionally, several layers can be deposited before planarization and curing, or multiple layers can be deposited and cured followed by one or more layers being deposited and then planarized without curing. Planarization corrects the thickness of one or more layers prior to curing the material by evening the dispensed material to remove excess material and create a uniformly smooth exposed or flat up-facing surface on the support platform of the printer.

In another embodiment, mechanical, magnetic, and/or piezoelectric sensitive micro- or nanoparticles or patterns are incorporated during 3-D printing to stimulate cellular functions upon implantation under a variety of in vivo or in vitro mechanical magnetic or pressure conditions.

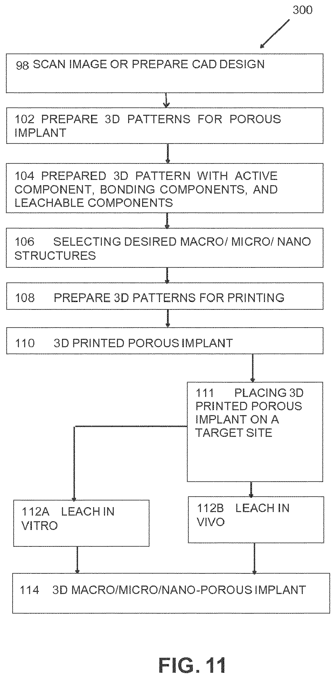

FIG. 11 is a flow diagram of representative steps of method 300 of a computer implemented method of producing a custom porous implant in one embodiment. Method 300 includes step 98 for obtaining a 3-D image of the intended implant location or intended tissue repair site including the topography of the repair site or the implant, or the implant location. Step 98 can be accomplished by using many known techniques of obtaining a 3-D image including, but not limited to scanning (i) one or more X-ray images; (ii) a computer aided design (CAD) program; (iii) a cone beam imaging device; (iv) a computed tomography (CT) scan device; (v) a magnetic resonance imaging (MRI); or a combination thereof. In step 98, the image is scanned or a CAD design is prepared. In step 102, the images obtained in step 98 are utilized to prepare 3-D patterns for the porous implant. In step 104, the 3-D pattern of the porous implant can be prepared with active components such as bone particles, bonding components such as polymers and leachable components, for example porogens. In step 106, a desired macro/micro or nano pore structure is selected by selecting an appropriate porogen for the desired pore structure. In step 108 the 3-D patterns are prepared for printing and the porous implant is 3-D printed in step 110. Once printed, in step 111, the porous implant can be placed on or at a target site, namely an intended tissue repair site. Thereafter, the porogen of the porous implant can be leached or otherwise removed *in vitro* or *in vivo* as illustrated in steps 112A and 112B. The cycle of the 3-D printed macro/micro or nano porous implant is now complete as shown in step 114.

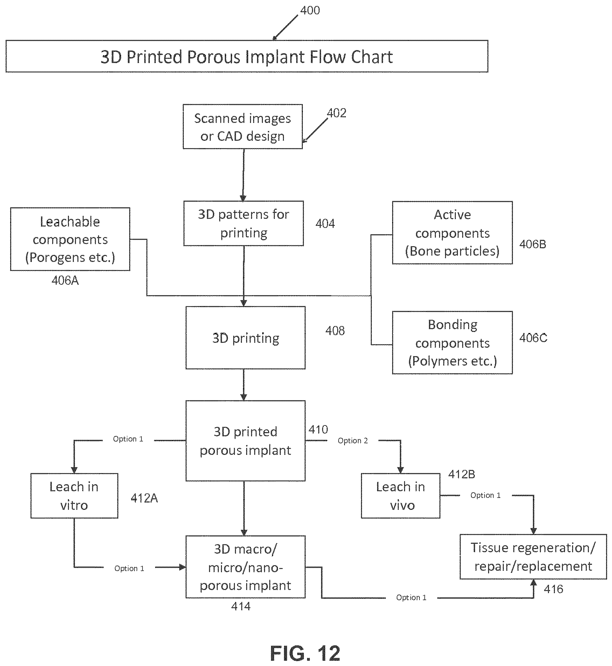

In another embodiment, FIG. 12 is a flow diagram of representative steps of method 400 of a computer implemented method of producing a custom porous implant. Method 400 includes step 402 wherein required images for 3-D printing are scanned or obtained by CAD design. Step 402 can be accomplished by using many known techniques of obtaining a 3-D image including, but not limited to scanning (i) one or more X-ray images; (ii) a computer aided design (CAD) program; (iii) a cone beam imaging device; (iv) a computed tomography (CT) scan device; (v) a magnetic resonance imaging (MRI); or a combination thereof. In step 404, the images obtained in step 402 are utilized to prepare 3-D patterns for printing the porous implant. In steps 406A, 406B and 406C, active components such as bone particles, bonding components such as polymers and leachable components, for example, porogens are combined and fed into step 408 3-D printing to provide in step 410 a 3-D printed porous implant. Once printed, in steps 412A and 412B, the porogen of the porous implant can be leached or otherwise removed *in vitro* or *in vivo.* In step 412A, the *in vitro* leaching occurs before the porous implant is implanted at an intended tissue repair site. The *in vitro* leaching is completed in step 414, so that the 3-D macro-porous, micro-porous, or nano-porous implant can be placed at the tissue regeneration, repair or replacement site in step 416. The *in vivo* leaching is completed in step 412B, so that the 3-D macro-, micro-, or nano-porous implant can be placed at the tissue regeneration, repair or replacement site in step 416.

### A Layered 3-D Printed Porous implant

In certain embodiments, the computer implemented method described herein provides a layered 3-D printed porous implant. In some implementations, the 3-D printed porous implant includes a first layer of implant material; a second layer of porogen disposed on the first layer of implant material; a third layer of implant material disposed on the second layer, each layer repeating until a 3-D printer has completed the porous implant. In other embodiments, the 3-D printed porous implant includes a first layer of implant material mixed with porogen; a second layer of implant material mixed with porogen, the second layer disposed on the first layer; a third layer of implant material mixed with porogen, the third layer disposed on the second layer, each layer repeating until the 3-D printer has completed the porous implant.

As discussed above in connection with the computer implemented method for producing the customized porous implant of this disclosure, in some embodiments, (i) the porogen is a gas, liquid or solid; (ii) macropores have a pore diameter greater than about 100 µ, micropores have the pore diameter below about 10 µ and nanopores have the pore diameter of about 1nm; or (iii) the porogen is spheroidal, cuboidal, rectangular, elongated, tubular, fibrous, disc-shaped, platelet-shaped, polygonal or a mixture thereof. In particular, the porogen can be (i) carbon dioxide, nitrogen, argon or air; (ii) water, blood lymph, plasma, serum or marrow; (iii) polysaccharides comprising cellulose, starch, amylose, dextran, poly(dextrose), glycogen, poly(vinylpyrollidone), pullulan, poly(glycolide), poly(lactide), poly(lactide-co-glycolide; or (iv) sodium chloride, sugar, hydroxyapatite or polyethylene oxide, polylactic acid, polycaprolactone; (v) peptides, proteins of fifty amino acids or less or a parathyroid hormone.

As discussed above in connection with the computer implemented method for producing the customized porous implant of this disclosure, in some embodiments, (i) the implant material comprises a carrier material and a bone material or (ii) the implant material comprises natural material, a biodegradable carrier and a growth factor. In other aspects, the carrier material of the implant material can comprise a metal, a biodegradable polymer or a combination thereof and the bone material comprises mineralized or demineralized bone.

As discussed above in connection with the computer implemented method for producing the customized porous implant of this disclosure, in some embodiments, the bone material of the porous implant comprises (i) mineralized allograft and non-demineralized allograft or a combination thereof; or (ii) allograft, demineralized bone matrix fiber and demineralized bone chips or a combination thereof. In other embodiments, the layered 3-D printed porous implant contains bone material which comprises (i) fully demineralized bone fibers and surface demineralized bone chips, or (ii) a demineralized bone matrix material comprising fully demineralized bone matrix fibers and surface demineralized bone chips in a ratio of from about 25:75 to about 75:25.

In various embodiments, as described above, the polymer of the carrier material comprises a curable biocompatible and/or biodegradable polymer. In these embodiments, the biodegradable polymer comprises at least one of poly(lactic acid), poly(glycolic acid), poly(lactic acid-glycolic acid), polydioxanone, PVA, polyurethanes, polycarbonates, polyhydroxyalkanoates (polyhydroxybutyrates and polyhydroxyvalerates and copolymers), polysaccharides, polyhydroxyalkanoates, polyglycolide-co-caprolactone, polyethylene oxide, polypropylene oxide, polyglycolide-co-trimethylene carbonate, poly(lactic-co-glycolic acid) or combinations thereof. In other embodiments, the biodegradable polymer further comprises at least one of a polymer sugar, protein, hydrophilic block copolymer, hyaluronic acid, polyuronic acid, mucopolysaccharide, proteoglycan, polyoxyethylene, surfactant, polyhydroxy compound, polyhydroxy ester, fatty alcohol, fatty alcohol ester, fatty acid, fatty acid ester, liquid silicone, or combinations thereof.

In some uses, the carrier acts as a temporary scaffold until replaced by new bone. Polylactic acid (PLA), polyglycolic acid (PGA), and various combinations have different dissolution rates in vivo.

### Sterilization of the Porous implant

In various aspects, the 3-D printed porous implants obtained by the methods of this application can be terminally sterilized as they are formed, during the curing process or in the final packaging step. In various embodiments, one or more components of the porous implant may be sterilizable by radiation in a terminal sterilization step in the final packaging. Terminal sterilization of a product provides greater assurance of sterility than from processes such as an aseptic process, which require individual product components to be sterilized separately and the final package assembled in a sterile environment.

Typically, in various embodiments, gamma radiation is used in the terminal sterilization step, which involves utilizing ionizing energy from gamma rays that penetrates deeply in the device. Gamma rays are highly effective in killing microorganisms, they leave no residues nor have sufficient energy to impart radioactivity to the device. Gamma rays can be employed when the device is in the package and gamma sterilization does not require high pressures or vacuum conditions, thus, package seals and other components are not stressed. In addition, gamma radiation eliminates the need for permeable packaging materials.

In some embodiments, the porous implant may be packaged in a moisture resistant package and then terminally sterilized by gamma irradiation. In use, the surgeon removes the one or all components from the sterile package for use.

In various embodiments, electron beam (e-beam) radiation may be used to sterilize one or more components of the implant. E-beam radiation comprises a form of ionizing energy, which is generally characterized by low penetration and high-dose rates. E-beam irradiation is similar to gamma processing in that it alters various chemical and molecular bonds on contact, including the reproductive cells of microorganisms. Beams produced for e-beam sterilization are concentrated, highly-charged streams of electrons generated by the acceleration and conversion of electricity.

Other methods may also be used to sterilize the porous implant and/or one or more components of the implant, including, but not limited to, gas sterilization, such as, for example, with ethylene oxide or steam sterilization.

### Applications of the Porous Implant

As a result of the computer implemented method described in this application, a 3-D printed customized porous implant is provided which can meet dimensional, structural, mechanical and biological requirements for a variety of tissue repair sites, including bone defects, osteochondral defects and or cartilage defects. The 3-D printed process can be used to produce customized porous implants quickly. The customized porous implants can be easily and efficiently controlled to include macro-, micro-, and/or nano-structures.

The layered 3-D printed porous implants obtained by the computer implemented method described herein are useful in many applications, including without limitations, in oral maxillofacial surgery, dental implants, orthopedic surgery or any type of reconstructive hard tissue surgery, in some instances, in cortical or trabecular bone. The matrix can be utilized in a wide variety of orthopedic, periodontal, neurosurgical, oral and maxillofacial surgical procedures such as the repair of simple and/or compound fractures and/or non-unions; external and/or internal fixations; joint reconstructions such as arthrodesis; general arthroplasty; cup arthroplasty of the hip; femoral and humeral head replacement; femoral head surface replacement and/or total joint replacement; repairs of the vertebral column including spinal fusion and internal fixation; tumor surgery, for example, deficit filling; discectomy; laminectomy; excision of spinal cord tumors; anterior cervical and thoracic operations; repairs of spinal injuries; scoliosis, lordosis and kyphosis treatments; intermaxillary fixation of fractures; mentoplasty; temporomandibular joint replacement; alveolar ridge augmentation and reconstruction; inlay implantable matrices; implant placement and revision; sinus lifts; cosmetic procedures; etc. Specific bones that can be repaired or replaced with the porous implant herein include the ethmoid, frontal, nasal, occipital, parietal, temporal, mandible, maxilla, zygomatic, cervical vertebra, thoracic vertebra, lumbar vertebra, sacrum, rib, sternum, clavicle, scapula, humerus, radius, ulna, carpal bones, metacarpal bones, phalanges, ilium, ischium, pubis, femur, tibia, fibula, patella, calcaneus, tarsal and/or metatarsal bones.

In clinical use, the layered 3-D printed porous implants of this application allow not only for customizing to accommodate the anatomy of an individual patient, but also can successfully be used to release a specific drug from the porous implant to an intended bone repair site. The layered 3-D printed porous implants obtained by the computer implemented methods described herein can be customized based on a donor tissue, including soft and hard tissues, including fine donor bone dust.

Accordingly, in some implementations, this application also provides a method of treating a bone defect in a patient. In certain aspects, the method comprises administering a layered 3-D printed porous implant which comprises a first layer of implant material; a second layer of porogen disposed on the first layer of implant material; a third layer of implant material disposed on the second layer, each layer repeating until a 3-D printer has completed the porous implant. In other aspects, the method of treatment includes administering a layered 3-D porous implant which comprises a first layer of implant material mixed with porogen; a second layer of implant material mixed with porogen, the second layer disposed on the first layer; a third layer of implant material mixed with porogen, the third layer disposed on the second layer, each layer repeating until the 3-D printer has completed the porous implant.

In certain embodiments, when the 3-D printed porous implant is a porous mesh bag, any suitable method may be used for loading a bone material into the porous mesh bag. In some embodiments, the bone material may be spooned into the porous mesh bag, placed in the porous mesh bag body using forceps, loaded into the porous mesh bag using a syringe (with or without a needle), or inserted into the porous mesh bag in any other suitable manner including using an automation.

For placement, the substance or substances may be provided in the mesh implant or bag and placed *in vivo,* for example, at a bone defect. In one embodiment, the porous mesh bag is placed *in vivo* by placing the porous mesh bag in a catheter or tubular inserter and delivering the porous mesh bag with the catheter or tubular inserter. The porous mesh bag, with a substance provided therein, may be steerable such that it can be used with flexible introducer instruments for, for example, minimally invasive spinal procedures. For example, the porous implant may be introduced down a tubular retractor or scope, during XLIF, TLIF, or other procedures.

In clinical use, a delivery system comprising a mesh implant and delivered substance may be used in any type of spinal fusion procedure including, for example, posterolateral fusion, interbody fusion (of any type), facet fusion, spinous process fusion, anterior only fusion, or other fusion procedure. Examples of such spinal procedures include posterior lumbar interbody fusion (PLIF), anterior lumbar fusion (ALIF) or posterior cervical or cervical interbody fusion approaches. In some embodiments, the mesh implant useful with TLIF, ALIF or XLIF procedures may be tubular and have dimensions of approximately 2.5 cm in length and approximately 0.5 cm in width. In other ALIF procedures, a mesh implant of approximately 1 cm by 1 cm can be used. In various embodiments, the mesh implants may be tubular and may have dimensions of approximately 5 mm to approximately 10 mm long and approximately 0.5 cm to 1 cm wide. In other embodiments, the mesh implant or bag (with or without substance loaded) may be placed in a cage, for example, for interbody fusion.

In some embodiments, the 3-D printed porous mesh bag may be prefilled with a substance for delivery and other compartments may be empty for filling by the surgeon. In some embodiments, the 3-D mesh implant comprises a first and a second compartment. In other embodiments, the first and second compartments of the 3-D mesh implant are in communication with each other. In several embodiments, one compartment may be bone filled while the other compartment of the 3-D mesh implant is not. In various embodiments, the 3-D printed seamless mesh implant conforms to surrounding bony contours when implanted *in vivo.*

The mesh implant or bag may be used in any suitable application. In some embodiments, the porous mesh bag may be used in healing vertebral compression fractures, interbody fusion, minimally invasive procedures, posterolateral fusion, correction of adult or pediatric scoliosis, treating long bone defects, osteochondral defects, ridge augmentation (dental/craniomaxillofacial, e.g. edentulous patients), beneath trauma plates, tibial plateau defects, filling bone cysts, wound healing, around trauma, contouring (cosmetic/plastic/reconstructive surgery), and others. The mesh implant or bag may be used in a minimally invasive procedure via placement through a small incision, via delivery through a tube, or other means. The size and shape may be designed with restrictions on delivery conditions.

In some embodiments, the porous mesh bag is flexible enough so that it can be folded upon itself before it is implanted at, near, or in the bone defect.

An exemplary application for using a porous mesh bag as disclosed is fusion of the spine. In clinical use, the porous mesh bag and delivered substance may be used to bridge the gap between the transverse processes of adjacent or sequential vertebral bodies. The porous mesh bag may be used to bridge two or more spinal motion segments. The porous mesh bag surrounds the substance to be implanted, and contains the substance to provide a focus for healing activity in the body.

Generally, the mesh implant or bag may be applied to a pre-existing defect, to a created channel, or to a modified defect. Thus, for example, a channel may be formed in a bone, or a pre-existing defect may be cut to form a channel, for receipt of the device. The mesh implant or bag may be configured to match the channel or defect. In some embodiments, the configuration of the porous mesh bag may be chosen to match the channel. In other embodiments, the channel may be created, or the defect expanded or altered, to reflect a configuration of the porous mesh bag. The mesh implant or bag may be placed in the defect or channel and, optionally, coupled using attachment mechanisms.

Although the invention has been described with reference to certain embodiments, persons skilled in the art will recognize that changes may be made in form and detail without departing from the scope of the invention.

For Example, further embodiments of the invention may be as follows:

According to a first further embodiment, the invention provides a computer implemented method for producing a porous implant, the method comprising: obtaining a 3-D image of an intended tissue repair site; generating a 3D digital model of the porous implant based on the 3-D image of the intended tissue repair site, the 3-D digital model of the porous implant being configured to fit within the tissue repair site; determining an amount of an implant material and a porogen to add to the implant material to obtain a desired porosity of the porous implant based on the 3-D digital model of the porous implant, the porosity being based on a plurality of macropores, micropores, nanopores structures or a combination thereof; storing the 3-D digital model of the digital model on a database coupled to a processor, the processor having instructions for combining the implant material with the porogen based on the stored 3-D digital model and for instructing a 3-D printer to produce the porous implant and removing the porogen prior to implantation of the porous implant at the intended tissue repair site.

## Claims

1. A computer implemented method for producing a porous implant, the method comprising: obtaining a 3-D image of an intended tissue repair site; generating a 3-D digital model of the porous implant based on the 3-D image of the intended tissue repair site, the 3-D digital model of the porous implant being configured to fit within the tissue repair site; determining an implant material and an amount of a porogen to add to an implant material to obtain a desired porosity of the porous implant based on the 3-D digital model of the porous implant, the porosity being based on a plurality of macropores, micropores, nanopores structures or a combination thereof; storing the 3-D digital model on a database coupled to a processor, the processor having instructions for combining the implant material with the porogen based on the stored 3-D digital model and for instructing a 3-D printer to produce the porous implant.

2. The computer implemented method of claim 1, wherein (i) the porogen is a gas, liquid or solid; (ii) the plurality of macropores have a pore diameter greater than about 100 µ, the plurality of micropores have the pore diameter below about 10 µ and the plurality of nanopores have the pore diameter of about 1 nm; or (iii) the porogen is spheroidal, cuboidal, rectangular, elongated, tubular, fibrous, disc-shaped, platelet-shaped, polygonal or a mixture thereof,
wherein, optionally, the porogen is (i) carbon dioxide, nitrogen, argon or air; or (ii) polysaccharides comprising cellulose, starch, amylose, dextran, poly(dextrose), glycogen, poly(vinylpyrollidone), pullulan, poly(glycolide), poly(lactide), poly(lactide-co-glycolide; (iii) sodium chloride, sugar, hydroxyapatite or polyethylene oxide, polylactic acid, polycaprolactone; (iv) a peptide or protein; or (v) a parathyroid hormone.

3. The computer implemented method of claim 1 or 2, further comprising (i) removing the porogen prior to implantation of the porous implant at the intended tissue repair site or (ii) removing the porogen after implantation of the porous implant at the intended tissue repair site.

4. The computer implemented method of any one of claims 1 to 3, wherein (i) the intended tissue repair site is a bone repair site, an osteochondral defect site, an articular cartilage defect site or a combination thereof; (ii) the implant material comprises a carrier material and a bone material; or
(iii) the implant material comprises a natural material, a biodegradable carrier and a growth factor.

5. The computer implemented method of claim 4, wherein the carrier material comprises a metal, a biodegradable polymer or a combination thereof, and the bone material comprises mineralized or demineralized bone, wherein, optionally, the porous implant comprises from about 10% to about 80% by weight of porogen, from about 20% to about 90% by weight of the polymer and from about 30% to about 50% by weight of bone material.

6. The computer implemented method of claim 4 or 5, wherein the carrier material is a biodegradable polymer comprising poly(L-lactide-co-D,L-lactide), polyglyconate, poly(arylates), poly(anhydrides), poly(hydroxy acids), polyesters, poly(ortho esters), poly(alkylene oxides), polycarbonates, poly(propylene fumarates), poly(propylene glycol-co fumaric acid), poly(caprolactones), polyamides, polyesters, polyethers, polyureas, polyamines, polyamino acids, polyacetals, poly(orthoesters), poly(pyrolic acid), poly(glaxanone), poly(phosphazenes), poly(organophosphazene), polylactides, polyglycolides, poly(dioxanones), polyhydroxybutyrate, polyhydroxyvalyrate, polyhydroxy-butyrate/valerate copolymers, poly(vinyl pyrrolidone), polycyanoacrylates, polyurethanes, polysaccharides or a combination thereof.

7. The computer implemented method of any one of claims 4 to 6, wherein the bone material comprises autograft, allograft, demineralized bone matrix fiber, demineralized bone chips or a combination thereof.

8. The computer implemented method of any one of claims 4 to 7, wherein the bone material comprises (i) fully demineralized bone fibers and surface demineralized bone chips; or (ii) fully demineralized bone matrix fibers and surface demineralized bone chips in a ratio of from about 25:75 to about 75:25.

9. The computer implemented method according to any one of claims 1 to 8, wherein the implant material further comprises a drug, a growth factor, a protein or a combination thereof.

10. The computer implemented method according to any one of claims 1 to 9, wherein the implant material further comprises (i) an inorganic polymer; (ii) soft tissue particles comprises cartilage particles; or (iii) inorganic particles comprise hydroxy apatite, calcium HA, carbonated calcium HA, beta- tricalcium phosphate (beta-TCP), alpha-tricalcium phosphate (alpha-TCP), hydroxyapatite, amorphous calcium phosphate (ACP), octacalcium phosphate (OCP), tetracalcium phosphate, biphasic calcium phosphate (BCP), anhydrous dicalcium phosphate (DCPA), dicalcium phosphate dihydrate (DCPD), anhydrous monocalcium phosphate (MCPA), monocalcium phosphate monohydrate (MCPM), synthetic calcium phosphate ceramic or combinations thereof.

11. The computer implemented method of any one of claims 1 to 10, wherein the 3-D image of an intended porous implant site is a computed tomography image of an unhealthy bone tissue repair site based on a computed tomography image of a healthy tissue repair site.

12. The computer implemented method of any one of claims 1 to 11, wherein the 3-D image is obtained from (i) one or more X-ray images; (ii) a computer aided design (CAD) program; (iii) a cone beam imaging device; (iv) a computed tomography (CT) scan device; or (v) a magnetic resonance imaging (MRI).

13. A layered 3-D printed porous implant, the layered porous implant comprising:
(i) a first layer of implant material; a second layer of porogen disposed on the first layer of implant material; a third layer of implant material disposed on the second layer, each layer repeating until a 3-D printer has completed the porous implant; or (ii) a first layer of implant material mixed with porogen; a second layer of implant material mixed with porogen, the second layer disposed on the first layer; a third layer of implant material mixed with porogen, the third layer disposed on the second layer, each layer repeating until the 3-D printer has completed the porous implant.

14. A layered 3-D printed porous implant of claim 13, wherein (i) the porogen is a gas, liquid or solid; (ii) the porous implant comprises macropores that have a pore diameter greater than about 100 µ, micropores that have a pore diameter below about 10 µ and nanopores that have a pore diameter of about 1 nm; or (iii) the porogen is spheroidal, cuboidal, rectangular, elongated, tubular, fibrous, disc-shaped, platelet-shaped, polygonal or a mixture thereof,
wherein, optionally, the porogen is (i) carbon dioxide, nitrogen, argon or air; (ii) polysaccharides comprising cellulose, starch, amylose, dextran, poly(dextrose), glycogen, poly(vinylpyrollidone), pullulan, poly(glycolide), poly(lactide), poly(lactide-co-glycolide; (iii) sodium chloride, sugar, hydroxyapatite or polyethylene oxide, polylactic acid, polycaprolactone; (iv) a peptide or protein; or (v) a parathyroid hormone.

15. A layered 3-D printed porous implant of claim 13 or 14, wherein (i) the implant material comprises a carrier material and a bone material, wherein, optionally, the carrier material comprises a metal, a biodegradable polymer or a combination thereof and the bone material comprises mineralized or demineralized bone; or (ii) the implant material comprises a natural material, a biodegradable carrier and a growth factor.
